(19) Europäisches Patentamt
European Patent Office
Office européen des brevets

(11) EP 4 789 666 A1

# (12) EUROPEAN PATENT APPLICATION
published in accordance with Art. 153(4) EPC

(43) Date of publication:
**12.08.2026 Bulletin 2026/33**

(21) Application number: **24911393.7**

(22) Date of filing: **27.12.2024**

(51) International Patent Classification (IPC):
***A61K 31/403*** *(2006.01)* ***A61K 9/00*** *(2006.01)*
***A61P 17/00*** *(2006.01)*

(52) Cooperative Patent Classification (CPC):
**A61K 9/00; A61K 31/403; A61P 17/00; A61P 17/06; C07D 209/88**

(86) International application number:
**PCT/CN2024/142961**

(87) International publication number:
**WO 2025/140472 (03.07.2025 Gazette 2025/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.12.2023 CN 202311844405**

(71) Applicant: **SHANGHAI MERNA THERAPEUTICS CO. LTD**
**Shanghai 201203 (CN)**

(72) Inventors:
- **PENG, Cheng**
  **Shanghai 201203 (CN)**
- **WANG, Jingfang**
  **Shanghai 201203 (CN)**
- **SONG, Zhidong**
  **Shanghai 201203 (CN)**
- **WANG, Ying**
  **Shanghai 201203 (CN)**
- **TONG, Siyu**
  **Shanghai 201203 (CN)**

(74) Representative: **Ter Meer Steinmeister & Partner Patentanwälte mbB**
**Nymphenburger Straße 4**
**80335 München (DE)**

Remarks:
The complete document including Reference Table(s) and the Sequence Listing(s) can be downloaded from the EPO website

## (54) RNA M6A REGULATOR COMPOSITION, PREPARATION METHOD THEREFOR, AND USE THEREOF

(57) An RNA m6A regulator composition, a preparation method therefor, and use thereof are provided. The composition includes: a compound of formula **(I)** or a pharmaceutically acceptable salt thereof or a pharmaceutically acceptable co-crystal thereof, an oil phase substance 1, an oil phase substance 2, and an oil phase substance 3. The composition may further include a penetration enhancer and an antioxidant. The RNA m6A regulator composition can be used for preventing or treating skin diseases caused by RNA m6A methylation.

1.6
1.2
0.8
0.4
0
Pathological score
Blank control
Sorafenib
Compound of formula (I)
Blisters
Inflammation
Congestion
FIG.10

EP 4 789 666 A1

## Description

## CROSS REFERENCE TO RELATED APPLICATION

The present disclosure claims priority to Chinese patent application No. 2023118444059, filed with the China National Intellectual Property Administration on December 28, 2023, entitled "RNA M6A REGULATOR COMPOSITION, PREPARATION METHOD THEREFOR, AND USE THEREOF", the entire contents of which are incorporated herein by reference.

## TECHNICAL FIELD

The present disclosure relates to the technical field of medicinal chemistry, and particularly to an RNA m6A regulator composition, a preparation method therefor, and use thereof.

## BACKGROUND

A hand-foot syndrome (HFS) and a hand-foot skin reaction (HFSR) are erythematous skin lesions of palms and soles, which are mainly caused by cytotoxic chemotherapeutic drugs and tumor-targeted drugs, and in severe cases, may lead to loss of self-care ability in patients. Main pathological characteristics of the hand-foot syndrome and the hand-foot skin reaction include vacuolar degeneration of basal keratinocytes, infiltration of perivascular lymphocytes in the skin, apoptosis of keratinocytes, and hydroderma; and under a microscope, inflammatory changes, vasodilation, edema, and leukocyte infiltration can be observed.

Drugs that can cause the hand-foot syndrome include chemotherapeutic drugs such as capecitabine, liposomal doxorubicin, cytarabine, docetaxel, vinorelbine, continuously infused doxorubicin, and gemcitabine; and drugs that can cause the hand-foot skin reaction include targeted drugs such as sunitinib (Sutent), sorafenib (Nexavar), imatinib (Glivec), and erlotinib (Tarceva). HFS is classified by the World Health Organization (WHO) into 4 grades: Grade 1, dysesthesia, paresthesia, or tingling sensation in hands and feet; Grade 2, discomfort, painless swelling, or erythema when holding objects or walking; Grade 3, painful erythema, edema of palms and soles, periungual erythema, and swelling; and Grade 4, desquamation, ulceration, blistering, and severe pain. Currently, severe symptoms of the hand-foot syndrome and the hand-foot skin reaction occurring clinically are often only alleviated by superficial skin care or even require discontinuation of medication. Existing treatment means only address the symptoms, but not the root cause, thereby severely limiting the use of first-line chemotherapeutic drugs for tumors. Therefore, there are significant unmet medical demands. It is required to develop a drug with good efficacy, few side effects, and a low cost for preventing and treating the hand-foot syndrome and the hand-foot skin reaction to meet increasing global medical demands.

RNA m6A methylation modification is a most prevalent RNA modification in mammals, which can regulate multiple signaling pathways and cellular processes (such as growth, development, and diseases), thus playing critical biological actions (Frye M., et al. Science 2018, 361, 2073-2092; Yang C., et al. Cell Death & Disease 2020, 11, 960; Meyer K.D. & Jaffrey S.R. Nature Review Molecular Cell Biology 2014, 15, 313-326). The m6A methylation modification of mRNA, miRNA, circRNA, and lncRNA is a dynamically reversible process, in which methyltransferases (such as METTL3, METTL14, and METTL16) bind methyl to RNA, while demethylases (FTO and ALKBH5) can remove the methyl from RNA, thereby forming an m6A regulation basis. m6A affects processes, such as processing, translation, and degradation, of RNA by recruiting specific binding proteins (such as YTHDF1, YTHDF2, YTHDC1, and IGF2BP), thereby resulting in changes in downstream protein functions and cellular biological behaviors (Hsu P.J., et al. Journal of Biological Chemistry 2019, 294, 19889-19895; Yao Y., et al. FASEB Journal 2019, 33, 7529-7544).

Currently, there are few known direct associations between RNA m6A and skin-related diseases, and most of m6A-related methyltransferases or demethylases play roles in skin diseases (especially skin tumors). For example, the mRNA expression levels of METTL3 and ALKBH5 in tumor tissues of patients with acral melanoma are significantly higher than those in adjacent non-tumor tissues, and the mRNA expression level of METTL3 in patients with advanced acral melanoma is significantly higher than that in early-stage patients (Le Zhanghui, Preliminary Study on the Pathogenesis of LncRNA and RNA m6A Methylation in Acral Melanoma, Dissertation, 2019). In the aspect of diagnosis of melanoma, m6A-specific binding proteins YTHDF1 and HNRNPA2B1 can serve as novel biomarkers (Li T. D., et al. Cancer Cell 2020, 20, 239). In the aspect of keratinocytes, it has only been reported currently that long-term and low-level arsenic exposure can inhibit selective autophagy caused by the m6A demethylases, thereby inducing the occurrence of skin tumors (Cui Y. H., et al. Nature Communications 2021, 12, 2183). In summary, currently, there are no methods for preventing and treating skin diseases by regulating RNA m6A methylation.

## SUMMARY

Problems to be solved

To address the above technical problems, an objective of the present disclosure is to provide an RNA m6A regulator, a preparation method therefor, and use thereof, so as to significantly alleviate skin diseases caused by RNA m6A methylation.

Technical solutions

The present disclosure provides an RNA m6A regulator composition, where the composition includes: a compound of formula (I) or a pharmaceutically acceptable salt thereof or a pharmaceutically acceptable co-crystal thereof, and an oil phase substance,

(I).

Preferably, the pharmaceutically acceptable salt includes hydrochloride, sulfate, phosphate, acetate, methanesulfonate, benzenesulfonate, and p-toluenesulfonate;

preferably, a co-crystal former of the pharmaceutically acceptable co-crystal includes nicotinamide, isonicotinamide, L-proline, and glycolic acid;
preferably, the oil phase substance includes an oil phase substance 1, an oil phase substance 2, and an oil phase substance 3;
preferably, the oil phase substance 1 is selected from one or more of lanolin, beeswax, white beeswax, and paraffin, and preferably white beeswax;
preferably, the oil phase substance 2 is selected from one or more of petrolatum and white petrolatum, and preferably white petrolatum; and
preferably, the oil phase substance 3 is selected from one or more of light liquid paraffin and vegetable oil, and preferably light liquid paraffin.

Preferably, in terms of mass percentage, the composition includes 0.1%-20% of the compound of formula (I) or the pharmaceutically acceptable salt thereof or the pharmaceutically acceptable co-crystal thereof, 10%-30% of the oil phase substance 1, 30%-70% of the oil phase substance 2, and 15%-40% of the oil phase substance 3;

preferably, in terms of mass percentage, the composition includes 0.2%-15% of the compound of formula (I) or the pharmaceutically acceptable salt thereof or the pharmaceutically acceptable co-crystal thereof, 15%-25% of the oil phase substance 1, 35%-64% of the oil phase substance 2, and 20%-35% of the oil phase substance 3;
preferably, in terms of mass percentage, the composition includes 0.3%-10% of the compound of formula (I) or the pharmaceutically acceptable salt thereof or the pharmaceutically acceptable co-crystal thereof, 18%-20% of the oil phase substance 1, 40%-58.7% of the oil phase substance 2, and 22%-30% of the oil phase substance 3;
preferably, in terms of mass percentage, the composition includes 0.3% of the compound of formula (I) or the pharmaceutically acceptable salt thereof or the pharmaceutically acceptable co-crystal thereof, 18% of the oil phase substance 1, 58.7% of the oil phase substance 2, and 23% of the oil phase substance 3;
preferably, in terms of mass percentage, the composition includes 1% of the compound of formula (I) or the pharmaceutically acceptable salt thereof or the pharmaceutically acceptable co-crystal thereof, 18% of the oil phase substance 1, 58% of the oil phase substance 2, and 23% of the oil phase substance 3;
preferably, in terms of mass percentage, the composition includes 3% of the compound of formula (I) or the pharmaceutically acceptable salt thereof or the pharmaceutically acceptable co-crystal thereof, 18% of the oil phase substance 1, 57% of the oil phase substance 2, and 22% of the oil phase substance 3; and
preferably, in terms of mass percentage, the composition includes 10% of the compound of formula (I) or the pharmaceutically acceptable salt thereof or the pharmaceutically acceptable co-crystal thereof, 20% of the oil phase substance 1, 40% of the oil phase substance 2, and 30% of the oil phase substance 3.

Preferably, the composition includes the compound of formula (I) or the pharmaceutically acceptable salt thereof or the pharmaceutically acceptable co-crystal thereof, the white beeswax, the white petrolatum, and the light liquid paraffin;

preferably, in terms of mass percentage, the composition includes 0.1%-20% of the compound of formula (I) or the pharmaceutically acceptable salt thereof or the pharmaceutically acceptable co-crystal thereof, 10%-30% of the white beeswax, 30%-70% of the white petrolatum, and 15%-40% of the light liquid paraffin;
preferably, in terms of mass percentage, the composition includes 0.2%-15% of the compound of formula (I) or the pharmaceutically acceptable salt thereof or the pharmaceutically acceptable co-crystal thereof, 15%-25% of the white beeswax, 35%-64% of the white petrolatum, and 20%-35% of the light liquid paraffin;
preferably, in terms of mass percentage, the composition includes 0.3%-10% of the compound of formula (I) or the pharmaceutically acceptable salt thereof or the pharmaceutically acceptable co-crystal thereof, 18%-20% of the white beeswax, 40%-58.7% of the white petrolatum, and 22%-30% of the light liquid paraffin;
preferably, in terms of mass percentage, the composition includes 0.3% of the compound of formula (I), 18% of the white beeswax, 58.7% of the white petrolatum, and 23% of the light liquid paraffin;
preferably, in terms of mass percentage, the composition includes 1% of the compound of formula (I), 18% of the white beeswax, 58% of the white petrolatum, and 23% of the light liquid paraffin;
preferably, in terms of mass percentage, the composition includes 3% of the compound of formula (I), 18% of the white beeswax, 57% of the white petrolatum, and 22% of the light liquid paraffin; and
preferably, in terms of mass percentage, the composition includes 10% of the compound of formula (I), 20% of the white beeswax, 40% of the white petrolatum, and 30% of the light liquid paraffin.

Preferably, the composition further includes a penetration enhancer, more preferably, the penetration enhancer is selected from one or more of isopropyl myristate, isopropyl palmitate, propylene glycol dipelargonate, diethyl sebacate, laurocapram, and propylene glycol, and further preferably, the penetration enhancer is isopropyl myristate;

preferably, the composition further includes an antioxidant, more preferably, the antioxidant is selected from one or more of vitamin E, alkyl gallate, butylated hydroxyanisole (BHA), and butylated hydroxytoluene (BHT), and further preferably, the antioxidant is butylated hydroxytoluene (BHT); and
preferably, the composition further includes other pharmaceutically acceptable auxiliary materials.

Preferably, in terms of mass percentage, the composition includes 0.1%-20% of the compound of formula (I) or the pharmaceutically acceptable salt thereof or the pharmaceutically acceptable co-crystal thereof, 10%-30% of the oil phase substance 1, 30%-70% of the oil phase substance 2, 15%-30% of the oil phase substance 3, 0.1%-20% of the penetration enhancer, and 0%-2% of the antioxidant;

preferably, in terms of mass percentage, the composition includes 0.2%-15% of the compound of formula (I) or the pharmaceutically acceptable salt thereof or the pharmaceutically acceptable co-crystal thereof, 15%-25% of the oil phase substance 1, 35%-63% of the oil phase substance 2, 20%-25% of the oil phase substance 3, 1%-15% of the penetration enhancer, and 0%-1% of the antioxidant; and
preferably, in terms of mass percentage, the composition includes 0.3%-10% of the compound of formula (I) or the pharmaceutically acceptable salt thereof or the pharmaceutically acceptable co-crystal thereof, 18%-20% of the oil phase substance 1, 40%-58% of the oil phase substance 2, 20%-21% of the oil phase substance 3, 2%-10% of the penetration enhancer, and 0%-0.2% of the antioxidant.

Preferably, the composition includes the compound of formula (I) or the pharmaceutically acceptable salt thereof or the pharmaceutically acceptable co-crystal thereof, the white beeswax, the white petrolatum, the light liquid paraffin, and the isopropyl myristate;

preferably, in terms of mass percentage, the composition includes 0.1%-20% of the compound of formula (I) or the pharmaceutically acceptable salt thereof or the pharmaceutically acceptable co-crystal thereof, 10%-30% of the white beeswax, 30%-70% of the white petrolatum, 15%-30% of the light liquid paraffin, and 0.1%-20% of the isopropyl myristate;
preferably, in terms of mass percentage, the composition includes 0.2%-15% of the compound of formula (I) or the pharmaceutically acceptable salt thereof or the pharmaceutically acceptable co-crystal thereof, 15%-25% of the white beeswax, 35%-63% of the white petrolatum, 20%-25% of the light liquid paraffin, and 1%-15% of the isopropyl myristate;
preferably, in terms of mass percentage, the composition includes 0.3%-10% of the compound of formula (I) or the

pharmaceutically acceptable salt thereof or the pharmaceutically acceptable co-crystal thereof, 18%-20% of the white beeswax, 40%-58% of the white petrolatum, 20%-21% of the light liquid paraffin, and 2%-10% of the isopropyl myristate;
preferably, in terms of mass percentage, the composition includes 1% of the compound of formula (I), 18% of the white beeswax, 58% of the white petrolatum, 21% of the light liquid paraffin, and 2% of the isopropyl myristate;
preferably, in terms of mass percentage, the composition includes 10% of the compound of formula (I), 20% of the white beeswax, 40% of the white petrolatum, 20% of the light liquid paraffin, and 10% of the isopropyl myristate;
preferably, the composition includes the compound of formula (I) or the pharmaceutically acceptable salt thereof or the pharmaceutically acceptable co-crystal thereof, the white beeswax, the white petrolatum, the light liquid paraffin, the isopropyl myristate, and the butylated hydroxytoluene (BHT);
preferably, in terms of mass percentage, the composition includes 0.1%-20% of the compound of formula (I) or the pharmaceutically acceptable salt thereof or the pharmaceutically acceptable co-crystal thereof, 10%-30% of the white beeswax, 30%-70% of the white petrolatum, 15%-30% of the light liquid paraffin, 0.1%-20% of the isopropyl myristate, and 0%-2% of the butylated hydroxytoluene (BHT);
preferably, in terms of mass percentage, the composition includes 0.2%-15% of the compound of formula (I) or the pharmaceutically acceptable salt thereof or the pharmaceutically acceptable co-crystal thereof, 15%-25% of the white beeswax, 35%-63% of the white petrolatum, 20%-25% of the light liquid paraffin, 1%-15% of the isopropyl myristate, and 0%-1% of the butylated hydroxytoluene (BHT);
preferably, in terms of mass percentage, the composition includes 0.3%-10% of the compound of formula (I) or the pharmaceutically acceptable salt thereof or the pharmaceutically acceptable co-crystal thereof, 18%-20% of the white beeswax, 40%-58% of the white petrolatum, 20%-21% of the light liquid paraffin, 2%-10% of the isopropyl myristate, and 0%-0.2% of the butylated hydroxytoluene (BHT); and
further preferably, in terms of mass percentage, the composition includes 1% of the compound of formula (I), 18% of the white beeswax, 58% of the white petrolatum, 20.9% of the light liquid paraffin, 2% of the isopropyl myristate, and 0.1% of the butylated hydroxytoluene (BHT).

The present disclosure further provides a method for preparing the composition, where

when the composition does not contain the penetration enhancer and/or the antioxidant, the method includes:

(1) weighing the oil phase substance 1 and the oil phase substance 2, heating to melt in a water bath, and homogenizing to obtain a homogeneous mixture A;
(2) weighing the oil phase substance 3, adding the weighed compound of formula (I) or the pharmaceutically acceptable salt thereof or the pharmaceutically acceptable co-crystal thereof, heating in a water bath, and stirring to obtain a homogeneous mixture B; and
(3) cooling the mixture A, then adding the mixture B into the mixture A, stirring, then homogenizing, and cooling to obtain the composition; and

when the composition contains the penetration enhancer and/or the antioxidant, the method includes:

(1) weighing the oil phase substance 1, the oil phase substance 2, the penetration enhancer and/or the antioxidant, heating to melt in a water bath, and homogenizing to obtain a homogeneous mixture A;
(2) weighing the oil phase substance 3, adding the weighed compound of formula (I) or the pharmaceutically acceptable salt thereof or the pharmaceutically acceptable co-crystal thereof, heating in a water bath, and stirring to obtain a homogeneous mixture B; and
(3) cooling the mixture A, then adding the mixture B into the mixture A, stirring, then homogenizing, and cooling to obtain the composition.

The present disclosure further provides use of the RNA m6A regulator composition or the method in preparation of a formulation for preventing or treating a skin disease caused by RNA m6A methylation, where

preferably, the skin disease includes acanthosis, eczema, ichthyosis, psoriasis, keratosis, systemic lupus erythematosus, a hand-foot syndrome, a hand-foot skin reaction, and dermatitis; and
more preferably, the skin disease includes a hand-foot syndrome and a hand-foot skin reaction.

Preferably, the formulation of the RNA m6A regulator composition includes an ointment, an emulsifiable paste, a gel, a cream, an emulsion, a lotion, a solution, a paste, a film, an oil formulation, or a patch;

preferably, the formulation of the RNA m6A regulator composition is an ointment; and
more preferably, the formulation of the RNA m6A regulator composition is a suspension ointment.

Technical effects

In the RNA m6A regulator composition provided by the present disclosure, the compound of formula (I) has a high exposure amount in the skin and can effectively prevent or treat the skin disease by inhibiting RNA m6A methylation. The composition of the present disclosure has good stability, and exhibits excellent appearance, viscosity, spreadability, physical stability, and pharmacokinetic properties.

## BRIEF DESCRIPTION OF THE DRAWINGS

FIG. 1 describes exemplary results of a cellular m6A level and an NAP1L2 m6A level determined by nanopore sequencing after administration of sorafenib, capecitabine, docetaxel, and osimertinib to HaCaT keratinocytes for 24 hours.

FIG. 2 describes exemplary results of a cellular m6A level and an NAP1L2 m6A level in skin tissues of hind limb toes of mice in Example 3.

FIG. 3 describes exemplary results of an NAP1L2 mRNA expression amount determined by reverse transcription-polymerase chain reaction (RT-PCR) after administration of a recombinant human METTL3 protein and a recombinant human FTO protein or transfection with METTL3 siRNA and FTO siRNA to HaCaT keratinocytes for 24 hours.

FIG. 4 describes exemplary results of mRNA and protein expression amounts of matrix metalloproteinases determined by RT-PCR and Western blot after administration of a recombinant human NAP1L2 protein, sorafenib, capecitabine, docetaxel, and osimertinib or transfection with NAP1L2 siRNA to HaCaT keratinocytes for 24 hours.

FIG. 5 describes exemplary results of mRNA and protein expression amounts of keratinocyte differentiation markers KRT1, KRT10, Loricrin, and Involucrin determined by RT-PCR and Western blot after administration of a recombinant human HBEGF protein and an HBEGF antibody or transfection with METTL3 and NAP1L2 siRNA to HaCaT keratinocytes for 24 hours.

FIG. 6 describes exemplary results of a cellular m6A methylation inhibition level determined by liquid chromatography-mass spectrometry (LC-MS) after administration of a compound of formula (I), nicotinamide, and UZH2 to THP-1 cells for 24 hours.

FIG. 7 describes exemplary results of mRNA expression amounts of keratinocyte differentiation markers KRT1, KRT10, Loricrin, and Involucrin determined by RT-PCR after administration of a recombinant human HBEGF protein and the compound of formula (I) to HaCaT keratinocytes for 24 hours.

FIG. 8 describes exemplary stratum corneum thickness measurement results of paw plantar skin of rats in Example 10.

FIG. 9 describes exemplary histopathological results of epithelial blisters, inflammatory cell infiltration, and skin tissue congestion situations of paw plantar skin of rats after tissue staining in Example 10.

FIG. 10 describes exemplary histopathological scoring results of the epithelial blisters, the inflammatory cell infiltration, and the skin tissue congestion situations of the paw plantar skin of the rats after tissue staining in Example 10.

FIG. 11 describes exemplary results of the skin of hind limb toes of mice in Example 11.

FIG. 12 describes exemplary results of the degree of swelling of hind limb toes of mice in Example 11.

FIG. 13 describes exemplary histopathological results of skin tissues of hind limb toes of mice in Example 11.

FIG. 14 describes exemplary immunohistochemical results of cytokine IL-8 in skin tissues of hind limb toes of mice in capecitabine modeling series groups in Example 11.

FIG. 15 describes exemplary immunohistochemical results of cytokine IL-6 in skin tissues of hind limb toes of mice in docetaxel modeling series groups in Example 11.

FIG. 16 describes exemplary results of the skin of hind limb toes of mice in Example 12.

FIG. 17 describes exemplary results of the degree of swelling of hind limb toes of mice in Example 12.

FIG. 18 describes exemplary histopathological results of skin tissues of hind limb toes of mice in Example 12.

FIG. 19 describes exemplary immunohistochemical results of cytokine IL-1β in skin tissues of hind limb toes of mice in sorafenib modeling series groups in Example 12.

FIG. 20 describes exemplary immunohistochemical results of cytokine IL-1β in skin tissues of hind limb toes of mice in osimertinib modeling series groups in Example 12.

FIG. 21 is an X-ray powder diffraction (XRPD) pattern of a pharmaceutical co-crystal formed by the compound of formula (I) and nicotinamide.

FIG. 22 is an XRPD pattern of a pharmaceutical co-crystal formed by the compound of formula (I) and isonicotinamide.
FIG. 23 is an XRPD pattern of a pharmaceutical co-crystal formed by the compound of formula (I) and L-proline.
FIG. 24 is an XRPD pattern of a pharmaceutical co-crystal formed by the compound of formula (I) and glycolic acid.

## DETAILED DESCRIPTION OF THE EMBODIMENTS

To make the technical solutions and beneficial effects of the present disclosure more apparent and understandable, the following provides a detailed description by the way of listing specific examples. The drawings are not necessarily drawn to scale, and local features may be enlarged or reduced to more clearly show details of the local features. Unless otherwise defined, technical and scientific terms used herein have the same meanings as those in the technical field to which the present disclosure belongs.

The present disclosure provides an RNA m6A regulator composition, where the composition includes: a compound of formula (I) or a pharmaceutically acceptable salt thereof or a pharmaceutically acceptable co-crystal thereof, and an oil phase substance,

(I).

In some embodiments, the pharmaceutically acceptable salt includes hydrochloride, sulfate, phosphate, acetate, methanesulfonate, benzenesulfonate, and p-toluenesulfonate.

In some embodiments, a co-crystal former of the pharmaceutically acceptable co-crystal includes nicotinamide, isonicotinamide, L-proline, and glycolic acid.

In some embodiments, the compound of formula (I) or the pharmaceutically acceptable salt thereof or the pharmaceutically acceptable co-crystal thereof is present in the form of a solid particle.

In some embodiments, a D90 of the solid particle is 0.01-50 μm.

In some embodiments, the D90 of the solid particle is 0.01-20 μm.

In some embodiments, the D90 of the solid particle is 0.01-10 μm.

In some embodiments, the oil phase substance includes an oil phase substance 1, an oil phase substance 2, and an oil phase substance 3.

In some embodiments, the oil phase substance 1 is selected from one or more of lanolin, beeswax, white beeswax, and paraffin.

In some embodiments, the oil phase substance 1 is white beeswax.

In some embodiments, the oil phase substance 2 is selected from one or more of petrolatum and white petrolatum.

In some embodiments, the oil phase substance 2 is white petrolatum.

In some embodiments, the oil phase substance 3 is selected from one or more of light liquid paraffin and vegetable oil.

In some embodiments, the oil phase substance 3 is light liquid paraffin.

In some embodiments, in terms of mass percentage, the composition includes 0.1%-20% of the compound of formula (I) or the pharmaceutically acceptable salt thereof or the pharmaceutically acceptable co-crystal thereof, 10%-30% of the oil phase substance 1, 30%-70% of the oil phase substance 2, and 15%-40% of the oil phase substance 3.

In some embodiments, in terms of mass percentage, the composition includes 0.1%, or 0.2%, or 0.3%, or 0.4%, or 0.5%, or 0.6%, or 0.7%, or 0.8%, or 0.9%, or 1%, or 2%, or 3%, or 4%, or 5%, or 6%, or 7%, or 8%, or 9%, or 10%, or 11%, or 12%, or 13%, or 14%, or 15%, or 16%, or 17%, or 18%, or 19%, or 20% of the compound of formula (I) or the pharmaceutically acceptable salt thereof or the pharmaceutically acceptable co-crystal thereof.

In some embodiments, in terms of mass percentage, the composition includes 10%, or 11%, or 12%, or 13%, or 14%, or 15%, or 16%, or 17%, or 18%, or 19%, or 20%, or 21%, or 22%, or 23%, or 24%, or 25%, or 26%, or 27%, or 28%, or 29%, or 30% of the oil phase substance 1.

In some embodiments, in terms of mass percentage, the composition includes 30%, or 31%, or 32%, or 33%, or 34%, or 35%, or 36%, or 37%, or 38%, or 39%, or 40%, or 41%, or 42%, or 43%, or 44%, or 45%, or 46%, or 47%, or 48%, or 49%, or 50%, or 51%, or 52%, or 53%, or 54%, or 55%, or 56%, or 57%, or 58%, or 59%, or 60%, or 61%, or 62%, or 63%, or

64%, or 65%, or 66%, or 67%, or 68%, or 69%, or 70% of the oil phase substance 2.

In some embodiments, in terms of mass percentage, the composition includes 15%, or 16%, or 17%, or 18%, or 19%, or 20%, or 21%, or 22%, or 23%, or 24%, or 25%, or 26%, or 27%, or 28%, or 29%, or 30%, or 31%, or 32%, or 33%, or 34%, or 35%, or 36%, or 37%, or 38%, or 39%, or 40% of the oil phase substance 3.

In some embodiments, the composition includes 0.2%-15% of the compound of formula (I) or the pharmaceutically acceptable salt thereof or the pharmaceutically acceptable co-crystal thereof, 15%-25% of the oil phase substance 1, 35%-64% of the oil phase substance 2, and 20%-35% of the oil phase substance 3.

In some embodiments, the composition includes 0.3%-10% of the compound of formula (I) or the pharmaceutically acceptable salt thereof or the pharmaceutically acceptable co-crystal thereof, 18%-20% of the oil phase substance 1, 40%-58.7% of the oil phase substance 2, and 22%-30% of the oil phase substance 3.

In some embodiments, the composition includes 0.3% of the compound of formula (I) or the pharmaceutically acceptable salt thereof or the pharmaceutically acceptable co-crystal thereof, 18% of the oil phase substance 1, 58.7% of the oil phase substance 2, and 23% of the oil phase substance 3.

In some embodiments, the composition includes 1% of the compound of formula (I) or the pharmaceutically acceptable salt thereof or the pharmaceutically acceptable co-crystal thereof, 18% of the oil phase substance 1, 58% of the oil phase substance 2, and 23% of the oil phase substance 3.

In some embodiments, the composition includes 3% of the compound of formula (I) or the pharmaceutically acceptable salt thereof or the pharmaceutically acceptable co-crystal thereof, 18% of the oil phase substance 1, 57% of the oil phase substance 2, and 22% of the oil phase substance 3.

In some embodiments, the composition includes 10% of the compound of formula (I) or the pharmaceutically acceptable salt thereof or the pharmaceutically acceptable co-crystal thereof, 20% of the oil phase substance 1, 40% of the oil phase substance 2, and 30% of the oil phase substance 3.

In some embodiments, the composition includes the compound of formula (I) or the pharmaceutically acceptable salt thereof or the pharmaceutically acceptable co-crystal thereof, the white beeswax, the white petrolatum, and the light liquid paraffin.

In some embodiments, in terms of mass percentage, the composition includes 0.1%-20% of the compound of formula (I) or the pharmaceutically acceptable salt thereof or the pharmaceutically acceptable co-crystal thereof, 10%-30% of the white beeswax, 30%-70% of the white petrolatum, and 15%-40% of the light liquid paraffin.

In some embodiments, in terms of mass percentage, the composition includes 0.1%, or 0.2%, or 0.3%, or 0.4%, or 0.5%, or 0.6%, or 0.7%, or 0.8%, or 0.9%, or 1%, or 2%, or 3%, or 4%, or 5%, or 6%, or 7%, or 8%, or 9%, or 10%, or 11%, or 12%, or 13%, or 14%, or 15%, or 16%, or 17%, or 18%, or 19%, or 20% of the compound of formula (I) or the pharmaceutically acceptable salt thereof or the pharmaceutically acceptable co-crystal thereof.

In some embodiments, in terms of mass percentage, the composition includes 10%, or 11%, or 12%, or 13%, or 14%, or 15%, or 16%, or 17%, or 18%, or 19%, or 20%, or 21%, or 22%, or 23%, or 24%, or 25%, or 26%, or 27%, or 28%, or 29%, or 30% of the white beeswax.

In some embodiments, in terms of mass percentage, the composition includes 30%, or 31%, or 32%, or 33%, or 34%, or 35%, or 36%, or 37%, or 38%, or 39%, or 40%, or 41%, or 42%, or 43%, or 44%, or 45%, or 46%, or 47%, or 48%, or 49%, or 50%, or 51%, or 52%, or 53%, or 54%, or 55%, or 56%, or 57%, or 58%, or 59%, or 60%, or 61%, or 62%, or 63%, or 64%, or 65%, or 66%, or 67%, or 68%, or 69%, or 70% of the white petrolatum.

In some embodiments, in terms of mass percentage, the composition includes 15%, or 16%, or 17%, or 18%, or 19%, or 20%, or 21%, or 22%, or 23%, or 24%, or 25%, or 26%, or 27%, or 28%, or 29%, or 30% of the light liquid paraffin.

In some embodiments, in terms of mass percentage, the composition includes 0.2%-15% of the compound of formula (I) or the pharmaceutically acceptable salt thereof or the pharmaceutically acceptable co-crystal thereof, 15%-25% of the white beeswax, 35%-64% of the white petrolatum, and 20%-35% of the light liquid paraffin.

In some embodiments, in terms of mass percentage, the composition includes 0.3%-10% of the compound of formula (I) or the pharmaceutically acceptable salt thereof or the pharmaceutically acceptable co-crystal thereof, 18%-20% of the white beeswax, 40%-58.7% of the white petrolatum, and 22%-30% of the light liquid paraffin.

In some embodiments, the composition includes the compound of formula (I), the white beeswax, the white petrolatum, and the light liquid paraffin.

In some embodiments, in terms of mass percentage, the composition includes 0.1%-20% of the compound of formula (I), 10%-30% of the white beeswax, 30%-70% of the white petrolatum, and 15%-40% of the light liquid paraffin.

In some embodiments, in terms of mass percentage, the composition includes 0.2%-15% of the compound of formula (I), 15%-25% of the white beeswax, 35%-64% of the white petrolatum, and 20%-35% of the light liquid paraffin.

In some embodiments, in terms of mass percentage, the composition includes 0.3%-10% of the compound of formula (I), 18%-20% of the white beeswax, 40%-58.7% of the white petrolatum, and 22%-30% of the light liquid paraffin.

In some embodiments, in terms of mass percentage, the composition includes 0.3% of the compound of formula (I), 18% of the white beeswax, 58.7% of the white petrolatum, and 23% of the light liquid paraffin.

In some embodiments, in terms of mass percentage, the composition includes 1% of the compound of formula (I),

18% of the white beeswax, 58% of the white petrolatum, and 23% of the light liquid paraffin.

In some embodiments, in terms of mass percentage, the composition includes 3% of the compound of formula (I), 18% of the white beeswax, 57% of the white petrolatum, and 22% of the light liquid paraffin.

In some embodiments, in terms of mass percentage, the composition includes 10% of the compound of formula (I), 20% of the white beeswax, 40% of the white petrolatum, and 30% of the light liquid paraffin.

In some embodiments, the composition consists of the compound of formula (I) or the pharmaceutically acceptable salt thereof or the pharmaceutically acceptable co-crystal thereof, the white beeswax, the white petrolatum, and the light liquid paraffin.

In some embodiments, in terms of mass percentage, the composition consists of 0.1%-20% of the compound of formula (I) or the pharmaceutically acceptable salt thereof or the pharmaceutically acceptable co-crystal thereof, 10%-30% of the white beeswax, 30%-70% of the white petrolatum, and 15%-40% of the light liquid paraffin.

In some embodiments, in terms of mass percentage, the composition consists of 0.2%-15% of the compound of formula (I) or the pharmaceutically acceptable salt thereof or the pharmaceutically acceptable co-crystal thereof, 15%-25% of the white beeswax, 35%-64% of the white petrolatum, and 20%-35% of the light liquid paraffin.

In some embodiments, in terms of mass percentage, the composition consists of 0.3%-10% of the compound of formula (I) or the pharmaceutically acceptable salt thereof or the pharmaceutically acceptable co-crystal thereof, 18%-20% of the white beeswax, 40%-58.7% of the white petrolatum, and 22%-30% of the light liquid paraffin.

In some embodiments, the composition consists of the compound of formula (I), the white beeswax, the white petrolatum, and the light liquid paraffin.

In some embodiments, in terms of mass percentage, the composition consists of 0.1%-20% of the compound of formula (I), 10%-30% of the white beeswax, 30%-70% of the white petrolatum, and 15%-40% of the light liquid paraffin.

In some embodiments, in terms of mass percentage, the composition consists of 0.2%-15% of the compound of formula (I), 15%-25% of the white beeswax, 35%-64% of the white petrolatum, and 20%-35% of the light liquid paraffin.

In some embodiments, in terms of mass percentage, the composition consists of 0.3%-10% of the compound of formula (I), 18%-20% of the white beeswax, 40%-58.7% of the white petrolatum, and 22%-30% of the light liquid paraffin.

In some embodiments, in terms of mass percentage, the composition consists of 0.3% of the compound of formula (I), 18% of the white beeswax, 58.7% of the white petrolatum, and 23% of the light liquid paraffin.

In some embodiments, in terms of mass percentage, the composition consists of 1% of the compound of formula (I), 18% of the white beeswax, 58% of the white petrolatum, and 23% of the light liquid paraffin.

In some embodiments, in terms of mass percentage, the composition consists of 3% of the compound of formula (I), 18% of the white beeswax, 57% of the white petrolatum, and 22% of the light liquid paraffin.

In some embodiments, in terms of mass percentage, the composition consists of 10% of the compound of formula (I), 20% of the white beeswax, 40% of the white petrolatum, and 30% of the light liquid paraffin.

In some embodiments, the composition further includes a penetration enhancer.

In some embodiments, the penetration enhancer is selected from one or more of isopropyl myristate, isopropyl palmitate, propylene glycol dipelargonate, diethyl sebacate, laurocapram, and propylene glycol.

In some embodiments, the penetration enhancer is isopropyl myristate.

In some embodiments, the composition further includes an antioxidant.

In some embodiments, the antioxidant is selected from one or more of vitamin E, alkyl gallate, butylated hydroxyanisole (BHA), and butylated hydroxytoluene (BHT).

In some embodiments, the antioxidant is butylated hydroxytoluene (BHT).

In some embodiments, the composition further includes other pharmaceutically acceptable auxiliary materials.

In some embodiments, in terms of mass percentage, the composition includes 0.1%-20% of the compound of formula (I) or the pharmaceutically acceptable salt thereof or the pharmaceutically acceptable co-crystal thereof, 10%-30% of the oil phase substance 1, 30%-70% of the oil phase substance 2, 15%-30% of the oil phase substance 3, 0.1%-20% of the penetration enhancer, and 0%-2% of the antioxidant.

In some embodiments, in terms of mass percentage, the composition includes 0.1%, or 0.2%, or 0.3%, or 0.4%, or 0.5%, or 0.6%, or 0.7%, or 0.8%, or 0.9%, or 1%, or 2%, or 3%, or 4%, or 5%, or 6%, or 7%, or 8%, or 9%, or 10%, or 11%, or 12%, or 13%, or 14%, or 15%, or 16%, or 17%, or 18%, or 19%, or 20% of the compound of formula (I) or the pharmaceutically acceptable salt thereof or the pharmaceutically acceptable co-crystal thereof.

In some embodiments, in terms of mass percentage, the composition includes 10%, or 11%, or 12%, or 13%, or 14%, or 15%, or 16%, or 17%, or 18%, or 19%, or 20%, or 21%, or 22%, or 23%, or 24%, or 25%, or 26%, or 27%, or 28%, or 29%, or 30% of the oil phase substance 1.

In some embodiments, in terms of mass percentage, the composition includes 30%, or 31%, or 32%, or 33%, or 34%, or 35%, or 36%, or 37%, or 38%, or 39%, or 40%, or 41%, or 42%, or 43%, or 44%, or 45%, or 46%, or 47%, or 48%, or 49%, or 50%, or 51%, or 52%, or 53%, or 54%, or 55%, or 56%, or 57%, or 58%, or 59%, or 60%, or 61%, or 62%, or 63%, or 64%, or 65%, or 66%, or 67%, or 68%, or 69%, or 70% of the oil phase substance 2.

In some embodiments, in terms of mass percentage, the composition includes 15%, or 16%, or 17%, or 18%, or

19%, or 20%, or 21%, or 22%, or 23%, or 24%, or 25%, or 26%, or 27%, or 28%, or 29%, or 30% of the oil phase substance 3.

In some embodiments, in terms of mass percentage, the composition includes 0.1%, or 0.2%, or 0.3%, or 0.4%, or 0.5%, or 0.6%, or 0.7%, or 0.8%, or 0.9%, or 1%, or 2%, or 3%, or 4%, or 5%, or 6%, or 7%, or 8%, or 9%, or 10%, or 11%, or 12%, or 13%, or 14%, or 15%, or 16%, or 17%, or 18%, or 19%, or 20% of the penetration enhancer.

In some embodiments, in terms of mass percentage, the composition includes 0%, or 0.1%, or 0.2%, or 0.3%, or 0.4%, or 0.5%, or 0.6%, or 0.7%, or 0.8%, or 0.9%, or 1%, or 1.1%, or 1.2%, or 1.3%, or 1.4%, or 1.5%, or 1.6%, or 1.7%, or 1.8%, or 1.9%, or 2% of the antioxidant.

In some embodiments, in terms of mass percentage, the composition includes 0.2%-15% of the compound of formula (I) or the pharmaceutically acceptable salt thereof or the pharmaceutically acceptable co-crystal thereof, 15%-25% of the oil phase substance 1, 35%-63% of the oil phase substance 2, 20%-25% of the oil phase substance 3, 1%-15% of the penetration enhancer, and 0%-1% of the antioxidant.

In some embodiments, in terms of mass percentage, the composition includes 0.3%-10% of the compound of formula (I) or the pharmaceutically acceptable salt thereof or the pharmaceutically acceptable co-crystal thereof, 18%-20% of the oil phase substance 1, 40%-58% of the oil phase substance 2, 20%-21% of the oil phase substance 3, 2%-10% of the penetration enhancer, and 0%-0.2% of the antioxidant.

In some embodiments, in terms of mass percentage, the composition includes 0.1%-20% of the compound of formula (I), 10%-30% of the oil phase substance 1, 30%-70% of the oil phase substance 2, 15%-30% of the oil phase substance 3, 0.1%-20% of the penetration enhancer, and 0%-2% of the antioxidant.

In some embodiments, in terms of mass percentage, the composition includes 0.2%-15% of the compound of formula (I), 15%-25% of the oil phase substance 1, 35%-63% of the oil phase substance 2, 20%-25% of the oil phase substance 3, 1%-15% of the penetration enhancer, and 0%-1% of the antioxidant.

In some embodiments, in terms of mass percentage, the composition includes 0.3%-10% of the compound of formula (I), 18%-20% of the oil phase substance 1, 40%-58% of the oil phase substance 2, 20%-21% of the oil phase substance 3, 2%-10% of the penetration enhancer, and 0%-0.2% of the antioxidant.

In some embodiments, the composition includes the compound of formula (I) or the pharmaceutically acceptable salt thereof or the pharmaceutically acceptable co-crystal thereof, the white beeswax, the white petrolatum, the light liquid paraffin, and the isopropyl myristate.

In some embodiments, in terms of mass percentage, the composition includes 0.1%-20% of the compound of formula (I) or the pharmaceutically acceptable salt thereof or the pharmaceutically acceptable co-crystal thereof, 10%-30% of the white beeswax, 30%-70% of the white petrolatum, 15%-30% of the light liquid paraffin, and 0.1%-20% of the isopropyl myristate.

In some embodiments, in terms of mass percentage, the composition includes 0.1%, or 0.2%, or 0.3%, or 0.4%, or 0.5%, or 0.6%, or 0.7%, or 0.8%, or 0.9%, or 1%, or 2%, or 3%, or 4%, or 5%, or 6%, or 7%, or 8%, or 9%, or 10%, or 11%, or 12%, or 13%, or 14%, or 15%, or 16%, or 17%, or 18%, or 19%, or 20% of the compound of formula (I) or the pharmaceutically acceptable salt thereof or the pharmaceutically acceptable co-crystal thereof.

In some embodiments, in terms of mass percentage, the composition includes 10%, or 11%, or 12%, or 13%, or 14%, or 15%, or 16%, or 17%, or 18%, or 19%, or 20%, or 21%, or 22%, or 23%, or 24%, or 25%, or 26%, or 27%, or 28%, or 29%, or 30% of the white beeswax.

In some embodiments, in terms of mass percentage, the composition includes 30%, or 31%, or 32%, or 33%, or 34%, or 35%, or 36%, or 37%, or 38%, or 39%, or 40%, or 41%, or 42%, or 43%, or 44%, or 45%, or 46%, or 47%, or 48%, or 49%, or 50%, or 51%, or 52%, or 53%, or 54%, or 55%, or 56%, or 57%, or 58%, or 59%, or 60%, or 61%, or 62%, or 63%, or 64%, or 65%, or 66%, or 67%, or 68%, or 69%, or 70% of the white petrolatum.

In some embodiments, in terms of mass percentage, the composition includes 15%, or 16%, or 17%, or 18%, or 19%, or 20%, or 21%, or 22%, or 23%, or 24%, or 25%, or 26%, or 27%, or 28%, or 29%, or 30% of the light liquid paraffin.

In some embodiments, in terms of mass percentage, the composition includes 0.1%, or 0.2%, or 0.3%, or 0.4%, or 0.5%, or 0.6%, or 0.7%, or 0.8%, or 0.9%, or 1%, or 2%, or 3%, or 4%, or 5%, or 6%, or 7%, or 8%, or 9%, or 10%, or 11%, or 12%, or 13%, or 14%, or 15%, or 16%, or 17%, or 18%, or 19%, or 20% of the isopropyl myristate.

In some embodiments, in terms of mass percentage, the composition includes 0.2%-15% of the compound of formula (I) or the pharmaceutically acceptable salt thereof or the pharmaceutically acceptable co-crystal thereof, 15%-25% of the white beeswax, 35%-63% of the white petrolatum, 20%-25% of the light liquid paraffin, and 1%-15% of the isopropyl myristate.

In some embodiments, in terms of mass percentage, the composition includes 0.3%-10% of the compound of formula (I) or the pharmaceutically acceptable salt thereof or the pharmaceutically acceptable co-crystal thereof, 18%-20% of the white beeswax, 40%-58% of the white petrolatum, 20%-21% of the light liquid paraffin, and 2%-10% of the isopropyl myristate.

In some embodiments, in terms of mass percentage, the composition includes 0.1%-20% of the compound of formula (I), 10%-30% of the white beeswax, 30%-70% of the white petrolatum, 15%-30% of the light liquid paraffin, and

0.1%-20% of the isopropyl myristate.

In some embodiments, in terms of mass percentage, the composition includes 0.2%-15% of the compound of formula (I), 15%-25% of the white beeswax, 35%-63% of the white petrolatum, 20%-25% of the light liquid paraffin, and 1%-15% of the isopropyl myristate.

In some embodiments, in terms of mass percentage, the composition includes 0.3%-10% of the compound of formula (I), 18%-20% of the white beeswax, 40%-58% of the white petrolatum, 20%-21% of the light liquid paraffin, and 2%-10% of the isopropyl myristate.

In some embodiments, in terms of mass percentage, the composition includes 1% of the compound of formula (I), 18% of the white beeswax, 58% of the white petrolatum, 21% of the light liquid paraffin, and 2% of the isopropyl myristate.

In some embodiments, in terms of mass percentage, the composition includes 10% of the compound of formula (I), 20% of the white beeswax, 40% of the white petrolatum, 20% of the light liquid paraffin, and 10% of the isopropyl myristate.

In some embodiments, the composition consists of the compound of formula (I) or the pharmaceutically acceptable salt thereof or the pharmaceutically acceptable co-crystal thereof, the white beeswax, the white petrolatum, the light liquid paraffin, and the isopropyl myristate.

In some embodiments, in terms of mass percentage, the composition consists of 0.1%-20% of the compound of formula (I) or the pharmaceutically acceptable salt thereof or the pharmaceutically acceptable co-crystal thereof, 10%-30% of the white beeswax, 30%-70% of the white petrolatum, 15%-30% of the light liquid paraffin, and 0.1%-20% of the isopropyl myristate.

In some embodiments, in terms of mass percentage, the composition consists of 0.2%-15% of the compound of formula (I) or the pharmaceutically acceptable salt thereof or the pharmaceutically acceptable co-crystal thereof, 15%-25% of the white beeswax, 35%-63% of the white petrolatum, 20%-25% of the light liquid paraffin, and 1%-15% of the isopropyl myristate.

In some embodiments, in terms of mass percentage, the composition consists of 0.3%-10% of the compound of formula (I) or the pharmaceutically acceptable salt thereof or the pharmaceutically acceptable co-crystal thereof, 18%-20% of the white beeswax, 40%-58% of the white petrolatum, 20%-21% of the light liquid paraffin, and 2%-10% of the isopropyl myristate.

In some embodiments, in terms of mass percentage, the composition consists of 0.1%-20% of the compound of formula (I), 10%-30% of the white beeswax, 30%-70% of the white petrolatum, 15%-30% of the light liquid paraffin, and 0.1%-20% of the isopropyl myristate.

In some embodiments, in terms of mass percentage, the composition consists of 0.2%-15% of the compound of formula (I), 15%-25% of the white beeswax, 35%-63% of the white petrolatum, 20%-25% of the light liquid paraffin, and 1%-15% of the isopropyl myristate.

In some embodiments, in terms of mass percentage, the composition consists of 0.3%-10% of the compound of formula (I), 18%-20% of the white beeswax, 40%-58% of the white petrolatum, 20%-21% of the light liquid paraffin, and 2%-10% of the isopropyl myristate.

In some embodiments, in terms of mass percentage, the composition consists of 1% of the compound of formula (I), 18% of the white beeswax, 58% of the white petrolatum, 21% of the light liquid paraffin, and 2% of the isopropyl myristate.

In some embodiments, in terms of mass percentage, the composition consists of 10% of the compound of formula (I), 20% of the white beeswax, 40% of the white petrolatum, 20% of the light liquid paraffin, and 10% of the isopropyl myristate.

In some embodiments, the composition includes the compound of formula (I) or the pharmaceutically acceptable salt thereof or the pharmaceutically acceptable co-crystal thereof, the white beeswax, the white petrolatum, the light liquid paraffin, the isopropyl myristate, and the butylated hydroxytoluene (BHT).

In some embodiments, in terms of mass percentage, the composition includes 0.1%-20% of the compound of formula (I) or the pharmaceutically acceptable salt thereof or the pharmaceutically acceptable co-crystal thereof, 10%-30% of the white beeswax, 30%-70% of the white petrolatum, 15%-30% of the light liquid paraffin, 0.1%-20% of the isopropyl myristate, and 0%-2% of the butylated hydroxytoluene (BHT).

In some embodiments, in terms of mass percentage, the composition includes 0.1%, or 0.2%, or 0.3%, or 0.4%, or 0.5%, or 0.6%, or 0.7%, or 0.8%, or 0.9%, or 1%, or 2%, or 3%, or 4%, or 5%, or 6%, or 7%, or 8%, or 9%, or 10%, or 11%, or 12%, or 13%, or 14%, or 15%, or 16%, or 17%, or 18%, or 19%, or 20% of the compound of formula (I) or the pharmaceutically acceptable salt thereof or the pharmaceutically acceptable co-crystal thereof.

In some embodiments, in terms of mass percentage, the composition includes 10%, or 11%, or 12%, or 13%, or 14%, or 15%, or 16%, or 17%, or 18%, or 19%, or 20%, or 21%, or 22%, or 23%, or 24%, or 25%, or 26%, or 27%, or 28%, or 29%, or 30% of the white beeswax.

In some embodiments, in terms of mass percentage, the composition includes 30%, or 31%, or 32%, or 33%, or 34%, or 35%, or 36%, or 37%, or 38%, or 39%, or 40%, or 41%, or 42%, or 43%, or 44%, or 45%, or 46%, or 47%, or 48%, or

49%, or 50%, or 51%, or 52%, or 53%, or 54%, or 55%, or 56%, or 57%, or 58%, or 59%, or 60%, or 61%, or 62%, or 63%, or 64%, or 65%, or 66%, or 67%, or 68%, or 69%, or 70% of the white petrolatum.

In some embodiments, in terms of mass percentage, the composition includes 15%, or 16%, or 17%, or 18%, or 19%, or 20%, or 21%, or 22%, or 23%, or 24%, or 25%, or 26%, or 27%, or 28%, or 29%, or 30% of the light liquid paraffin.

In some embodiments, in terms of mass percentage, the composition includes 0.1%, or 0.2%, or 0.3%, or 0.4%, or 0.5%, or 0.6%, or 0.7%, or 0.8%, or 0.9%, or 1%, or 2%, or 3%, or 4%, or 5%, or 6%, or 7%, or 8%, or 9%, or 10%, or 11%, or 12%, or 13%, or 14%, or 15%, or 16%, or 17%, or 18%, or 19%, or 20% of the isopropyl myristate.

In some embodiments, in terms of mass percentage, the composition includes 0%, or 0.1%, or 0.2%, or 0.3%, or 0.4%, or 0.5%, or 0.6%, or 0.7%, or 0.8%, or 0.9%, or 1%, or 1.1%, or 1.2%, or 1.3%, or 1.4%, or 1.5%, or 1.6%, or 1.7%, or 1.8%, or 1.9%, or 2% of the butylated hydroxytoluene (BHT).

In some embodiments, in terms of mass percentage, the composition includes 0.2%-15% of the compound of formula (I) or the pharmaceutically acceptable salt thereof or the pharmaceutically acceptable co-crystal thereof, 15%-25% of the white beeswax, 35%-63% of the white petrolatum, 20%-25% of the light liquid paraffin, 1%-15% of the isopropyl myristate, and 0%-1% of the butylated hydroxytoluene (BHT).

In some embodiments, in terms of mass percentage, the composition includes 0.3%-10% of the compound of formula (I) or the pharmaceutically acceptable salt thereof or the pharmaceutically acceptable co-crystal thereof, 18%-20% of the white beeswax, 40%-58% of the white petrolatum, 20%-21% of the light liquid paraffin, 2%-10% of the isopropyl myristate, and 0%-0.2% of the butylated hydroxytoluene (BHT).

In some embodiments, in terms of mass percentage, the composition includes 0.1%-20% of the compound of formula (I), 10%-30% of the white beeswax, 30%-70% of the white petrolatum, 15%-30% of the light liquid paraffin, 0.1%-20% of the isopropyl myristate, and 0%-2% of the butylated hydroxytoluene (BHT).

In some embodiments, in terms of mass percentage, the composition includes 0.2%-15% of the compound of formula (I), 15%-25% of the white beeswax, 35%-63% of the white petrolatum, 20%-25% of the light liquid paraffin, 1%-15% of the isopropyl myristate, and 0%-1% of the butylated hydroxytoluene (BHT).

In some embodiments, in terms of mass percentage, the composition includes 0.3%-10% of the compound of formula (I), 18%-20% of the white beeswax, 40%-58% of the white petrolatum, 20%-21% of the light liquid paraffin, 2%-10% of the isopropyl myristate, and 0%-0.2% of the butylated hydroxytoluene (BHT).

In some embodiments, in terms of mass percentage, the composition includes 1% of the compound of formula (I), 18% of the white beeswax, 58% of the white petrolatum, 20.9% of the light liquid paraffin, 2% of the isopropyl myristate, and 0.1% of the butylated hydroxytoluene (BHT).

In some embodiments, the composition consists of the compound of formula (I) or the pharmaceutically acceptable salt thereof or the pharmaceutically acceptable co-crystal thereof, the white beeswax, the white petrolatum, the light liquid paraffin, the isopropyl myristate, and the butylated hydroxytoluene (BHT).

In some embodiments, in terms of mass percentage, the composition consists of 0.1%-20% of the compound of formula (I) or the pharmaceutically acceptable salt thereof or the pharmaceutically acceptable co-crystal thereof, 10%-30% of the white beeswax, 30%-70% of the white petrolatum, 15%-30% of the light liquid paraffin, 0.1%-20% of the isopropyl myristate, and 0%-2% of the butylated hydroxytoluene (BHT).

In some embodiments, in terms of mass percentage, the composition consists of 0.2%-15% of the compound of formula (I) or the pharmaceutically acceptable salt thereof or the pharmaceutically acceptable co-crystal thereof, 15%-25% of the white beeswax, 35%-63% of the white petrolatum, 20%-25% of the light liquid paraffin, 1%-15% of the isopropyl myristate, and 0%-1% of the butylated hydroxytoluene (BHT).

In some embodiments, in terms of mass percentage, the composition consists of 0.3%-10% of the compound of formula (I) or the pharmaceutically acceptable salt thereof or the pharmaceutically acceptable co-crystal thereof, 18%-20% of the white beeswax, 40%-58% of the white petrolatum, 20%-21% of the light liquid paraffin, 2%-10% of the isopropyl myristate, and 0%-0.2% of the butylated hydroxytoluene (BHT).

In some embodiments, in terms of mass percentage, the composition consists of 0.1%-20% of the compound of formula (I), 10%-30% of the white beeswax, 30%-70% of the white petrolatum, 15%-30% of the light liquid paraffin, 0.1%-20% of the isopropyl myristate, and 0%-2% of the butylated hydroxytoluene (BHT).

In some embodiments, in terms of mass percentage, the composition consists of 0.2%-15% of the compound of formula (I), 15%-25% of the white beeswax, 35%-63% of the white petrolatum, 20%-25% of the light liquid paraffin, 1%-15% of the isopropyl myristate, and 0%-1% of the butylated hydroxytoluene (BHT).

In some embodiments, in terms of mass percentage, the composition consists of 0.3%-10% of the compound of formula (I), 18%-20% of the white beeswax, 40%-58% of the white petrolatum, 20%-21% of the light liquid paraffin, 2%-10% of the isopropyl myristate, and 0%-0.2% of the butylated hydroxytoluene (BHT).

In some embodiments, in terms of mass percentage, the composition consists of 1% of the compound of formula (I), 18% of the white beeswax, 58% of the white petrolatum, 20.9% of the light liquid paraffin, 2% of the isopropyl myristate, and 0.1% of the butylated hydroxytoluene (BHT).

The present disclosure further provides a method for preparing the composition, where

when the composition does not contain the penetration enhancer and/or the antioxidant, the method includes:

(1) weighing the oil phase substance 1 and the oil phase substance 2, heating to melt in a water bath, and homogenizing to obtain a homogeneous mixture A;
(2) weighing the oil phase substance 3, adding the weighed compound of formula (I) or the pharmaceutically acceptable salt thereof or the pharmaceutically acceptable co-crystal thereof, heating in a water bath, and stirring to obtain a homogeneous mixture B; and
(3) cooling the mixture A, then adding the mixture B into the mixture A, stirring, then homogenizing, and cooling to obtain the composition; and

when the composition contains the penetration enhancer and/or the antioxidant, the method includes:

(1) weighing the oil phase substance 1, the oil phase substance 2, the penetration enhancer and/or the antioxidant, heating to melt in a water bath, and homogenizing to obtain a homogeneous mixture A;
(2) weighing the oil phase substance 3, adding the weighed compound of formula (I) or the pharmaceutically acceptable salt thereof or the pharmaceutically acceptable co-crystal thereof, heating in a water bath, and stirring to obtain a homogeneous mixture B; and
(3) cooling the mixture A, then adding the mixture B into the mixture A, stirring, then homogenizing, and cooling to obtain the composition.

In some embodiments, in the step (1), the heating in the water bath is heating up to 65-70°C.
In some embodiments, in the step (1), the homogenizing is homogenizing at 350 rpm for 15-20 minutes.
In some embodiments, in the step (2), the heating in the water bath is heating up to 40°C.
In some embodiments, in the step (2), the stirring is stirring at 300-400 rpm for 10 minutes.
In some embodiments, in the step (3), the cooling is cooling to 40°C.
In some embodiments, in the step (3), a speed of a homogenizer is 10,000 rpm, and a homogenizing time is 2 minutes.
In some embodiments, in the step (3), the cooling is cooling to room temperature.
The present disclosure further provides use of the RNA m6A regulator composition or the method in preparation of a formulation for preventing or treating a skin disease caused by RNA m6A methylation.
In some embodiments, the skin disease includes acanthosis, eczema, ichthyosis, psoriasis, keratosis, systemic lupus erythematosus, a hand-foot syndrome, a hand-foot skin reaction, and dermatitis.
In some embodiments, the skin disease includes a hand-foot syndrome and a hand-foot skin reaction.
In some embodiments, the formulation of the RNA m6A regulator composition includes an ointment, an emulsifiable paste, a gel, a cream, an emulsion, a lotion, a solution, a paste, a film, an oil formulation, or a patch.
In some embodiments, the formulation of the RNA m6A regulator composition is an ointment.
In some embodiments, the formulation of the RNA m6A regulator composition is a suspension ointment.

**Example 1: Preparation of the compound of formula (I)**

150°C, 3h
LiOH
DCM, $NH_4OH$
separate by a Chiralpak AD chiral column
compound **1**
compound of formula (I)

Ethyl 3-bromo-2-oxocyclohexane-1-formate (20 mg, 0.08 mmol) and 4-chloroaniline (25 mg, 0.2 mmol) were mixed and then heated to 150°C for a reaction for 3 hours. The reaction solution was cooled to room temperature, diluted with 100 mL of dichloromethane, washed 3 times with 100 mL of 1 N HCl, and washed 1 time with 100 mL of saturated $NaHCO_3$. The organic layer was dried over anhydrous $Na_2SO_4$, concentrated under vacuum, and separated and purified by silica gel column chromatography to obtain ethyl 6-chloro-2,3,4,9-tetrahydro-1H-carbazole-1-formate (16 mg). 10 mg of the ethyl 6-chloro-2,3,4,9-tetrahydro-1H-carbazole-1-formate (0.036 mmol) was weighed and dissolved in 10 mL of ethanol, 2 mL of a 2 M LiOH solution was added, and the mixture was stirred at room temperature for a reaction for 1 hour. After rotary evaporation, the mixture was diluted with 20 mL of water, adjusted to a pH of 2, and then extracted 3 times with

50 mL of dichloromethane. The organic phases were combined, dried, and concentrated, followed by the addition of ammonia water and heating at 60°C for a reaction for 24 hours. The mixture was separated and purified by silica gel column chromatography to obtain 6-chloro-2,3,4,9-tetrahydro-1H-carbazole-1-carboxamide as a white solid (compound 1, 7.6 mg), which was separated by a Chiralpak AD chiral column to obtain (S)-6-chloro-2,3,4,9-tetrahydro-1H-carbazole-carboxamide (the compound of formula (I)). LCMS $[M+H]^+$ of the compound of formula (I): 249. $^1$H-NMR (400 MHz, DMSO-$d_6$): $\delta$ 10.79 (s, 1H), 7.37-7.39 (m, 2H), 7.28 (d, $J$ = 8.0 Hz, 1H), 7.08 (s, 1H), 6.98-7.01 (m, 1H), 3.64-3.67 (m, 1H), 2.58-2.61 (m, 2H), 1.66-2.04 (m, 4H).

**Example 2: Changes in an mRNA methylation level of drug-induced keratinocytes**

$1*10^8$ HaCaT cells were seeded in culture dishes, into which 50 nM drugs (including sorafenib, capecitabine, docetaxel, and osimertinib) were added, respectively. The cells were cultured in an incubator containing 5% $CO_2$ at 37°C for 24 hours and washed with PBS after culture media were removed. Centrifugation was performed, the supernatant was discarded, and then, 400 μL of an RLT cell lysis buffer (QIAGEN, Cat.#79216) was added. A portion of samples were selected, into which 400 μL of 70% ethanol was added. The mixture was evenly stirred and then transferred to an RNeasy mini column, and RNA was extracted using an RNeasy mini kit (QIAGEN, Cat.#74104). 2 μL of an RNA sample was selected and dissolved in 98 μL of a 10 mM Tris-HCl buffer solution, and the absorbance at 260 nm was measured using a Nanodrop (Thermo Fisher Scientific) to determine the RNA concentration.

40 μg of an RNA sample was selected for enrichment of mRNA (PolyA+RNA) using an NEBNext Poly(A) mRNA magnetic isolation module (NEB, Cat.#E7490), and then, a 3' end of the above enriched mRNA (PolyA+RNA) molecule was ligated with reverse transcription adapter RTA using a direct RNA sequencing kit (Oxford Nanopore Technologies, Cat.#SQK-RNA002). A reverse transcription reaction was performed with the mRNA (PolyA+RNA) molecule as a template to synthesize a complementary strand, a terminal end of the reverse transcription adapter RTA was ligated with a sequencing adapter (RNA adapter) to form a final sequencing library, and quantitative detection was performed using a Qubit™ dsDNA HS assay kit (Invitrogen, Cat.#LOT2133187).

After quality inspection of the library, the prepared sequencing library was loaded onto a PromethION flow cell chip (Oxford Nanopore Technologies, Cat.#FLO-MIN106D), and placed in a PromethION sequencer (Oxford Nanopore Technologies) for sequencing by selecting a matched sequencing mode. Basecalling was performed on sequencing data through guppy, and m6A methylation analysis was performed on original fast5 data.

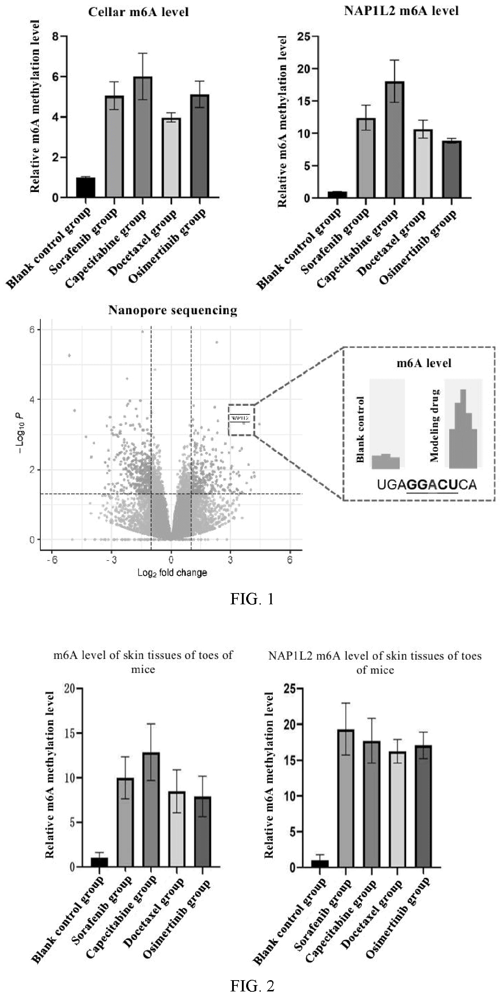

Results are shown in FIG. 1. Compared with a blank control group, the m6A methylation levels of HaCaT cells were increased by 5.05-fold, 6.01-fold, 3.97-fold and 5.13-fold higher than the control group after the addition of the sorafenib, the capecitabine, the docetaxel, and the osimertinib, respectively. This indicates that antitumor drugs (including the sorafenib, the capecitabine, the docetaxel, and the osimertinib) induced the abnormal m6A methylation of mRNA of human keratinocytes. It is further found through bioinformatics analysis that the m6A methylation level of NAP1L2 had a significant difference, mainly showing in the m6A methylation of a UGAGGACUCA fragment.

**Example 3: Changes in an mRNA methylation level in drug-induced mouse models with adverse skin reactions**

After adaptive feeding for 7 days, male ICR mice (5-6 weeks old, with a body weight of approximately 35 g) were randomly divided into groups, with 6 mice in each group, including a blank control group, a sorafenib group, a capecitabine group, a docetaxel group, and an osimertinib group. The groups were respectively administered corresponding modeling drugs by gavage (sorafenib at 100 mg/kg, capecitabine at 200 mg/kg, docetaxel at 25 mg/kg, and osimertinib at 10 mg/kg) once daily. After continuous administration for 30 days, the mice were sacrificed, and skin tissues of hind limb toes of the mice were collected. A small amount of a tissue sample was selected, placed in a mortar containing liquid nitrogen, and ground into powder. A single-phase lysis buffer was added. After being placed at room temperature for 5 minutes, the sample was centrifuged (12,000 rpm) for 5 minutes. 1 mL of the supernatant was collected, 200 μL of chloroform was added, and the mixture was evenly mixed by shaking, placed at room temperature for 15 minutes, and centrifuged (12,000 rpm, 4°C) for 15 minutes. An aqueous phase on an upper layer was sucked, added into an equal volume of isopropanol, placed at -20°C for 1 hour, and then centrifuged (12,000 rpm, 4°C) to discard the supernatant. 400 μL of 70% ethanol was added to a precipitate, the mixture was evenly stirred and then transferred to an RNeasy mini column, and RNA was extracted using an RNeasy mini kit (QIAGEN, Cat.#74104). 2 μL of an RNA sample was selected and dissolved in 98 μL of a 10 mM Tris-HCl buffer solution, and the absorbance at 260 nm was measured using a Nanodrop (Thermo Fisher Scientific) to determine the RNA concentration.

40 μg of an RNA sample was selected for enrichment of mRNA (PolyA+RNA) using an NEBNext Poly(A) mRNA magnetic isolation module (NEB, Cat.#E7490), and then, a 3' end of the above enriched mRNA (PolyA+RNA) molecule was ligated with reverse transcription adapter RTA using a direct RNA sequencing kit (Oxford Nanopore Technologies, Cat.#SQK-RNA002). A reverse transcription reaction was performed with the mRNA (PolyA+RNA) molecule as a

template to synthesize a complementary strand, a terminal end of the reverse transcription adapter RTA was ligated with a sequencing adapter (RNA adapter) to form a final sequencing library, and quantitative detection was performed using a Qubit™ dsDNA HS assay kit (Invitrogen, Cat.#LOT2133187).

After quality inspection of the library, the prepared sequencing library was loaded onto a PromethION flow cell chip (Oxford Nanopore Technologies, Cat.#FLO-MIN106D), and placed in a PromethION sequencer (Oxford Nanopore Technologies) for sequencing by selecting a matched sequencing mode. Basecalling was performed on sequencing data through guppy, and m6A methylation analysis was performed on original fast5 data.

Results are shown in FIG. 2. Compared with a positive control group, the m6A methylation levels of skin tissues of hind limb toes of the mice in the drug modeling groups were significantly increased. The m6A methylation level of NAP1L2 in the drug addition groups had a significant difference from the blank control group.

**Example 4: Regulation of an mRNA expression level of NAP1L2 by m6A**

$1*10^8$ HaCaT cells were seeded in culture dishes, into which a recombinant protein of m6A methyltransferase METTL3 (Abcam, Cat.#ab271611) and a recombinant protein of m6A demethylase FTO (Abcam, Cat.#ab271525) were added, or METTL3 siRNA (sh-METTL3, GeneChem) and FTO siRNA (sh-FTO, GeneChem) were transfected, respectively. The cells were placed in an incubator containing 5% carbon dioxide for culture at 37°C for 24 hours, washed with PBS, and then collected by high-speed centrifugation. Total RNA was extracted using Trizol reagent (Sigma Aldrich). 1 μg of RNA was reverse-transcribed into cDNA using a cDNA reverse transcription kit (Transgene Biotech, Cat.#AT311-03). 1.25 μL of a primer (Beyotime, Cat.#QH18721S), 10 μL of an iTag Universal SYBR Green supermix (Bio-Rad, Cat.#172-5125), and an appropriate amount of DEPC ultrapure water were added to formulate 20 μL of a reaction solution to perform an RT-PCR reaction. Upon completion of the reaction, the reaction solution was subjected to agarose gel electrophoresis to determine the mRNA expression level of NAP1L2.

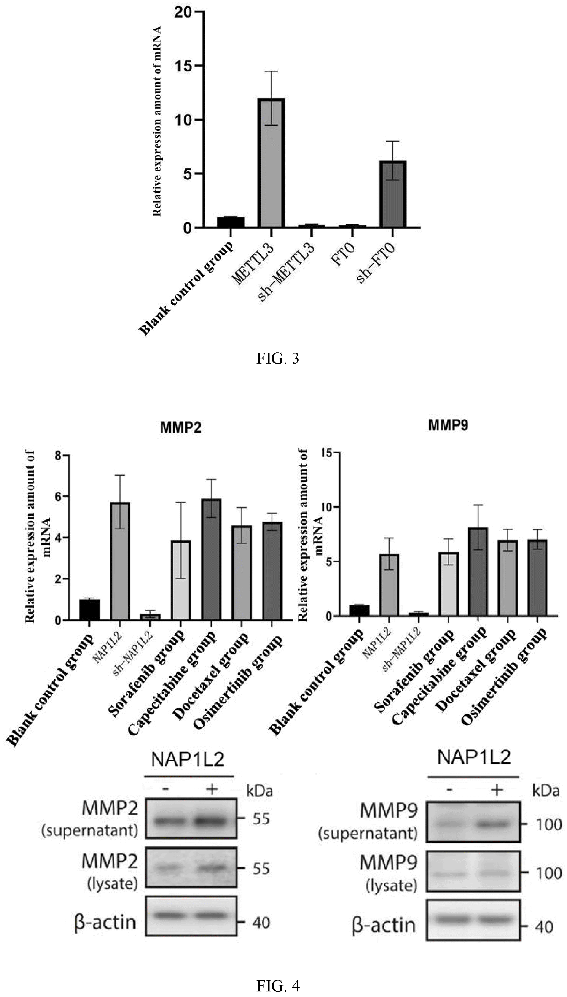

Results are shown in FIG. 3. When the m6A methyltransferase METTL3 was added to the HaCaT keratinocytes or the function of the m6A demethylase FTO was inhibited (via sh-FTO), the mRNA expression level of NAP1L2 was significantly increased; and when the m6A demethylase FTO was added to the HaCaT keratinocytes or a function of the m6A methyltransferase METTL3 was inhibited (via sh-METTL3), the mRNA expression level of NAP1L2 was significantly decreased. This indicates that m6A methylation regulated the mRNA expression level of NAP1L2.

**Example 5: Regulation of the expression of matrix metalloproteinases (MMPs) in human HaCaT keratinocytes by NAP1L2**

$1*10^8$ HaCaT cells were seeded in culture dishes, into which a recombinant human NAP1L2 protein (Abcam, Cat.#ab117213), sorafenib, capecitabine, docetaxel, and osimertinib were added, or NAP1L2 siRNA (sh-NAP1L2, GeneChem) was transfected, respectively. The cells were placed in an incubator containing 5% carbon dioxide for culture at 37°C for 24 hours, washed with PBS, and then collected by high-speed centrifugation. An RLT lysis buffer was added to the above collected cells, the mixture was shaken at 4°C for half an hour and centrifuged to collect the supernatant, and the expression of matrix metalloproteinases (MMPs) was measured by Western blot.

Total RNA was extracted from the above collected cells using Trizol reagent (Sigma Aldrich). 1 μg of RNA was reverse-transcribed into cDNA using a cDNA reverse transcription kit (Transgene Biotech, Cat.#AT311-03). 1.25 μL of a primer (SinoBiological, Cat.#HP100168&HP100367), 10 μL of an iTag Universal SYBR Green supermix (Bio-Rad, Cat.#172-5125), and an appropriate amount of DEPC ultrapure water were added to formulate 20 μL of a reaction solution to perform an RT-PCR reaction. Upon completion of the reaction, the reaction solution was subjected to agarose gel electrophoresis to determine the mRNA expression level of the matrix metalloproteinases.

Results are shown in FIG. 4. After the recombinant NAP1L2 protein was added to the HaCaT keratinocytes, the mRNA and protein expression amounts of matrix metalloproteinases MMP2 and MMP9 were significantly increased; and when NAP1L2 siRNA was added to the HaCaT keratinocytes to inhibit the expression of NAP1L2, the mRNA and protein expression amounts of the matrix metalloproteinases MMP2 and MMP9 were significantly decreased. Numerous studies have confirmed that abnormal expression of matrix metalloproteinases is closely associated with various skin-related diseases (Kumper M. et al., Am. J. Physiol. Cell. Physiol. 2022, 323(4): 1290-1303).

**Example 6: Regulation of the expression of human keratinocyte differentiation markers by m6A**

$1*10^8$ HaCaT cells were seeded in culture dishes, into which a recombinant HBEGF protein (Thermo Fisher Scientific, Cat.#100-47-1mg) was added. The cells were placed in an incubator containing 5% carbon dioxide for culture at 37°C for 24 hours, into which an HBEGF antibody (Invitrogen, Cat.#406316) was added, or METTL3 siRNA (sh-METTL3, GeneChem) and NAP1L2 siRNA (sh-NAP1L2, GeneChem) were transfected. The cells were placed in an incubator containing 5% carbon dioxide for culture at 37°C for 24 hours, washed with PBS, and then collected by high-speed

centrifugation. An RLT lysis buffer was added to the above collected cells, the mixture was shaken at 4°C for half an hour and centrifuged to collect the supernatant, and the protein expression amounts of keratinocyte differentiation markers KRT1, KRT10, Loricrin, and Involucrin were measured by Western blot.

Total RNA was extracted using Trizol reagent (Sigma Aldrich). 1 μg of RNA was reverse-transcribed into cDNA using a cDNA reverse transcription kit (Transgene Biotech, Cat.#AT311-03). 1.25 μL of a primer (with a primer sequence shown in Table 2), 10 μL of an iTag Universal SYBR Green supermix (Bio-Rad, Cat.#172-5125), and an appropriate amount of DEPC ultrapure water were added to formulate 20 μL of a reaction solution to perform an RT-PCR reaction. Upon completion of the reaction, the reaction solution was subjected to agarose gel electrophoresis to determine the mRNA expression level of the keratinocyte differentiation markers KRT1, KRT10, Loricrin, and Involucrin.

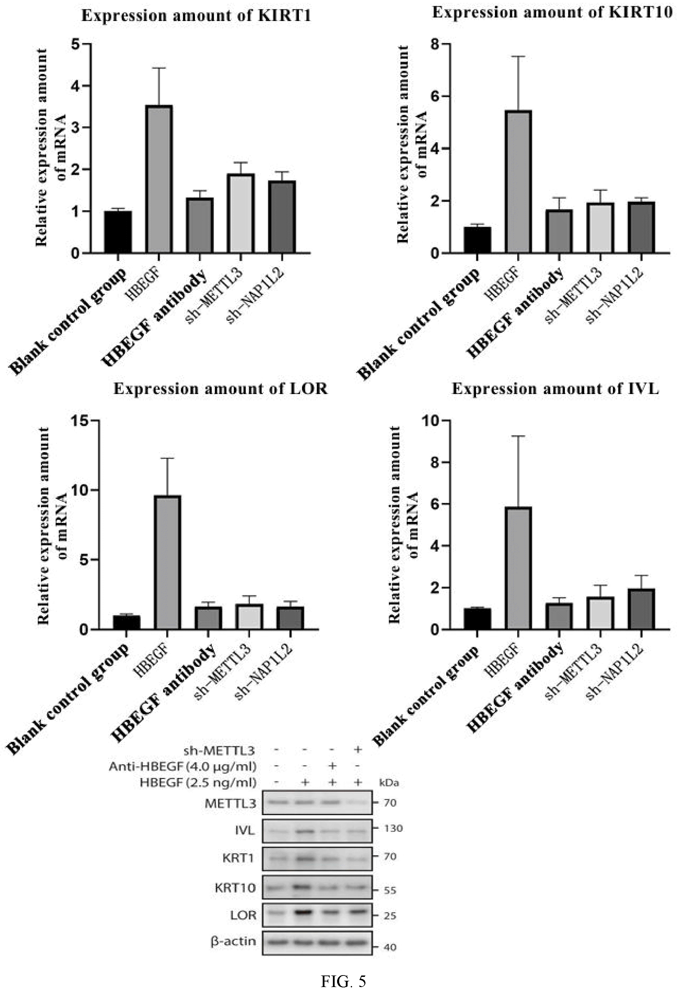

Results are shown in FIG. 5. After the recombinant HBEGF protein was added to the HaCaT keratinocytes, the mRNA and protein expression amounts of the keratinocyte differentiation markers KRT1, KRT10, Loricrin, and Involucrin were significantly increased, indicating that the HaCaT keratinocytes were in a highly differentiated state; however, when the HBEGF antibody or METTL3 or NAP1L2 siRNA was added to inhibit an m6A methylation function in the cells, the mRNA and protein expression amounts of the keratinocyte differentiation markers KRT1, KRT10, Loricrin, and Involucrin are significantly decreased. This indicates that m6A regulated the expression of the keratinocyte differentiation markers, thereby regulating the differentiation of the keratinocytes.

**Example 7: Impacts of compounds on an m6A methylation level of THP-1 cells**

$1*10^8$ THP-1 cells were seeded in culture dishes, into which 4 nM and 400 nM compound of formula (I), 4 nM and 4 μM nicotinamide, and 5 μM m6A methyltransferase inhibitor UZH2 were added, respectively. The cells were cultured in an incubator containing 5% $CO_2$ at 37°C for 24 hours. After discarding culture media and washing with PBS, the cells were centrifuged, and the supernatant was discarded. 400 μL of an RLT cell lysis buffer was added, and the cells were stored at -80°C. A portion of samples were selected, into which 400 μL of 70% ethanol was added and evenly stirred. 700 μL of a sample was transferred to an RNeasy centrifugation column and centrifuged for 15 seconds (8,000 g, 25°C). Then, 700 μL of an RW1 buffer solution was added and centrifuged for 15 seconds (8,000 g, 25°C), followed by the addition of 500 μL of an RPE buffer solution and centrifugation for 16 seconds (8,000 g, 25°C). The above steps were repeated twice, followed by centrifugation for 2 minutes to completely remove the eluent. 30 μL of nuclease-free water was added to a chromatographic column, and the cells were incubated for 5 minutes and then centrifuged for 2 minutes (12,000 g, 25°C). The steps were repeated 3 times. 2 μL of an RNA sample was selected and dissolved in 98 μL of a 10 mM Tris buffer solution, and the absorbance at 260 nm was measured using a Nanodrop to determine the RNA concentration.

50 μg of total RNA was selected and dissolved in 100 μL of nuclease-free water. Oligo (dT) magnetic beads were resuspended, 50 μL of the magnetic beads were transferred to a 1.5 mL test tube, 500 μL of a binding buffer solution was added, the mixture was allowed to stand, and the supernatant was removed. 100 μL of an RNA sample was added to 200 μL of a magnetic bead suspension for mixing, the mixture was stirred and incubated in a Thermomixer at 25°C for 5 minutes, the supernatant was discarded, and then 200 μL of a washing buffer solution was added. The steps were repeated 2 times, and then centrifugation was performed for 10 seconds (2,000 g, 25°C). 50 μL of an elution buffer solution was added and evenly mixed, the mixture was heated to 75°C, the supernatant was transferred to a new 1.5 mL test tube and purified using an RNA Clean & Concentrator kit, and the RNA concentration was determined.

20 μL (approximately 200 ng) of an mRNA sample and 20 μL of a Nuclease P1 digestion premix (including 0.5 μL of 2 unit/μL Nuclease P1, 0.4 μL of 5 M NaCl, 2 μL of 0.1 M $ZnCl_2$, and 17.1 μL of PCR-grade water) were added into each test tube, and the mixture was stirred and incubated in a Thermomixer at 37°C for 2 hours. 2 μL of a 2 M $NH_4HCO_3$ solution and 1 unit of alkaline phosphatase were added, and the mixture was stirred and incubated in a Thermomixer at 37°C for 2 hours. 1 μL of a 1.2 M HCl neutralization solution was added, and centrifugation was performed for 30 minutes (16,000 g, 4°C). 20 μL of the supernatant was collected, an m6A methylation fragment was analyzed by LC-MS, and the m6A inhibition rate was calculated.

m6A inhibition rate (%)=(measured ion peak area-blank control)/(positive ion peak area-blank control)*100.

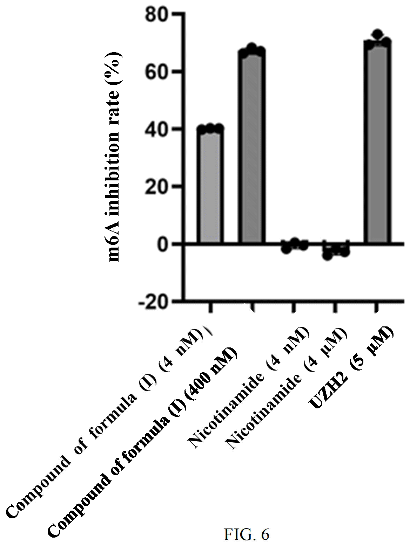

Results are shown in FIG. 6. The compound of formula (I) can significantly inhibit the m6A methylation level of mRNA of the cells in a concentration-dependent manner. At a concentration of 400 nM, the compound of formula (I) exhibited m6A inhibition activity comparable to that of the m6A methyltransferase inhibitor UZH2 at a concentration of 5 μM. However, nicotinamide exhibits no inhibitory effect on the m6A methylation level of mRNA of the cells at either a low concentration (4 nM) or a high concentration (4 μM).

**Example 8: Impacts of compounds on an m6A methylation level of human keratinocytes**

HaCaT cells were cultured in DMEM culture medium containing 10% fetal bovine serum, 100 U/mL penicillin, and 100 μg/mL streptomycin. Culture dishes were placed in an incubator containing 5% $CO_2$ for culture at 37°C for 24 hours. An equal volume of DMSO was added in control group 1, 50 nM sorafenib, capecitabine, docetaxel, and osimertinib were respectively added in control groups A, B, C, and D, and 400 nM test compounds or nicotinamide were respectively added in sample groups A1, A2, B1, B2, C1, C2, D1, and D2 after the addition of 50 nM corresponding compounds. The cells were continuously cultured for 24 hours, washed with PBS, and then collected by high-speed centrifugation. Total RNA was extracted using Trizol reagent (Sigma Aldrich), and RNA m6A methylation of a sample was quantitatively detected using an EpiQuik m6A RNA methylation quantification kit. The concentrations of positive control samples for a standard curve were 0.01 ng/μl, 0.02 ng/μl, 0.05 ng/μl, 0.1 ng/μl, 0.2 ng/μl, and 0.5 ng/μl. The absorbance at 450 nm was read using a microplate reader (Tecan GENios). A quantitative calculation formula for m6A is as follows:

$$\text{m6A (ng)=(absorbance of sample well–absorbance of background well)/slope of standard curve}$$

$$\text{m6A (\%)=m6A (ng)/RNA amount of sample (ng)*100\%.}$$

Results are shown in Table 1. The treatment with the sorafenib, the capecitabine, the docetaxel, and the osimertinib can significantly increase the m6A methylation level of human HaCaT keratinocytes (as shown in Table 1, a specific calculation method is: fold change = amount of m6A determined/amount of m6A determined in control 1). The compound of formula (I) can effectively inhibit an abnormal increase in the RNA m6A level of human keratinocytes caused by chemotherapeutic drugs and multikinase inhibitors.

**Table 1: Fold change in m6A methylation level relative to control group 1**

| Group | Drug/compound | m6A fold change | Group | Drug/compound | m6A fold change |
|---|---|---|---|---|---|
| A | Sorafenib | 4.5 | B | Capecitabine | 6.7 |
| A1 | Compound of formula (I) | 1.4 | B1 | Compound of formula (I) | 1.1 |
| A2 | Nicotinamide | 4.7 | B2 | Nicotinamide | 4.5 |
| C | Docetaxel | 5.6 | D | Osimertinib | 4.8 |
| C1 | Compound of formula (I) | 1.0 | D1 | Compound of formula (I) | 1.2 |
| C2 | Nicotinamide | 5.4 | D2 | Nicotinamide | 5.0 |

**Example 9**: **Inhibitory effects of compounds on the differentiation of HaCaT keratinocytes**

Human HaCaT keratinocytes were cultured in DMEM culture medium containing 10% fetal bovine serum, 100 U/mL penicillin, and 100 μg/mL streptomycin. The cells were seeded into a 96-well plate at a density of $2*10^6$ cells per square centimeter and placed in an incubator containing 5% carbon dioxide for culture at 37°C for 24 hours. An equal volume of DMSO was added into control well 1, a 2.5 ng/mL recombinant human HBEGF protein (Abcam, ab205523) was added into control well 2, and a 2.5 ng/mL recombinant human HBEGF protein and corresponding concentrations of test compounds were added into sample wells. The cells were continuously cultured for 24 hours, washed with PBS, and then collected by high-speed centrifugation. Total RNA was extracted using Trizol reagent (Sigma Aldrich). 1 μg of RNA was reverse-transcribed into cDNA using a cDNA reverse transcription kit (Transgene Biotech, AT311-03). 1.25 μL of a primer (with a primer sequence shown in Table 2), 10 μL of an iTag Universal SYBR Green supermix (Bio-Rad, 172-5125), and an appropriate amount of DEPC ultrapure water were added to formulate 20 μL of a reaction solution to perform an RT-PCR reaction. Upon completion of the reaction, the reaction solution was subjected to agarose gel electrophoresis to determine the mRNA expression level of keratinocyte differentiation markers KRT1, KRT10, Loricrin, and Involucrin.

**Table 2: Sequence information of some primers**

| | | |
|---|---|---|
| KRT1 | Forward primer | 5'-GTCAAGTCCTCTGGTGGCAG-3' |
| | Reverse primer | 5'-AAGGCTGGGACAAATCGACC-3' |

(continued)

| | | |
|---|---|---|
| KRT10 | Forward primer | 5'-CCCGGGTGTTGATCTGACTC-3' |
| | Reverse primer | 5'-CCAGGCTTCAGCATCTTTGC-3' |
| Loricrin | Forward primer | 5'-TCATGATGCTACCCGAGGTTTG-3' |
| | Reverse primer | 5'-CAGAACTAGATGCAGCCGGAGA-3' |
| Involucrin | Forward primer | 5'-TCGCTCCTCAAGACTGTTCCTCC-3' |
| | Reverse primer | 5'-CAGGCAGTCCCTTTACAGCA-3' |

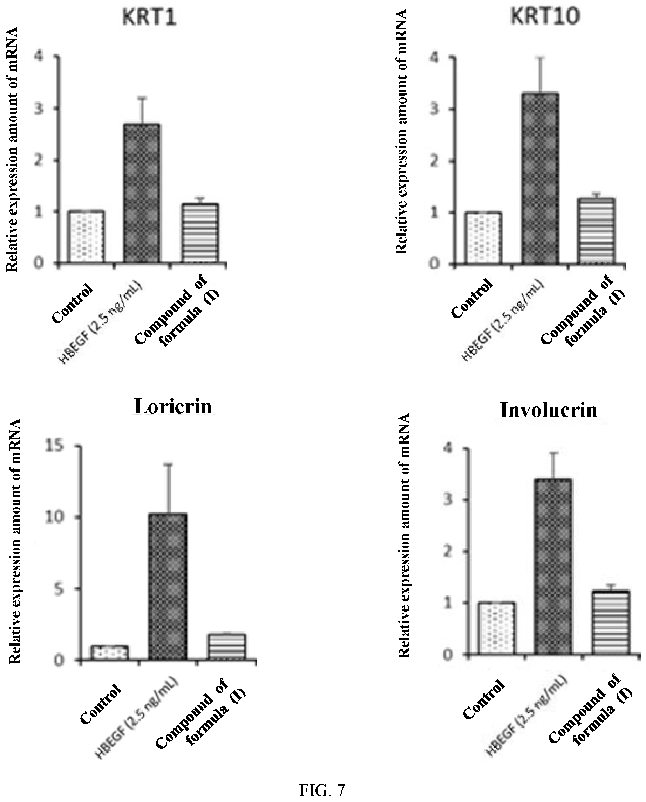

As shown in FIG. 7, after the recombinant human HBEGF protein was added for induction, the mRNA expression level of the HaCaT differentiation markers KRT1, KRT10, Loricrin, and Involucrin were significantly increased. However, after the compound of formula (I) was added, the mRNA expression level of the HaCaT differentiation markers KRT1, KRT10, Loricrin, and Involucrin were significantly inhibited.

**Example 10: Inhibitory effects of compounds on the differentiation of keratinocytes in rat models with hand-foot skin reaction**

After adaptive feeding for one week, SD rats (with a body weight of approximately 200 g) were randomly divided into groups with balanced body weight, with 12 rats in each group. Modeling drugs (including sorafenib, erlotinib, afatinib, and osimertinib) were respectively dissolved in a solution containing 5% of DMSO, 45% of PEG400, and 50% of $H_2O$, and the modeling drugs were diluted to required concentrations and then administered once daily by gavage at doses shown in Table 3. After administration by gavage for 1 hour, 0.05 g of ointments containing different mass proportions of test compounds were evenly applied to left paws of the rats, a blank matrix ointment was applied to right paws of the rats to serve as a self-control, and no ointment was applied to the rats in a blank control group. After drug application, four limbs were continuously immobilized for 2 hours, then wiped with clean water to remove the residual drugs, removed from immobilization, and restored to free movement. The modeling drugs, the blank matrix ointment, and the ointments containing the test compounds were all administered once daily. After continuous administration for 30 days, the rats were euthanized, and paw plantar skin tissues were collected, fixed in 10% neutral formalin, cut into 5 μm sections, dehydrated, and then embedded in paraffin. The ointments for the test compounds were prepared by mixing the compounds, white beeswax, white petrolatum, and light liquid paraffin in specific ratios (weight ratios of 3 ointments were respectively 1:18:58:23, 3:18:57:22, and 10:20:40:30). The blank matrix ointment was prepared by mixing white beeswax, white petrolatum, and light liquid paraffin in a specific ratio (weight ratio of 18:60:22).

Tissue staining: After dewaxing and rehydration, the paw plantar skin tissue sections of the above rats were stained in a hematoxylin solution for several minutes, cleaned, and placed in 1% acid alcohol for soaking until the sections faded to light blue-red. After cleaning with running water for 5 minutes, the sections were stained with eosin for 2-3 minutes, cleaned to remove excess dyes, dehydrated, then permeabilized by adding xylene for several minutes, and sealed and fixed with a neutral resin. The stratum corneum was observed under an optical microscope, and the thickness of the epidermal stratum corneum was measured using Dmetrix software.

Immunohistochemical staining: After dewaxing and hydration, the paw plantar skin tissue sections of the above rats were incubated with 3% $H_2O_2$ at room temperature for 30 minutes and blocked with 10% goat serum for 30 minutes after antigen retrieval. A KRT1 antibody (Abcam, ab93652) and a Loricrin antibody (Abcam, ab183646) were added dropwise, the sections were incubated overnight at 4°C, and an HRP secondary antibody (ZSGB-BIO, PV-6001) and a DAB kit (ZSG-BIO, ZLI9017) were added for color development. The sections were re-stained with hematoxylin, cleaned, and sealed and fixed with a neutral resin, and the expression amounts of KRT1 and Loricrin were observed under an optical microscope.

Criteria for determining successful modeling of the above rat models are as follows: (i) symptoms, such as erythema, swelling, desquamation, ulcers, or blisters, occur at paw sites; and/or (ii) the stratum corneum thickness in tissue staining is significantly higher than that of normal rats; and/or (iii) markers, such as KRT1, KRT5, and Loricrin, are significant increased. An incidence rate calculation method involves a proportion of animals in each group meeting the above criteria for determining successful modeling, that is, incidence rate = (number of successfully modeled rats/total number of rats in the group)*100%.

Pathological scoring criteria for tissue staining of the above rats are as follows: no blisters, recorded as 0 point; the number of blisters being 1-3, recorded as 1 point; the number of blisters being 4-6, recorded as 2 points; the number of blisters being 7-9, recorded as 3 points; and the number of blisters being more than 10, recorded as 4 points; an inflammation area accounting for less than 10% of a total section area, recorded as 0 point; the inflammation area accounting for 10-25% of the total section area, recorded as 1 point; the inflammation area accounting for 25-50% of the

total section area, recorded as 2 points; the inflammation area accounting for 50-75% of the total section area, recorded as 3 points; and the inflammation area accounting for more than 75% of the total section area, recorded as 4 points; an congestion area accounting for less than 10% of a total section area, recorded as 0 point; the congestion area accounting for 10-25% of the total section area, recorded as 1 point; the congestion area accounting for 25-50% of the total section area, recorded as 2 points; the congestion area accounting for 50-75% of the total section area, recorded as 3 points; and the congestion area accounting for more than 75% of the total section area, recorded as 4 points. Scoring was performed using a double-blind scoring system. After completion of statistics, data for each group was presented in the form of mean + SEM (N=12).

**Table 3: Doses of drugs used in rat models and experimental results**

| Serial number | Modeling drug | Dose | Compound and its mass proportion in an ointment | | Incidence rate | |
|---|---|---|---|---|---|---|
| | | | Left paw | Right paw | Left paw | Right paw |
| 1 | Sorafenib | 100 mg/kg | Compound of formula (I), 1% | None | 2/12 (17%) | 9/12 (75%) |
| 2 | Erlotinib | 70 mg/kg | Compound of formula (I), 1% | None | 3/12 (25%) | 10/12 (83%) |
| 3 | Afatinib | 50 mg/kg | Compound of formula (I), 1% | None | 3/12 (25%) | 9/12 (75%) |
| 4 | Osimertinib | 60 mg/kg | Compound of formula (I), 1% | None | 2/12 (17%) | 8/12 (67%) |
| 5 | Sorafenib | 100 mg/kg | Compound of formula (I), 3% | None | 2/12 (17%) | 4/12 (33%) |
| 6 | Erlotinib | 70 mg/kg | Compound of formula (I), 3% | None | 2/12 (17%) | 5/12 (42%) |
| 7 | Afatinib | 50 mg/kg | Compound of formula (I), 3% | None | 2/12 (17%) | 4/12 (33%) |
| 8 | Osimertinib | 60 mg/kg | Compound of formula (I), 3% | None | 2/12 (17%) | 4/12 (33%) |
| 9 | Sorafenib | 100 mg/kg | Compound of formula (I), 10% | None | 0/12 (0%) | 4/12 (33%) |
| 10 | Erlotinib | 70 mg/kg | Compound of formula (I), 10% | None | 1/12 (8%) | 4/12 (33%) |
| 11 | Afatinib | 50 mg/kg | Compound of formula (I), 10% | None | 0/12 (0%) | 3/12 (25%) |
| 12 | Osimertinib | 60 mg/kg | Compound of formula (I), 10% | None | 0/12 (0%) | 3/12 (25%) |
| 13 | Sorafenib | 100 mg/kg | None | None | 9/12 (75%) | |
| 14 | Erlotinib | 70 mg/kg | None | None | 10/12 (83.3%) | |
| 15 | Afatinib | 50 mg/kg | None | None | 8/12 (66.7%) | |
| 16 | Osimertinib | 60 mg/kg | None | None | 9/12 (75%) | |

As shown in Table 3, when the sorafenib, the erlotinib, the afatinib, and the osimertinib were respectively administered once daily by gavage at doses of 100 mg/kg, 70 mg/kg, 50 mg/kg, and 60 mg/kg to the SD rats, successful modeling rates for the hand-foot skin reaction were 75%, 83.3%, 66.7%, and 75%, respectively. The compound of formula (I) at a low dose (such as the compound at a mass content of 1%) can significantly decrease the incidence rate of hand-foot skin reaction at a drug application site. The compound of formula (I) at a high mass content (such as the compound at a mass content of 3% and 10%) can significantly decrease the incidence rate of hand-foot skin reaction in all paws of the rats.

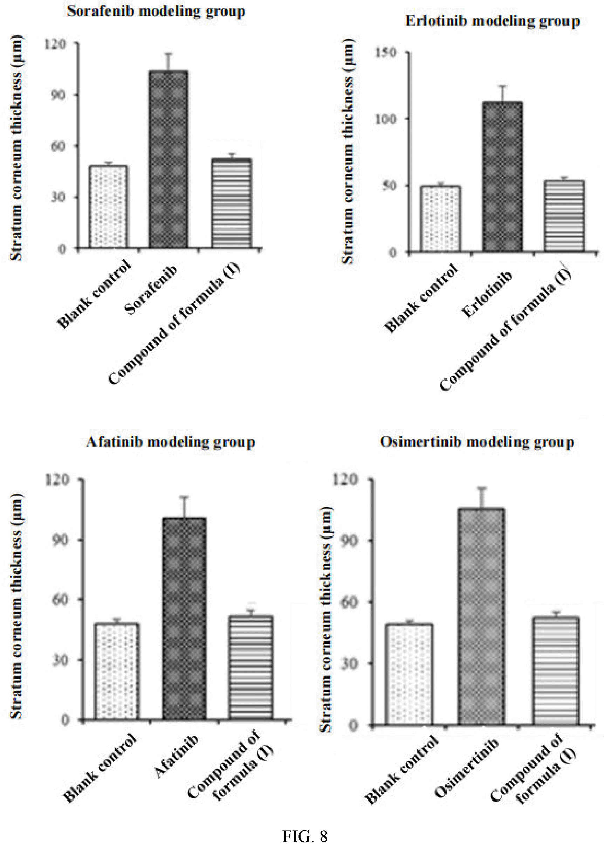

According to stratum corneum thickness measurement results shown in FIG. 8, in the successfully modeled rats using the sorafenib, the erlotinib, the afatinib, and the osimertinib, the stratum corneum thickness of the paw plantar skin was significantly higher than that of normal rats. The compound of formula (I) can effectively decrease the stratum corneum thickness of the paw plantar skin of the modeled rats.

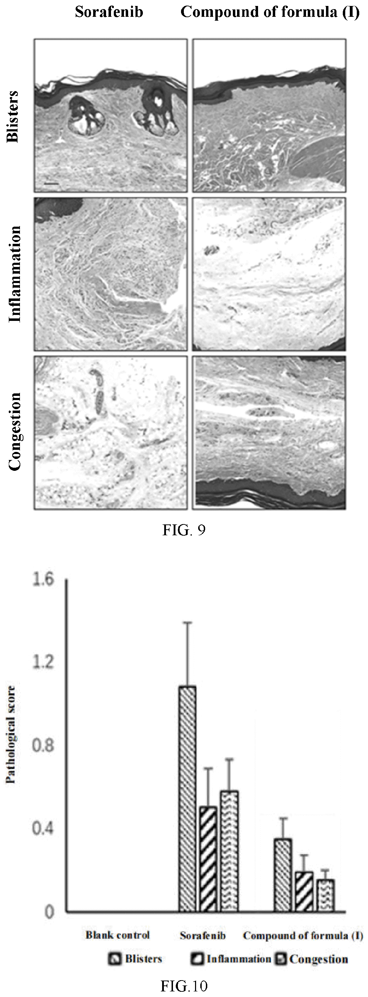

According to tissue staining results shown in FIG. 9, due to skin toxicity caused by the modeling drugs, blisters can be formed under the epidermis and in the epidermis of the paw plantar skin of the rats, inflammatory cell infiltration occurs in the dermis, and congestion symptoms occur in skin tissues. The compound of formula (I) can effectively alleviate the formation of blisters in the epidermis and subepidermis caused by the above modeling drugs, inhibit the inflammatory cell infiltration in the dermis, and significantly alleviate the skin congestion phenomenon.

According to pathological scoring results shown in FIG. 10, the modeling drugs can cause the formation of blisters under the epidermis and in the epidermis at paw plantar sites of the rats, inflammatory cell infiltration occurs in the dermis, and congestion symptoms occur in skin tissues. The compound of formula (I) can effectively alleviate the formation of blisters in the epidermis and subepidermis caused by the above modeling drugs, inhibit the inflammatory cell infiltration in the dermis, and significantly alleviate the skin congestion phenomenon.

Immunohistochemical staining results also showed that in the successfully modeled rats using the sorafenib, the erlotinib, the afatinib, and the osimertinib, KRT1 and Loricrin were significantly increased in tissues. However, with the addition of compounds, KRT1 and Loricrin were significantly decreased in the tissues. In summary, the compound of formula (I) can effectively inhibit the differentiation of keratinocytes both *in vitro* and *in vivo*, thus exhibiting potential for treatment of a hyperkeratosis-related disease.

**Example 11: Effect of the compound of formula (I) in a chemotherapeutic drug-induced mouse model with hand-foot syndrome**

After adaptive feeding for 7 days, SPF-grade ICR mice (male, 5-6 weeks old, with a body weight of approximately 35 g) were randomly divided into groups, including a blank control group (6 mice), a capecitabine modeling group (6 mice), a docetaxel modeling group (6 mice), a capecitabine + compound of formula (I) group (6 mice), a docetaxel + compound of formula (I) group (6 mice), a capecitabine + nicotinamide group (6 mice), and a docetaxel + nicotinamide group (6 mice). The groups were respectively administered corresponding modeling drugs by oral gavage (capecitabine at 200 mg/kg and docetaxel at 25 mg/kg) once daily; and the blank control group was administered an equivalent volume of normal saline. After administration with the chemotherapeutic drugs for 2 weeks, the ointments containing the compound of formula (I) (at a compound content of 3%) used in Example 10 were respectively applied topically in the groups, or nicotinamide was orally administered (100 mg/kg) once daily continuously for 16 days.

Measurement of the degree of swelling of toes: The degree of swelling of hind limb toes of the mice was measured using a toe swelling tester (KW-7C, Nanjing Calvin Biotechnology Co., Ltd.) before administration with the modeling drugs, after administration with the modeling drugs for 2 weeks, and before sacrifice, respectively. Before measurement, ankle joints of hind feet of the mice were marked at same positions before administration, after administration for 2 weeks, and before sacrifice. During measurement, water was properly located at the same level as the marked positions, and numerical values were recorded after becoming stable.

Pathological staining of skin tissues: Skin tissue samples were selected from hind limb toes of the mice and placed in 4% paraformaldehyde for fixation at room temperature for 4 hours. Then, the tissues were taken out and rinsed with running water for several hours. The samples were subjected to dehydration treatment with 70%, 80%, and 90% ethanol solutions, followed by treatment with a mixed solution of equal amounts of absolute alcohol and xylene for 15 minutes, and then permeabilized twice with a xylene solution for 15 minutes each time until the samples became transparent. The samples were placed in a mixture of half xylene and half paraffin for soaking for 15 minutes, and then, paraffin I and paraffin II were added for wax penetration for one hour, respectively. After embedding with paraffin, the samples were sectioned, baked, dewaxed, and hydrated. Then, the hydrated sections were placed in a hematoxylin solution (ZSGB-BIO, article No. 23041001) for staining for 3 minutes, and a differentiation solution of hydrochloric acid in ethanol was added for differentiation for 15 seconds. After rinsing with water, a Scott bluing solution (Servicebio, article No. 20230801) was added for bluing for 15 seconds. After rinsing with running water, the sections were stained with an eosin solution (Solarbio, article No. 33535) for 3 minutes, rinsed with running water, dehydrated, permeabilized, sealed, and inspected under a microscope.

Immunohistochemical staining: After baking, dewaxing, and hydration, paraffin sections of skin tissues of hind limb toes of the mice were subjected to antigen retrieval using a 0.2 M citric acid buffer solution (containing 1.534 g of citric acid, 3.1 g of sodium citrate, and 100 mL of distilled water, pH 4.7). Endogenous hydrogen peroxide was removed using 3% hydrogen peroxide (Shandong Annjet High-Tech Disinfection Technology Co., Ltd., article No. 20290902), and the sections were incubated at room temperature for 10 minutes and then fully rinsed with a PBS buffer solution (containing 8 g of NaCl, 0.2 g of KCl, 1.44 g of $Na_2HPO_4$, and 1 M HCl, pH 7.4). The sections were permeabilized with 0.5% Triton-X-100 (AmyJet Scientific, article No. A-CSH436-100 mL), respectively permeabilized with mouse anti-IL-1β and rabbit IL-8 for 10 minutes, and not permeabilized with rabbit anti-IL-6. After blocking with a 5% BSA antigen, 1/150 diluted mouse anti-IL-1β (Affinity Biosciences, article No. BF8021), 1/200 diluted rabbit anti-IL-8 (Affinity Biosciences, article No. DF6998), or 1/150 diluted rabbit anti-IL-6 (Affinity Biosciences, article No. DF6087) was added dropwise onto each slide, and the slides were placed in a wet box for incubation at 4°C overnight. After the overnight incubation, the wet box was taken out and allowed to stand at room temperature for 45 minutes. The slides were rinsed 3 times with a PBS buffer solution for 15 minutes each time. For the rabbit anti-IL-8 and rabbit anti-IL-6 indicators, 1/100 diluted horseradish peroxidase-labeled goat anti-rabbit IgG (H+L) (ZSGB-BIO, article No. 234750811) was added. For the mouse anti-IL-1β indicator, 1/100 diluted horseradish peroxidase-labeled goat anti-mouse IgG (H+L) (ZSGB-BIO, article No. 226700804) was added. The slides were incubated at 37°C for 30 minutes and then fully rinsed with a PBS buffer solution. DAB (CWBIO, article No. 13723) was added for color development for 3-5 minutes, and the sections were cleaned with a PBS buffer solution for 1 minute, re-stained with hematoxylin (ZSGB-BIO, article No. 23041001) for 3 minutes, differentiated with hydrochloric acid in alcohol, blued, and washed with water. The sections were sequentially placed in 80% ethanol, 95% ethanol, anhydrous ethanol, and anhydrous ethanol II for rapid dehydration, permeabilized with xylene I and xylene II, sealed with a neutral resin adhesive (Solarbio, Catalog No. 20230725), and inspected under a microscope.

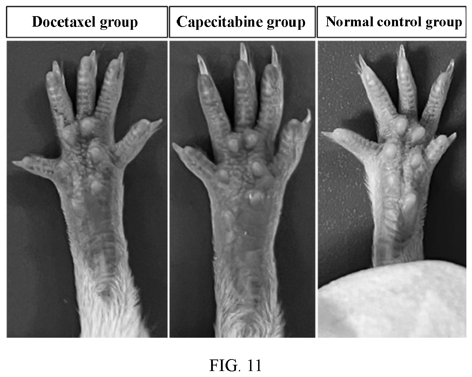

Mouse skin appearance results are shown in FIG. 11. After modeling with the capecitabine and the docetaxel for 14 days, the skin of the hind limb toes of the mice had obvious lesions. Compared with the normal control group, the skin of the hind limb toes of the mice in the capecitabine group and the docetaxel group had obvious erythema, rhagades, and blisters.

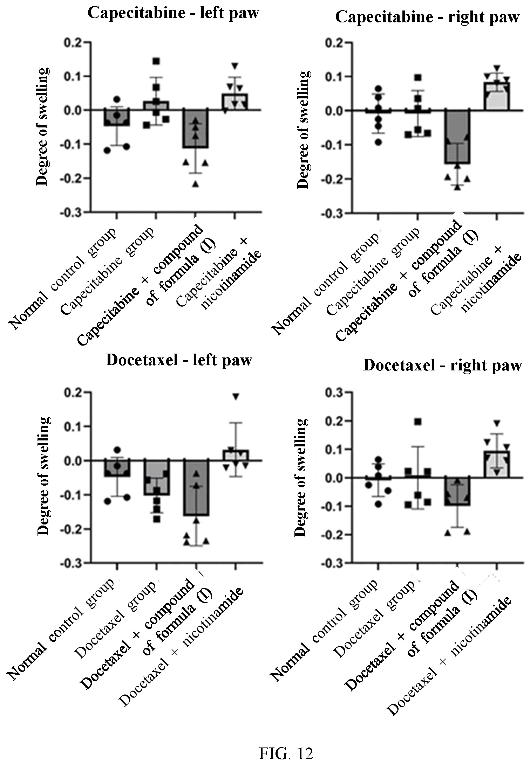

The degree of swelling of the hind limb toes of the mice is shown in FIG. 12. After continuous oral administration of the modeling drugs including the capecitabine and the docetaxel for 14 days, swelling of the hind limb toes of the mice was caused. Topical application of the ointments containing the compound of formula (I) to the hind limb toes of the mice can significantly alleviate the swelling of the toes caused by the modeling drugs ($p<0.05$). However, it was not observed that oral administration of the nicotinamide at 100 mg/kg significantly alleviated the swelling of the hind limb toes of the mice.

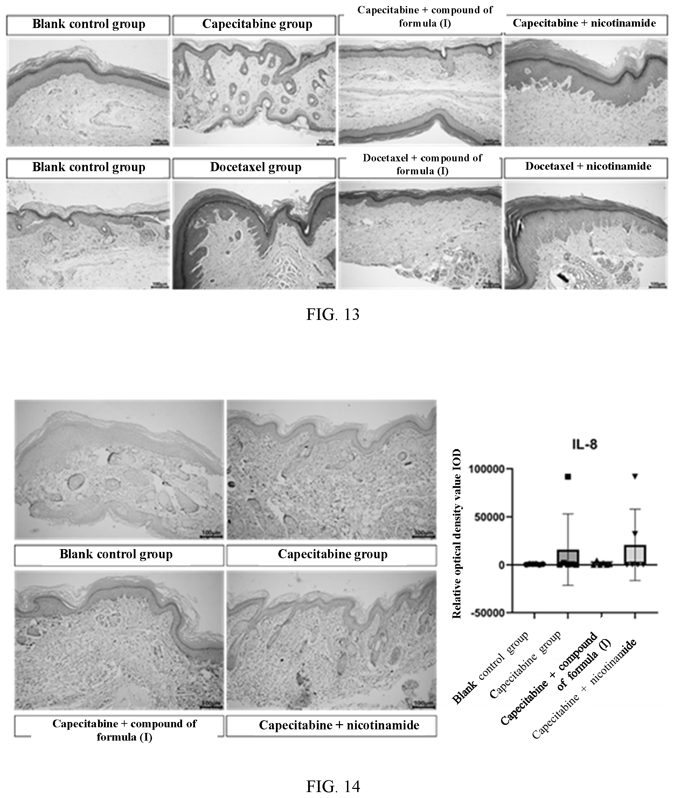

Pathological staining results of toe skin tissues are shown in FIG. 13. Compared with the normal control group, after continuous oral administration of the modeling drugs including the capecitabine and the docetaxel for 14 days, the epidermal layer and the stratum corneum in the skin tissues of the hind limb toes of the mice were significantly thickened, with increased granular cells in the basal layer and infiltration of inflammatory cells observed. Topical application of the ointments containing the compound of formula (I) to the hind limb toes of the mice can effectively alleviate the phenomenon of thickening of the epidermal layer and the stratum corneum in the skin tissues, with the morphology of basal layer cells restored to normal and no infiltration of inflammatory cells observed. However, oral administration of nicotinamide at 100 mg/kg mildly alleviated the thickening of the epidermal layer and the stratum corneum in the skin tissues of the hind limb toes of the mice, with slightly increased granular cells in the basal layer and infiltration of inflammatory cells still observed.

Immunohistochemical results of the capecitabine modeling series groups are shown in FIG. 14. Compared with the normal control group, after continuous oral administration of the modeling drug capecitabine for 14 days, cytokine IL-8 in the skin tissues of the hind limb toes of the mice was significantly increased ($p<0.05$). This indicates that oral administration of the capecitabine resulted in skin inflammation of the mice, which is consistent with a clinical trial observation result. Topical application of the ointments containing the compound of formula (I) to the hind limb toes of the mice can significantly decrease the expression level of cytokine IL-8 in the skin tissues of the hind limb toes of the mice ($p<0.05$). However, oral administration of the nicotinamide at 100 mg/kg didn't have a significant impact on the expression level of cytokine IL-8 in the skin tissues of the hind limb toes of the mice.

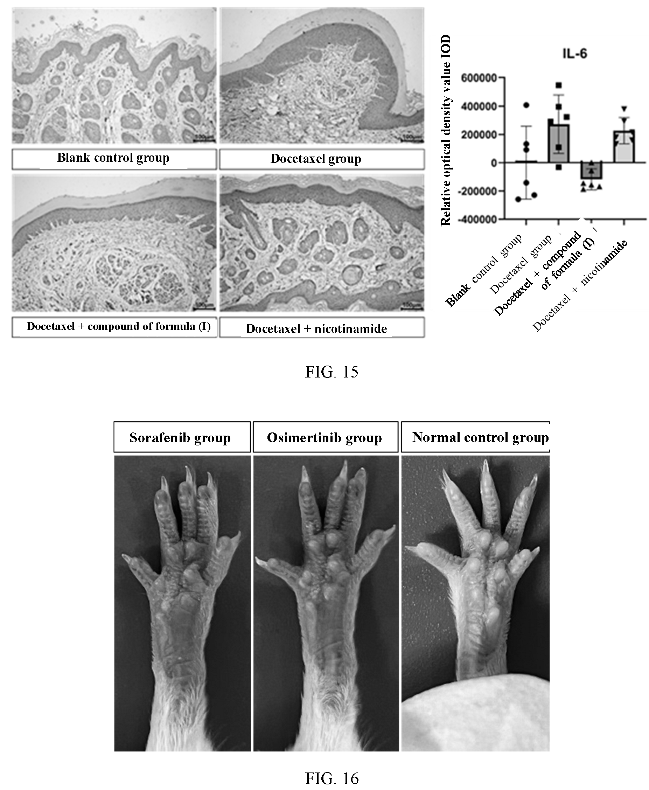

Immunohistochemical results of the docetaxel modeling series groups are shown in FIG. 15. Compared with the normal control group, after continuous oral administration of the modeling drug docetaxel for 14 days, cytokine IL-6 in the skin tissues of the hind limb toes of the mice was significantly increased ($p<0.05$). This indicates that oral administration of the docetaxel resulted in skin inflammation of the mice, which is consistent with a clinical trial observation result. Topical application of the ointments containing the compound of formula (I) to the hind limb toes of the mice can significantly decrease the expression level of cytokine IL-6 in the skin tissues of the hind limb toes of the mice ($p<0.05$). However, oral administration of the nicotinamide at 100 mg/kg didn't have a significant impact on the expression level of cytokine IL-6 in the skin tissues of the hind limb toes of the mice.

**Example 12: Effect of the compound of formula (I) in a kinase inhibitor-induced mouse model with hand-foot skin reaction**

After adaptive feeding for 7 days, SPF-grade ICR mice (male, 5-6 weeks old, with a body weight of approximately 35 g) were randomly divided into groups, including a blank control group (6 mice), a sorafenib modeling group (6 mice), an osimertinib modeling group (6 mice), a sorafenib + compound of formula (I) group (6 mice), an osimertinib + compound of formula (I) group (6 mice), a sorafenib + nicotinamide group (6 mice), and an osimertinib + nicotinamide group (6 mice). The groups were respectively administered corresponding modeling drugs by oral gavage (sorafenib at 100 mg/kg and osimertinib at 10 mg/kg) once daily; and the blank control group was administered an equivalent volume of normal saline. After administration with the chemotherapeutic drugs for 2 weeks, the ointments containing the compound of formula (I) (at a compound content of 3%) used in Example 10 were respectively applied topically in the groups, or nicotinamide was orally administered (100 mg/kg) once daily continuously for 16 days.

Measurement of the degree of swelling of toes: The degree of swelling of hind limb toes of the mice was measured using a toe swelling tester (KW-7C, Nanjing Calvin Biotechnology Co., Ltd.) before administration with the modeling drugs, after administration with the modeling drugs for 2 weeks, and before sacrifice, respectively. Before measurement, ankle joints of hind feet of the mice were marked at same positions before administration, after administration for 2 weeks, and before sacrifice. During measurement, water was properly located at the same level as the marked positions, and numerical values were recorded after becoming stable.

Pathological staining of skin tissues: Skin tissue samples were selected from hind limb toes of the mice and placed in 4% paraformaldehyde for fixation at room temperature for 4 hours. Then, the tissues were taken out and rinsed with running water for several hours. The samples were subjected to dehydration treatment with 70%, 80%, and 90% ethanol solutions, followed by treatment with a mixed solution of equal amounts of absolute alcohol and xylene for 15 minutes, and then permeabilized twice with a xylene solution for 15 minutes each time until the samples became

transparent. The samples were placed in a mixture of half xylene and half paraffin for soaking for 15 minutes, and then, paraffin I and paraffin II were added for wax penetration for one hour, respectively. After embedding with paraffin, the samples were sectioned, baked, dewaxed, and hydrated. Then, the hydrated sections were placed in a hematoxylin solution (ZSGB-BIO, article No. 23041001) for staining for 3 minutes, and a differentiation solution of hydrochloric acid in ethanol was added for differentiation for 15 seconds. After rinsing with water, a Scott bluing solution (Servicebio, article No. 20230801) was added for bluing for 15 seconds. After rinsing with running water, the sections were stained with an eosin solution (Solarbio, article No. 33535) for 3 minutes, rinsed with running water, dehydrated, permeabilized, sealed, and inspected under a microscope.

Immunohistochemical staining: After baking, dewaxing, and hydration, paraffin sections of skin tissues of hind limb toes of the mice were subjected to antigen retrieval using a 0.2 M citric acid buffer solution (containing 1.534 g of citric acid, 3.1 g of sodium citrate, and 100 mL of distilled water, pH 4.7). Endogenous hydrogen peroxide was removed using 3% hydrogen peroxide (Shandong Annjet High-Tech Disinfection Technology Co., Ltd., article No. 20290902), and the sections were incubated at room temperature for 10 minutes and then fully rinsed with a PBS buffer solution (containing 8 g of NaCl, 0.2 g of KCl, 1.44 g of $Na_2HPO_4$, and 1 M HCl, pH 7.4). The sections were permeabilized with 0.5% Triton-X-100 (AmyJet Scientific, article No. A-CSH436-100 mL), respectively permeabilized with mouse anti-IL-1β and rabbit IL-8 for 10 minutes, and not permeabilized with rabbit anti-IL-6. After blocking with a 5% BSA antigen, 1/150 diluted mouse anti-IL-1β (Affinity Biosciences, article No. BF8021), 1/200 diluted rabbit anti-IL-8 (Affinity Biosciences, article No. DF6998), or 1/150 diluted rabbit anti-IL-6 (Affinity Biosciences, article No. DF6087) was added dropwise onto each slide, and the slides were placed in a wet box for incubation at 4°C overnight. After the overnight incubation, the wet box was taken out and allowed to stand at room temperature for 45 minutes. The slides were rinsed 3 times with a PBS buffer solution for 15 minutes each time. For the rabbit anti-IL-8 and rabbit anti-IL-6 indicators, 1/100 diluted horseradish peroxidase-labeled goat anti-rabbit IgG (H+L) (ZSGB-BIO, article No. 234750811) was added. For the mouse anti-IL-1β indicator, 1/100 diluted horseradish peroxidase-labeled goat anti-mouse IgG (H+L) (ZSGB-BIO, article No. 226700804) was added. The slides were incubated at 37°C for 30 minutes and then fully rinsed with a PBS buffer solution. DAB (CWBIO, article No. 13723) was added for color development for 3-5 minutes, and the sections were cleaned with a PBS buffer solution for 1 minute, re-stained with hematoxylin (ZSGB-BIO, article No. 23041001) for 3 minutes, differentiated with hydrochloric acid in alcohol, blued, and washed with water. The sections were sequentially placed in 80% ethanol, 95% ethanol, anhydrous ethanol, and anhydrous ethanol II for rapid dehydration, permeabilized with xylene I and xylene II, sealed with a neutral resin adhesive (Solarbio, Catalog No. 20230725), and inspected under a microscope.

Results are shown in FIG. 16. After modeling with the sorafenib and the osimertinib for 14 days, the skin of the hind limb toes of the mice had obvious lesions. Compared with the normal control group, the skin of the hind limb toes of the mice in the sorafenib group and the osimertinib group had obvious erythema and rhagades.

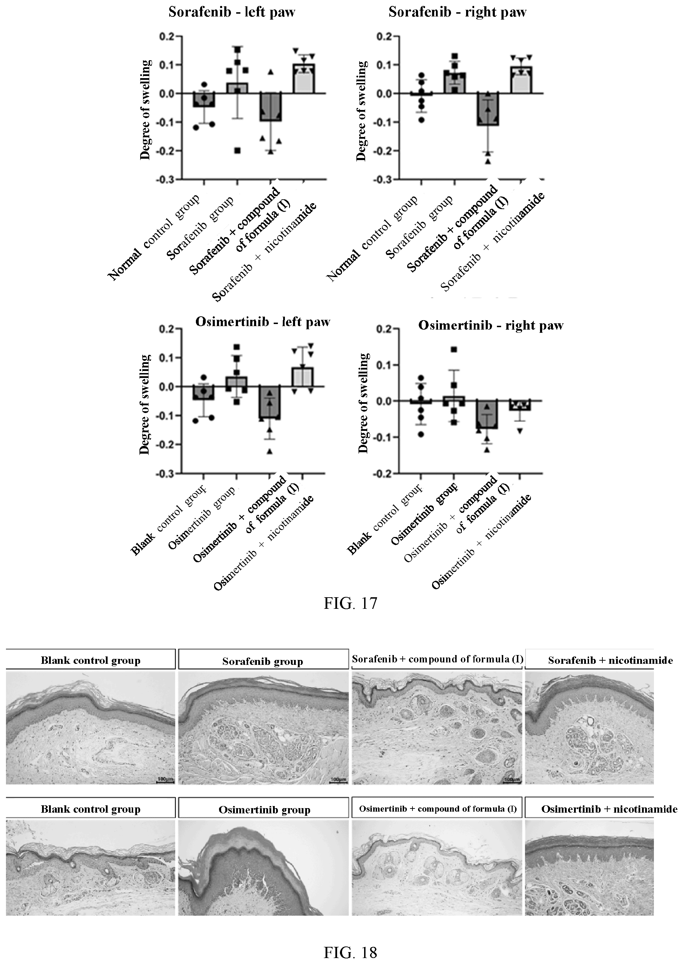

The degree of swelling of the hind limb toes of the mice is shown in FIG. 17. After continuous oral administration of the modeling drugs including the sorafenib and the osimertinib for 14 days, significant swelling of the hind limb toes of the mice was caused. Topical application of the ointments containing the compound of formula (I) to the hind limb toes of the mice can effectively alleviate the swelling of the toes caused by the modeling drugs. However, it was not observed that oral administration of the nicotinamide at 100 mg/kg significantly alleviated the swelling of the hind limb toes of the mice.

Pathological staining results of toe skin tissues are shown in FIG. 18. Compared with the normal control group, after continuous oral administration of the modeling drugs including the sorafenib and the osimertinib for 14 days, the epidermal layer and the stratum corneum in the skin tissues of the hind limb toes of the mice were significantly thickened, with increased granular cells in the basal layer and infiltration of inflammatory cells observed. Topical application of the ointments containing the compound of formula (I) to the hind limb toes of the mice can effectively alleviate the phenomenon of thickening of the epidermal layer and the stratum corneum in the skin tissues, with the morphology of basal layer cells restored to normal and no infiltration of inflammatory cells observed. However, oral administration of the nicotinamide at 100 mg/kg didn't significantly alleviate the thickening of the epidermal layer and the stratum corneum in the skin tissues of the hind limb toes of the mice, with slightly restored morphology of basal layer cells and infiltration of inflammatory cells still observed.

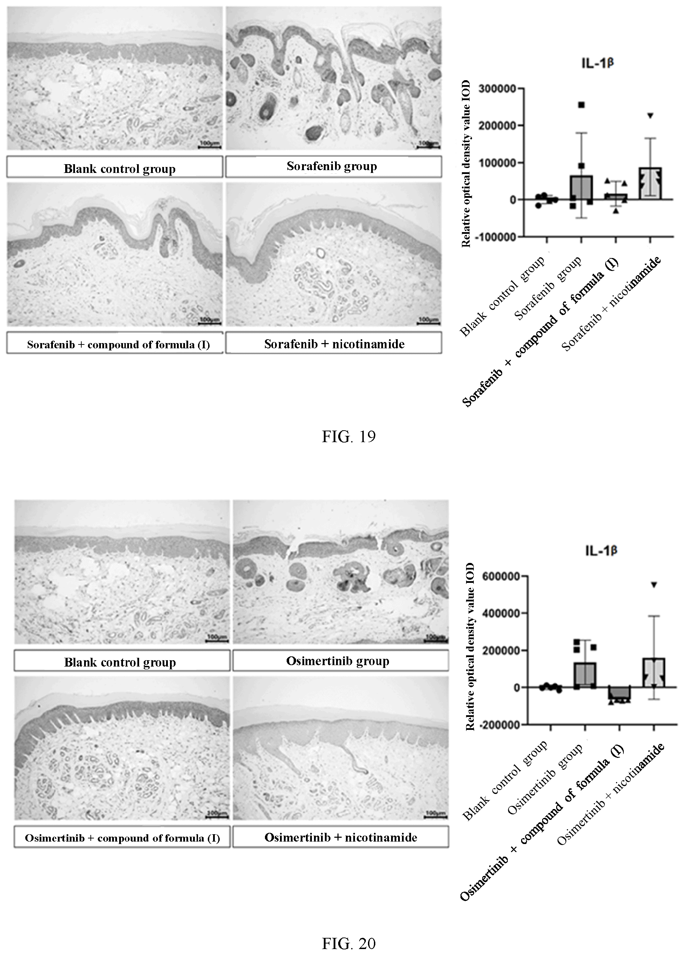

Immunohistochemical results of the sorafenib modeling series groups are shown in FIG. 19. Compared with the normal control group, after continuous oral administration of the modeling drug sorafenib for 14 days, cytokine IL-1β in the skin tissues of the hind limb toes of the mice was significantly increased ($p<0.05$). This indicates that oral administration of the sorafenib resulted in skin inflammation of the mice, which is consistent with a clinical trial observation result. Topical application of the ointments containing the compound of formula (I) to the hind limb toes of the mice can significantly decrease the expression level of cytokine IL-1β in the skin tissues of the hind limb toes of the mice ($p<0.05$). However, oral administration of the nicotinamide at 100 mg/kg didn't have a significant impact on the expression level of cytokine IL-1β in the skin tissues of the hind limb toes of the mice.

Immunohistochemical results of the osimertinib modeling series groups are shown in FIG. 20. Compared with the normal control group, after continuous oral administration of the modeling drug osimertinib for 14 days, cytokine IL-1β in the skin tissues of the hind limb toes of the mice was significantly increased ($p<0.05$). This indicates that oral administration

of the osimertinib resulted in skin inflammation of the mice, which is consistent with a clinical trial observation result. Topical application of the ointments containing the compound of formula (I) to the hind limb toes of the mice can significantly decrease the expression level of cytokine IL-1β in the skin tissues of the hind limb toes of the mice ($p<0.05$). However, oral administration of the nicotinamide at 100 mg/kg didn't have a significant impact on the expression level of cytokine IL-1β in the skin tissues of the hind limb toes of the mice.

**Example 13: Preparation of a pharmaceutical co-crystal formed by the compound of formula (I) and nicotinamide**

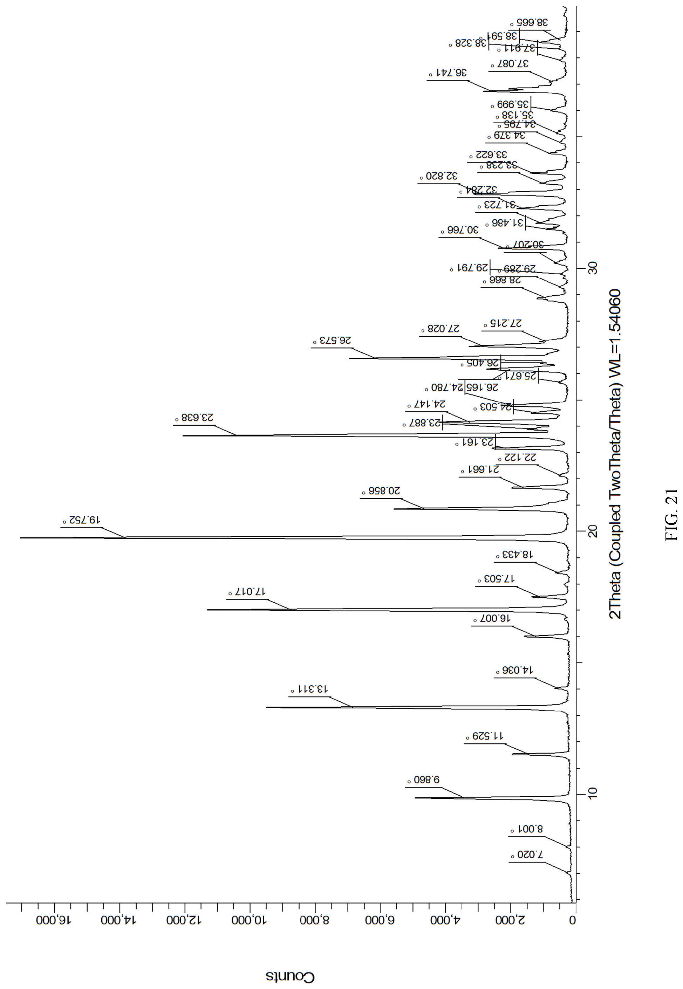

Approximately 20 mg of the compound of formula (I) and 10.3 mg of nicotinamide were weighed and placed in a 2 mL glass vial, and acetonitrile (0.25 mL) was added to obtain a suspension. The resulting sample was suspended and stirred at 5°C for 3 days. The resulting suspension was allowed to pass through a 0.45 μm nylon filter membrane and centrifuged at a rotation speed of 14,000 rpm, and the resulting solid was dried under vacuum at 25°C for 4 hours to obtain a product. X-ray powder diffraction detection showed that the product was a pharmaceutical co-crystal formed by the compound of formula (I) and the nicotinamide, with an XRPD pattern as shown in FIG. 21 and positions of characteristic peaks as shown in Table 4. A DSC spectrogram showed a melting $T_{onset}$ @ 156.75°C. A stoichiometric ratio of the resulting pharmaceutical co-crystal was 1:1.

**Table 4: XRPD diffraction peak data of the pharmaceutical co-crystal formed by the compound of formula (I) and the nicotinamide**

| 2θ(°) | D(Å) | Relative intensity (%) |
|---|---|---|
| 7.020 | 12.5824 | 0.80 |
| 8.001 | 11.0416 | 0.80 |
| 9.860 | 8.96307 | 23.80 |
| 11.529 | 7.66899 | 9.10 |
| 13.311 | 6.64637 | 48.70 |
| 14.036 | 6.30468 | 2.30 |
| 16.007 | 5.53235 | 7.40 |
| 17.017 | 5.20623 | 62.70 |
| 17.503 | 5.06265 | 6.30 |
| 18.433 | 4.80945 | 2.30 |
| 19.752 | 4.49118 | 100.00 |
| 20.856 | 4.25583 | 32.30 |
| 21.661 | 4.09935 | 10.10 |
| 22.122 | 4.01509 | 1.80 |
| 23.161 | 3.83715 | 14.10 |
| 23.638 | 3.76081 | 74.50 |
| 23.887 | 3.72224 | 6.80 |
| 24.147 | 3.68278 | 22.00 |
| 24.503 | 3.63001 | 7.10 |
| 24.780 | 3.59009 | 12.50 |
| 25.671 | 3.46747 | 1.50 |
| 26.165 | 3.40303 | 12.80 |
| 26.405 | 3.37275 | 4.50 |
| 26.573 | 3.35172 | 43.30 |
| 27.028 | 3.29633 | 18.90 |
| 27.215 | 3.27408 | 4.70 |

(continued)

| 2θ(°) | D(Å) | Relative intensity (%) |
|---|---|---|
| 28.866 | 3.09047 | 4.90 |
| 29.289 | 3.04678 | 1.60 |
| 29.791 | 2.9966 | 1.40 |
| 30.207 | 2.95624 | 2.30 |
| 30.766 | 2.90383 | 14.30 |
| 31.486 | 2.83909 | 4.20 |
| 31.723 | 2.81837 | 5.90 |
| 32.284 | 2.77071 | 9.90 |
| 32.820 | 2.72664 | 18.90 |
| 33.238 | 2.69328 | 5.30 |
| 33.622 | 2.66341 | 7.70 |
| 34.379 | 2.6065 | 3.70 |
| 34.795 | 2.57628 | 1.40 |
| 35.138 | 2.5519 | 1.90 |
| 35.999 | 2.49282 | 3.00 |
| 36.741 | 2.44414 | 16.80 |
| 37.087 | 2.42216 | 2.90 |
| 37.911 | 2.3714 | 1.40 |
| 38.328 | 2.34651 | 1.50 |
| 38.591 | 2.33115 | 5.50 |
| 38.665 | 2.32686 | 1.30 |

**Example 14: Preparation of a pharmaceutical co-crystal formed by the compound of formula (I) and isonicotinamide**

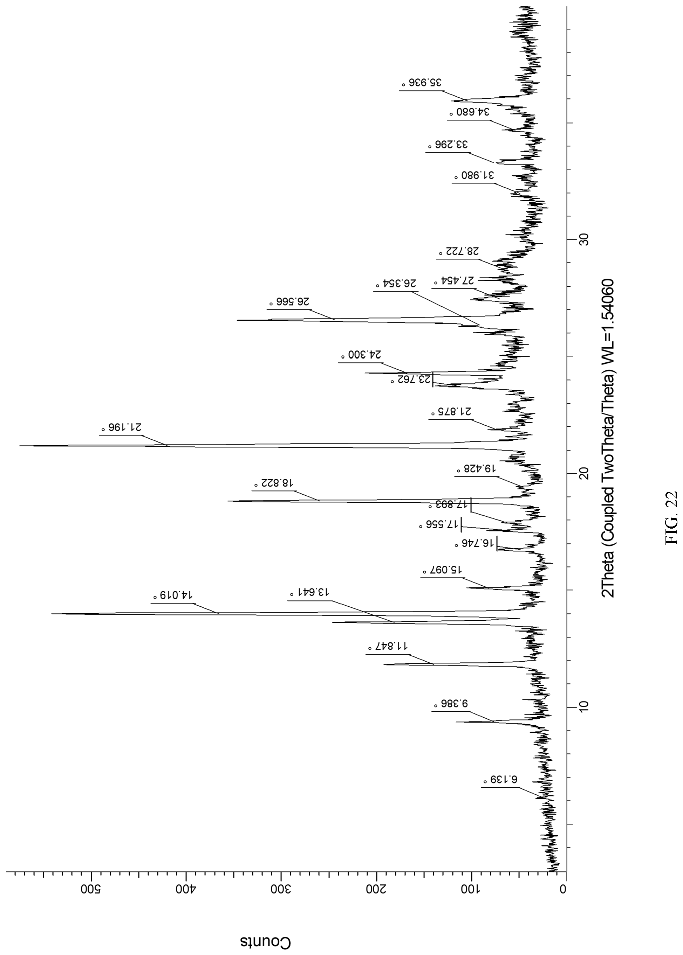

Approximately 20 mg of the compound of formula (I) and 10.2 mg of isonicotinamide were weighed and placed in a 2 mL glass vial, and ethanol (0.2 mL) was added to obtain a suspension. The resulting sample was suspended and stirred at 5°C for 3 days. The resulting suspension was allowed to pass through a 0.45 μm nylon filter membrane and centrifuged at a rotation speed of 14,000 rpm, and the resulting solid was dried under vacuum at 25°C for 4 hours to obtain a product. X-ray powder diffraction detection showed that the product was a pharmaceutical co-crystal formed by the compound of formula (I) and the isonicotinamide, with an XRPD pattern as shown in FIG. 22 and positions of characteristic peaks as shown in Table 5. A DSC spectrogram showed a melting $T_{onset}$ at 160.45°C. A stoichiometric ratio of the resulting pharmaceutical co-crystal was 1:1.

**Table 5: XRPD diffraction peak data of the pharmaceutical co-crystal formed by the compound of formula (I) and the isonicotinamide**

| 2θ(°) | D(Å) | Relative intensity (%) |
|---|---|---|
| 6.139 | 14.3846 | 1.90 |
| 9.386 | 9.41521 | 13.80 |
| 11.847 | 7.46425 | 29.10 |
| 13.641 | 6.48624 | 39.00 |
| 14.019 | 6.31196 | 87.70 |
| 15.097 | 5.86363 | 13.90 |

(continued)

| 2θ(°) | D(Å) | Relative intensity (%) |
|---|---|---|
| 16.746 | 5.29 | 6.30 |
| 17.556 | 5.04765 | 8.30 |
| 17.893 | 4.9532 | 8.70 |
| 18.822 | 4.71099 | 60.00 |
| 19.428 | 4.56536 | 4.00 |
| 21.196 | 4.1883 | 100.00 |
| 21.875 | 4.05976 | 8.30 |
| 23.762 | 3.74148 | 18.30 |
| 24.300 | 3.65983 | 31.30 |
| 26.354 | 3.37908 | 10.30 |
| 26.566 | 3.3526 | 50.30 |
| 27.454 | 3.24611 | 5.00 |
| 28.722 | 3.10567 | 5.20 |
| 31.980 | 2.79636 | 4.40 |
| 33.296 | 2.68876 | 11.10 |
| 34.680 | 2.58452 | 5.00 |
| 35.936 | 2.49706 | 17.30 |

**Example 15: Preparation of a pharmaceutical co-crystal formed by the compound of formula (I) and L-proline**

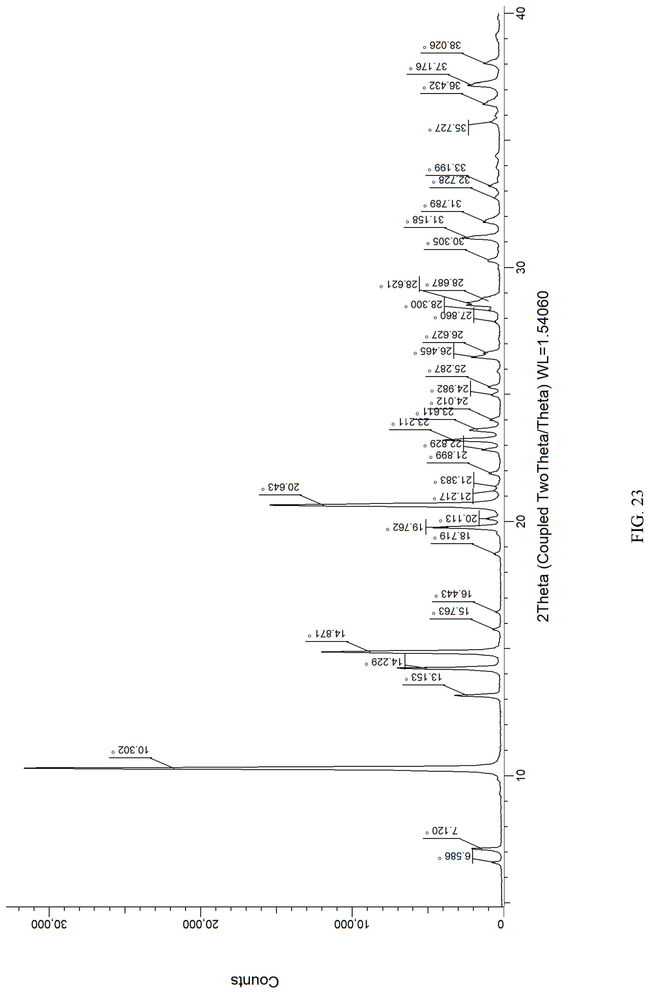

Approximately 20 mg of the compound of formula (I) and 9.7 mg of L-proline were weighed and placed in a 2 mL glass vial, and ethanol (0.25 mL) was added to obtain a suspension. The resulting sample was suspended and stirred at 5°C for 3 days. The resulting suspension was allowed to pass through a 0.45 μm nylon filter membrane and centrifuged at a rotation speed of 14,000 rpm, and the resulting solid was dried under vacuum at 25°C for 4 hours to obtain a product. X-ray powder diffraction detection showed that the product was a pharmaceutical co-crystal formed by the compound of formula (I) and the L-proline, with an XRPD pattern as shown in FIG. 23 and positions of characteristic peaks as shown in Table 6. A DSC spectrogram showed a melting $T_{onset}$ @ 183.55°C. A stoichiometric ratio of the resulting pharmaceutical co-crystal was 1:1.

**Table 6 XRPD diffraction peak data of the pharmaceutical co-crystal formed by the compound of formula (I) and the L-proline**

| 2θ(°) | D(Å) | Relative intensity (%) |
|---|---|---|
| 6.586 | 13.4092 | 2.00 |
| 7.120 | 12.4061 | 5.80 |
| 10.302 | 8.57947 | 100.00 |
| 13.153 | 6.72557 | 10.00 |
| 14.229 | 6.21936 | 22.20 |
| 14.871 | 5.95232 | 39.60 |
| 15.763 | 5.61748 | 1.70 |
| 16.443 | 5.38677 | 1.10 |
| 18.719 | 4.73667 | 1.50 |
| 19.762 | 4.48885 | 15.80 |
| 20.113 | 4.41121 | 3.20 |

(continued)

| 2θ(°) | D(Å) | Relative intensity (%) |
|---|---|---|
| 20.643 | 4.2992 | 53.80 |
| 21.217 | 4.18422 | 1.30 |
| 21.383 | 4.15208 | 1.10 |
| 21.899 | 4.05545 | 2.50 |
| 22.829 | 3.89225 | 4.20 |
| 23.211 | 3.8291 | 14.00 |
| 23.611 | 3.7651 | 6.70 |
| 24.012 | 3.70312 | 2.30 |
| 24.982 | 3.56148 | 2.20 |
| 25.287 | 3.51915 | 2.90 |
| 26.465 | 3.36512 | 7.30 |
| 26.627 | 3.34511 | 3.70 |
| 27.860 | 3.19976 | 1.00 |
| 28.300 | 3.15105 | 2.60 |
| 28.621 | 3.11643 | 10.20 |
| 28.687 | 3.10934 | 3.40 |
| 30.305 | 2.94693 | 3.20 |
| 31.158 | 2.86817 | 9.40 |
| 31.789 | 2.81271 | 4.10 |
| 32.728 | 2.73405 | 1.40 |
| 33.199 | 2.69639 | 2.70 |
| 35.727 | 2.51117 | 2.50 |
| 36.432 | 2.46414 | 4.10 |
| 37.176 | 2.41656 | 8.20 |
| 38.026 | 2.36448 | 4.00 |

**Example 16: Preparation of a pharmaceutical co-crystal formed by the compound of formula (I) and glycolic acid**

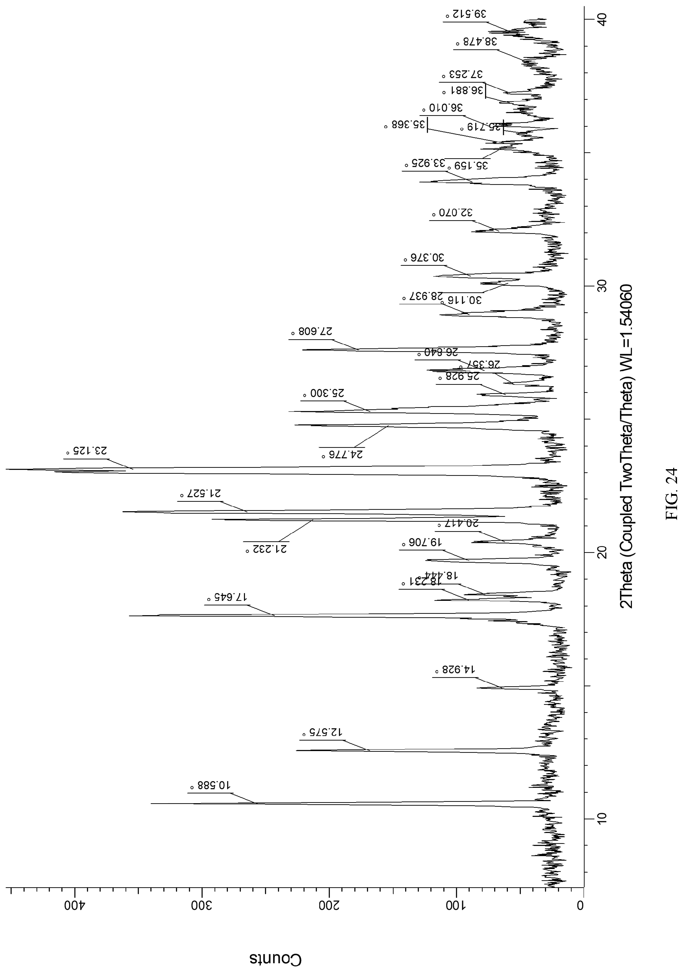

Approximately 20 mg of the compound of formula (I) and 6.3 mg of glycolic acid were weighed and placed in a 2 mL glass vial, and isopropyl acetate (0.2 mL) was added to obtain a suspension. The resulting sample was suspended and stirred at 5°C for 3 days. The resulting suspension was allowed to pass through a 0.45 μm nylon filter membrane and centrifuged at a rotation speed of 14,000 rpm, and the resulting solid was dried under vacuum at 25°C for 4 hours to obtain a product. X-ray powder diffraction detection showed that the product was a pharmaceutical co-crystal formed by the compound of formula (I) and the glycolic acid, with an XRPD pattern as shown in FIG. 24 and positions of characteristic peaks as shown in Table 7. A DSC spectrogram showed a melting $T_{onset}$ @ 135.68°C. A stoichiometric ratio of the resulting pharmaceutical co-crystal was 1:1.

**Table 7: XRPD diffraction peak data of the pharmaceutical co-crystal formed by the compound of formula (I) and the glycolic acid**

| 2θ(°) | D(Å) | Relative intensity (%) |
|---|---|---|
| 10.588 | 8.34856 | 69.90 |
| 12.575 | 7.0336 | 42.90 |

(continued)

| 2θ(°) | D(Å) | Relative intensity (%) |
|---|---|---|
| 14.928 | 5.92986 | 13.40 |
| 17.645 | 5.02244 | 67.40 |
| 18.231 | 4.8621 | 21.40 |
| 18.444 | 4.80666 | 17.70 |
| 19.706 | 4.50151 | 21.50 |
| 20.417 | 4.34628 | 12.20 |
| 21.232 | 4.18118 | 57.00 |
| 21.527 | 4.12465 | 72.50 |
| 23.125 | 3.84307 | 100.00 |
| 24.776 | 3.59063 | 38.50 |
| 25.300 | 3.51742 | 42.20 |
| 25.928 | 3.43368 | 9.80 |
| 26.357 | 3.37878 | 8.00 |
| 26.840 | 3.31902 | 15.00 |
| 27.608 | 3.22841 | 45.90 |
| 28.937 | 3.0831 | 20.80 |
| 30.116 | 2.96504 | 12.00 |
| 30.376 | 2.94025 | 20.60 |
| 32.070 | 2.78869 | 13.20 |
| 33.925 | 2.64028 | 19.70 |
| 35.159 | 2.55039 | 11.30 |
| 35.368 | 2.53583 | 10.60 |
| 35.719 | 2.51172 | 4.80 |
| 36.010 | 2.49209 | 13.70 |
| 36.881 | 2.43519 | 8.80 |
| 37.253 | 2.4117 | 9.10 |
| 38.478 | 2.33774 | 7.40 |
| 39.512 | 2.27891 | 7.90 |

**Example 17: Screening of drug-containing prescriptions**

According to properties of the compound of formula (I), prescription screening was performed by comprehensive evaluation of appearance, viscosity, spreadability, physical stability, and other aspects. Evaluation results are shown in Table 8.

**Table 8: Screening of drug-containing prescriptions**

| Ingredient | Prescription A | Prescription B | Prescription C | Prescription D |
|---|---|---|---|---|
| | Proportion % | | | |
| White bees-wax | 17 | 10 | - | - |
| Lanolin | - | - | 9 | - |

(continued)

| Ingredient | Prescription A | Prescription B | Prescription C | Prescription D |
|---|---|---|---|---|
| | Proportion % | | | |
| Paraffin | - | - | - | 17 |
| White petrolatum | 53 | 70 | 72 | 53 |
| Light liquid paraffin | 20 | 10 | 9 | 20 |
| Compound of formula (I) | 10 | 10 | 10 | 10 |
| Total | 100 | 100 | 100 | 100 |
| Appearance and properties | White ointment, appropriate viscosity, appropriate hardness, easy to apply | White ointment, appropriate viscosity, appropriate hardness, easy to apply | Light yellow ointment, low viscosity, soft ointment, quickly melts into an oily liquid after application, not suitable for application | White ointment, appropriate viscosity, high hardness, not suitable for application |

According to the above results, proportions of a prescription A and a prescription B exhibited superior ointment properties in appearance, viscosity, hardness, spreadability, and other aspects.

**Example 18: Ointment formulations containing diethylene glycol monomethyl ether and PEG400**

According to properties of the compound of formula (I), drug-containing prescriptions 1-4 were designed as shown in Table 9.

**Table 9: Drug-containing prescriptions 1-4**

| Number | Prescription 1 | Prescription 2 | Prescription 3 | Prescription 4 |
|---|---|---|---|---|
| Composition | Proportion % | | | |
| Compound of formula (I) | 1 | 1 | 1 | 1 |
| PEG400 | 55 | 55 | - | - |
| PEG3350 | 44 | 43.95 | - | - |
| BHT | - | 0.05 | - | 0.05 |
| White beeswax | - | - | 18 | 18 |
| White petrolatum | - | - | 61 | 60.95 |
| Light liquid paraffin | - | - | 10 | 10 |
| Diethylene glycol monomethyl ether | - | - | 10 | 10 |
| Total | 100 | 100 | 100 | 100 |
| Appearance and properties | Off-white ointment, appropriate viscosity, appropriate hardness, easy to apply | | Light yellow ointment, appropriate viscosity, appropriate hardness, easy to apply | |

Stability test results of the drug-containing prescriptions 1-4 are shown in Table 10 and Table 11.

**Table 10: Stability test of the drug-containing prescription 1 and the drug-containing prescription 2**

| Number | Drug-containing prescription 1 | | | | Drug-containing prescription 2 | | |
|---|---|---|---|---|---|---|---|
| Test item | Start point | 40°C/75%R H[2] | 30°C/65%R H | | Start point | 40°C/75%R H | 30°C/65%R H |
| | | 9 days | 2 weeks | | | 9 days | 2 weeks |
| Property | | Off-white ointment | | | | | |
| Related substances % | RRT[1]=0.6 9 | 0.06 | 0.08 | 0.08 | 0.06 | 0.07 | 0.08 |
| | RRT=0.8 6 | 0.08 | 0.15 | 0.13 | 0.08 | 0.12 | 0.11 |
| | RRT=0.9 1 | N.D. [3] | 0.05 | <0.05 | / | / | / |
| | RRT=0.9 3 | <0.0 5 | 0.07 | 0.06 | N.D. | <0.05 | <0.05 |
| | RRT=1.0 5 | 0.08 | 0.05 | 0.05 | 0.06 | 0.09 | 0.08 |
| | RRT=1.0 6 | N.D. | 0.05 | 0.07 | N.D. | N.D. | N.D. |
| | RRT=1.1 3 | 0.22 | 0.08 | 0.09 | 0.22 | 0.07 | 0.09 |
| | RRT=1.1 8 | <0.0 5 | 0.10 | 0.10 | <0.0 5 | 0.10 | 0.10 |
| | RRT=1.3 6 | 0.23 | 0.23 | 0.23 | 0.23 | 0.23 | 0.23 |
| | RRT=1.4 5 | 0.06 | <0.05 | <0.05 | / | / | / |
| | RRT=1.8 2 | 0.07 | 0.06 | 0.07 | 0.07 | 0.06 | 0.07 |
| | Total impuritie s | 0.80 | 0.92 | 0.88 | 0.72 | 0.79 | 0.76 |
| [1] RRT: relative retention time; [2] RH: relative humidity; [3] N.D.: not detected. | | | | | | | |

**Table 11: Stability test of the drug-containing prescription 3 and the drug-containing prescription 4**

| Number* | | Drug-containing prescription 3 | | | Drug-containing prescription 4 | |
|---|---|---|---|---|---|---|
| Test item | | Start point | 30°C/65%RH[2] | | Start point | 30°C/65%RH |
| | | | 2 weeks | 4 weeks | | 4 weeks |
| Property | | Off-white ointment | | | | |
| Related substances % | RRT[1]=0.77 | <0.05 | <0.05 | <0.05 | <0.05 | <0.05 |
| | RRT=0.86 | <0.05 | 0.05 | 0.11 | <0.05 | 0.05 |
| | RRT=0.93 | N.D.[3] | <0.05 | 0.05 | N.D. | N.D. |
| | RRT=1.05 | 0.06 | 0.07 | 0.08 | 0.05 | 0.06 |
| | RRT=1.13 | 0.35 | 0.25 | 0.11 | 0.34 | 0.12 |
| | RRT=1.16 | <0.05 | <0.05 | <0.05 | N.D. | <0.05 |
| | RRT=1.30 | N.D. | <0.05 | <0.05 | N.D. | <0.05 |
| | RRT=1.36 | 0.38 | 0.37 | 0.36 | 0.36 | 0.35 |
| | RRT=1.37 | 0.18 | 0.45 | 1.22 | 0.18 | 1.13 |
| | RRT=1.45 | N.D. | N.D. | <0.05 | N.D. | N.D. |
| | RRT=1.49 | N.D. | <0.05 | <0.05 | 0.06 | <0.05 |
| | RRT=1.82 | 0.07 | 0.06 | 0.06 | 0.06 | 0.06 |
| | Total impurities | 1.04 | 1.25 | 1.99 | 0.99 | 1.77 |
| [1] RRT: relative retention time; [2] RH: relative humidity; [3] N.D.: not detected. | | | | | | |

From the above test results, it can be seen that in a drug-containing ointment with the drug-containing prescription 1 containing a PEG matrix, impurities at RRT=0.86, RRT=1.06, and RRT=1.18 were obviously increased under accelerated stability test conditions; and after the addition of a BHT antioxidant in the drug-containing prescription 2,

increase of impurities at RRT=0.86 and RRT=1.06 was slightly improved, but impurities at RRT=1.18 still had a significant increasing trend.

In a drug-containing ointment with the drug-containing prescription 3 containing a diethylene glycol monomethyl ether matrix, impurities at RRT=0.86 and RRT=1.37 were obviously increased under accelerated stability test conditions; and after the addition of the BHT antioxidant in the drug-containing prescription 4, impurities at RRT=1.37 still had a significant changing trend.

**Example 19: Suspension ointment formulation (1%) containing the compound of formula (I)**

A suspension ointment formulation containing the compound of formula (I) was prepared, with a specific prescription as shown in Table 12.

**Table 12: Prescription information of the suspension ointment formulation (1%) containing the compound of formula (I)**

| Ingredient | Function | Use amount % (w/w) |
|---|---|---|
| Compound of formula (I) | Active ingredient | 1 |
| White beeswax | Ointment matrix | 18 |
| White petrolatum | Ointment matrix | 58 |
| Light liquid paraffin | Ointment matrix | 23 |

A preparation method includes:

(1) weighing the white beeswax and the white petrolatum, heating up to 65-70°C for melting in a water bath, and homogenizing at a stirring speed of 350 rpm for 20 minutes to obtain a homogeneous mixture A;
(2) weighing the light liquid paraffin, adding the weighed compound of formula (I) thereto, heating up to approximately 40°C in a water bath, and stirring at a stirring speed of 400 rpm for 10 minutes until the active ingredient is completely dispersed to obtain a homogeneous mixture B; and
(3) cooling the mixture A to 40°C, adding the mixture B into the mixture A, continually stirring, then homogenizing using a homogenizer at a speed of 10,000 rpm for 2 minutes, and then cooling to room temperature to obtain the suspension ointment formulation containing the compound of formula (I).

**Example 20: Suspension ointment formulation (3%) containing the compound of formula (I)**

A suspension ointment formulation containing the compound of formula (I) was prepared by the same preparation method as Example 19, with a specific prescription as shown in Table 13.

**Table 13: Prescription information of the suspension ointment formulation (3%) containing the compound of formula (I)**

| Ingredient | Function | Use amount % (w/w) |
|---|---|---|
| Compound of formula (I) | Active ingredient | 3 |
| White beeswax | Ointment matrix | 18 |
| White petrolatum | Ointment matrix | 57 |
| Light liquid paraffin | Ointment matrix | 22 |

**Example 21: Suspension ointment formulation (10%) containing the compound of formula (I)**

A suspension ointment formulation containing the compound of formula (I) was prepared by the same preparation method as Example 19, with a specific prescription as shown in Table 14.

**Table 14: Prescription information of the suspension ointment formulation (10%) containing the compound of formula (I)**

| Ingredient | Function | Use amount % (w/w) |
|---|---|---|
| Compound of formula (I) | Active ingredient | 10 |
| White beeswax | Ointment matrix | 20 |
| White petrolatum | Ointment matrix | 40 |
| Light liquid paraffin | Ointment matrix | 30 |

The ointment prepared in this example had appropriate viscosity and appropriate hardness and was easy to apply.

**Example 22: Stability test of binary mixtures of the compound of formula (I) and penetration enhancers**

Different types of penetration enhancers and the compound of formula (I) were mixed at a weight ratio of 10:1 to carry out a stability test under accelerated conditions of 40°C/75%RH. Test results are shown in Table 15.

**Table 15: Stability test of binary mixtures of the compound of formula (I) and penetration enhancers**

| Related substances (%) | Formula (I) + isopropyl myristate | | | Formula (I) + propylene carbonate | | Formula (I) + diisopropyl oxalate | |
|---|---|---|---|---|---|---|---|
| | Start point | 1 week | 3 weeks | Start point | 1 week | Start point | 1 week |
| RRT[1]=0.58 | / | / | / | N.D. [2] | 0.06 | / | / |
| RRT=0.69 | 0.07 | 0.07 | 0.07 | 0.08 | 0.10 | 0.06 | 0.08 |
| RRT=0.86 | 0.08 | 0.08 | 0.10 | 0.31 | 0.95 | 0.12 | 0.20 |
| RRT=0.93 | / | / | / | 0.12 | 0.32 | 0.08 | 0.15 |
| RRT=1.05 | 0.07 | 0.07 | 0.07 | 0.05 | 0.24 | 0.06 | 0.08 |
| RRT=1.06 | / | / | / | 0.06 | 0.10 | / | / |
| RRT=1.13 | 0.32 | 0.29 | 0.27 | 0.18 | 0.13 | / | / |
| RRT=1.36 | 0.22 | 0.23 | 0.21 | 0.23 | 0.22 | 0.21 | 0.23 |
| RRT=1.82 | 0.07 | 0.06 | 0.05 | 0.07 | 0.05 | 0.06 | 0.05 |
| Total impurities | 0.82 | 0.81 | 0.77 | 1.09 | 2.16 | 0.59 | 0.80 |
| [1] RRT: relative retention time; [2] N.D.: not detected. | | | | | | | |

The results showed that the compound of formula (I) was unstable in both propylene carbonate and diisopropyl oxalate.

**Example 23: Suspension ointment formulations containing the compound of formula (I) and a penetration enhancer**

Drug-containing prescriptions 5-6 were designed as shown in Table 16.

**Table 16: Proportions of the drug-containing prescriptions 5-6**

| Number | Drug-containing prescription 5 | Drug-containing prescription 6 |
|---|---|---|
| Composition | Proportion % | |
| Compound of formula (I) | 1 | 10 |
| White beeswax | 18 | 20 |
| White petrolatum | 58 | 40 |

(continued)

| Number | Drug-containing prescription 5 | Drug-containing prescription 6 |
|---|---|---|
| Composition | Proportion % | |
| Light liquid paraffin | 21 | 20 |
| Propylene glycol | 2 | - |
| Isopropyl myristate | - | 10 |
| Total | 100 | 100 |

Stability test results of the drug-containing prescriptions 5-6 are shown in Table 17.

**Table 17: Stability test of the drug-containing prescriptions 5-6**

| Number | | Drug-containing prescription 5 | | | Drug-containing prescription 6 | | |
|---|---|---|---|---|---|---|---|
| Test item | | Start point | 40°C/75%RH[2] | 30°C/65%RH | Start point | 40°C/75%RH | |
| | | | 2 weeks | 2 weeks | | 2 weeks | 4 weeks |
| Property | | Off-white ointment | | | | | |
| Related substances (%) | RRT[1]=0.86 | 0.05 | 0.13 | 0.08 | 0.05 | 0.06 | 0.06 |
| | RRT=0.93 | <0.05 | 0.13 | 0.08 | N.D. | N.D. | <0.05 |
| | RRT=1.05 | <0.05 | 0.06 | 0.08 | <0.05 | 0.07 | <0.05 |
| | RRT=1.06 | N.D. | 0.06 | N.D. | N.D. | <0.05 | N.D. |
| | RRT=1.11 | N.D. | N.D.[3] | N.D. | N.D. | N.D. | N.D. |
| | RRT=1.13 | 0.29 | 0.05 | 0.08 | 0.35 | 0.19 | 0.12 |
| | RRT=1.21 | N.D. | 0.13 | 0.05 | N.D. | N.D. | N.D. |
| | RRT=1.36 | 0.24 | 0.29 | 0.26 | 0.21 | 0.23 | 0.22 |
| | RRT=1.82 | 0.06 | 0.05 | 0.08 | 0.06 | 0.07 | 0.07 |
| | Total impurities | 0.82 | 1.02 | 0.82 | 0.75 | 0.68 | 0.53 |
| [1] RRT: relative retention time; [2] RH: relative humidity; [3] N.D.: not detected. | | | | | | | |

The above test results showed that in the drug-containing prescription 5 containing propylene glycol as a penetration enhancer, impurities at RRT=0.86, RRT=0.93, RRT=1.05, RRT=1.21, and RRT=1.36 were obviously increased under accelerated stability test conditions. The drug-containing prescription 6 containing isopropyl myristate as a penetration enhancer had good overall stability.

**Example 24: Suspension ointment formulation (1%) containing the compound of formula (I) and a penetration enhancer**

A suspension ointment formulation containing the compound of formula (I) and a penetration enhancer was prepared, with a specific prescription as shown in Table 18.

**Table 18: Prescription information of the suspension ointment formulation (1%) containing the compound of formula (I) and the penetration enhancer**

| Ingredient | Function | Use amount % (w/w) |
|---|---|---|
| Compound of formula (I) | Active ingredient | 1 |
| White beeswax | Ointment matrix | 18 |
| White petrolatum | Ointment matrix | 58 |
| Light liquid paraffin | Ointment matrix | 21 |

(continued)

| Ingredient | Function | Use amount % (w/w) |
|---|---|---|
| Isopropyl myristate | Penetration enhancer | 2 |

A preparation method includes:

(1) weighing the white beeswax, the white petrolatum, and the isopropyl myristate, heating up to 65-70°C for melting in a water bath, and homogenizing at a stirring speed of 350 rpm for 15 minutes to obtain a homogeneous mixture A;
(2) weighing the light liquid paraffin, adding the weighed compound of formula (I) thereto, heating up to approximately 40°C in a water bath, and stirring at a stirring speed of 300 rpm for 10 minutes until the active ingredient is completely dispersed to obtain a homogeneous mixture B; and
(3) cooling the mixture A to 40°C, adding the mixture B into the mixture A, continually stirring, then homogenizing using a homogenizer at a speed of 10,000 rpm for 2 minutes, and then cooling to room temperature to obtain the suspension ointment formulation containing the compound of formula (I) and the penetration enhancer.

**Example 25: Suspension ointment formulation (10%) containing the compound of formula (I) and a penetration enhancer**

A suspension ointment formulation containing the compound of formula (I) and a penetration enhancer was prepared by the same preparation method as Example 24, with a specific prescription as shown in Table 19.

**Table 19: Prescription information of the suspension ointment formulation (10%) containing the compound of formula (I) and the penetration enhancer**

| Ingredient | Function | Use amount % (w/w) |
|---|---|---|
| Compound of formula (I) | Active ingredient | 10 |
| White beeswax | Ointment matrix | 20 |
| White petrolatum | Ointment matrix | 40 |
| Light liquid paraffin | Ointment matrix | 20 |
| Isopropyl myristate | Penetration enhancer | 10 |

**Example 26: Suspension ointment formulation (1%) containing the compound of formula (I), a penetration enhancer, and an antioxidant**

A suspension ointment formulation containing the compound of formula (I), a penetration enhancer, and an antioxidant was prepared, with a specific prescription as shown in Table 20.

**Table 20: Prescription information of the suspension ointment formulation (1%) containing the compound of formula (I) , the penetration enhancer, and the antioxidant**

| Ingredient | Function | Use amount % (w/w) |
|---|---|---|
| Compound of formula (I) | Active ingredient | 1 |
| White beeswax | Ointment matrix | 18 |
| White petrolatum | Ointment matrix | 58 |
| Light liquid paraffin | Ointment matrix | 20.9 |
| Isopropyl myristate | Penetration enhancer | 2 |
| Butylated hydroxytoluene (BHT) | Antioxidant | 0.1 |

A preparation method includes:

(1) weighing the white beeswax, the white petrolatum, the isopropyl myristate, and the butylated hydroxytoluene (BHT), heating up to 65-70°C for melting in a water bath, and homogenizing at a stirring speed of 350 rpm for 15

minutes to obtain a homogeneous mixture A;
(2) weighing the light liquid paraffin, adding the weighed compound of formula (I) thereto, heating up to approximately 40°C in a water bath, and stirring at a stirring speed of 300 rpm for 10 minutes until the active ingredient is completely dispersed to obtain a homogeneous mixture B; and
(3) cooling the mixture A to 40°C, adding the mixture B into the mixture A, continually stirring, then homogenizing using a homogenizer at a speed of 10,000 rpm for 2 minutes, and then cooling to room temperature to obtain the suspension ointment formulation containing the compound of formula (I), the penetration enhancer, and the antioxidant.

After being placed under accelerated conditions of 40°C/75% for 1 month, the ointment with the specification of 1% containing the isopropyl myristate and the antioxidant BHT had good overall stability. Results are shown in Table 21.

**Table 21: Stability test results of the ointment containing the penetration enhancer and the antioxidant**

| Placing conditions | | 40°C/75%RH[2] | |
|---|---|---|---|
| Time point | | Start point | 4 weeks |
| Related substances (%) | RRT[1]=0.69 | 0.05 | <0.05 |
| | RRT=0.76 | N.D.[3] | <0.05 |
| | RRT=0.86 | <0.05 | <0.05 |
| | RRT=1.05 | 0.05 | 0.06 |
| | RRT=1.13 | 0.30 | 0.12 |
| | RRT=1.16 | <0.05 | <0.05 |
| | RRT=1.36 | 0.18 | 0.20 |
| | RRT=1.56 | N.D. | <0.05 |
| | RRT=1.83 | <0.05 | <0.05 |
| | Total impurities | 0.58 | 0.38 |
| [1] RRT: relative retention time; [2] RH: relative humidity; [3] N.D.: not detected. | | | |

**Example 27: Suspension ointment formulation (0.3%) containing the compound of formula (I)**

A suspension ointment formulation containing the compound of formula (I) was prepared by the same preparation method as Example 19, with a specific prescription as shown in Table 22.

**Table 22: Prescription information of the suspension ointment formulation (0.3%) containing the compound of formula (I)**

| Ingredient | Function | Use amount % (w/w) |
|---|---|---|
| Compound of formula (I) | Active ingredient | 0.3 |
| White beeswax | Ointment matrix | 18 |
| White petrolatum | Ointment matrix | 58.7 |
| Light liquid paraffin | Ointment matrix | 23 |

**Example 28: Pharmacokinetic study after single topical administration of an ointment containing the compound of formula (I) to the skin of CD-1 mice**

6 CD-1 mice were divided into 2 groups, with 3 mice per group, and then respectively received an ointment containing the compound of formula (I) by single topical administration to the backs at 1 mg/cm$^2$ (3% suspension ointment) with an application area of 1 cm$^2$. The plasma concentration and skin tissue homogenate concentration of the compound of formula (I) were determined by an LC-MS/MS method.
At 4 h, a plasma drug concentration of the compound of formula (I) in the first group of 3 CD-1 mice was 6.15 ng-h/mL. Meanwhile, a dorsal skin tissue homogenate was collected from the CD-1 mice at 4 h, and a mean drug concentration of the compound of formula (I) in the skin was 33,033 ng/g.

In the second group of 3 CD-1 mice, a mean area under the plasma drug concentration-time curve ($AUC_{0\text{-}last}$) of the compound of formula (I) from time 0 to the last quantifiable concentration time (24 h) was 40.7 ng·h/mL. A mean $C_{max}$ of the compound of formula **(I)** in plasma was 14.0 ng/mL, the time to reach maximum concentration ($T_{max}$) occurred at 1.67 h after administration, and a mean elimination half-life ($T_{1/2}$) was 5.15 h. For the three CD-1 mice, a dorsal skin tissue homogenate was collected at the endpoint of 24 h, and a mean drug concentration of the compound of formula (I) in the skin was 7,907 ng/g. At 24 h, a drug concentration of the compound of formula (I) in a plasma sample of the CD-1 mice was below a lower detection limit.

The concentration of the compound of formula (I) in skin tissues was much higher than the drug concentration in plasma.

At this dose, all the animals were tolerant. No abnormal manifestations were observed during the entire study process.

Mean PK parameters of the compound of formula (I) after single topical administration to the dorsal skin of the CD-1 mice are summarized in Table 23.

**Table 23: Pharmacokinetic parameters of the compound of formula (I) after single transdermal administration to the CD-1 mice**

| Administration route | | Topical administration to the skin |
|---|---|---|
| Prescription | | 3% suspension ointment |
| Dose level | | 1mg/cm$^2$ |
| Application area | | 1cm$^2$ |
| PK results | | Mean value |
| Plasma | $C_{max}$ (ng/mL) | 14.0 |
| | $T_{max}$ (h) | 1.67 |
| | $T_{1/2}$ (h) | 5.15 |
| | $AUC_{0\text{-}last}$ (ng·h/mL) | 40.7 |
| | $AUC_{0\text{-}inf}$ (ng·h/mL) | 57.5 |
| | AUC Extr (%) | 18.9 |
| | MRTInf (hr) | 5.54 |
| | AUCInf/Dose (hr·kg·ng/mL/mg) | 1.63 |
| | Plasma drug concentration at 4 h (ng/mL) | 6.15 |
| | Plasma drug concentration at 24 h (ng/mL) | Below a lower detection limit |
| Skin | Drug concentration in skin tissues at 4 h (ng/g) | 33033 |
| | Drug concentration in skin tissues at 24 h (ng/g) | 7907 |

**Example 29: Pharmacokinetic study after single topical administration of an ointment containing the compound of formula (I) to the skin of Bama miniature pigs**

2 Bama miniature pigs respectively received an ointment containing the compound of formula (I) by single topical application to the dorsal and abdominal skin at 20 mg/cm$^2$ (3% suspension ointment), with a total application area of 6.8% of a body surface area, and a ratio of application areas between the dorsal and abdominal skin being approximately 1:1. The plasma concentration and skin tissue homogenate concentration of the compound of formula (I) were determined by an LC-MS/MS method.

For the 2 Bama miniature pigs, a mean area under the plasma drug concentration-time curve ($AUC_{0\text{-}last}$) of the compound of formula (I) from time 0 to the last quantifiable concentration time (24 h) was 304 ng·h/mL. A mean $C_{max}$ of the compound of formula (I) in plasma was 21.6 ng/mL, and the time to reach maximum concentration ($T_{max}$) occurred at 16 h after administration. A mean plasma drug concentration at the endpoint of 24 h was 17.7 ng/mL.

A skin tissue homogenate was collected from the 2 Bama miniature pigs at the endpoint of 24 h. At 24 h, a mean drug concentration of the compound of formula (I) in collected dorsal dermis skin tissues was 88,336 ng/g; at 24 h, a mean drug concentration of the compound of formula (I) in a collected dorsal epidermis skin tissue sample was 1,109,245 ng/g; and at 24 h, a mean drug concentration of the compound of formula (I) in a collected abdominal skin tissue sample was

110,952 ng/g. The concentration of the compound of formula (I) in each skin tissue was much higher than the drug concentration in plasma.

At this dose, all the animals were tolerant. No abnormal manifestations were observed during the entire study process.

Mean PK parameters of the compound of formula (I) after single topical administration to the dorsal skin of the Bama miniature pigs are summarized in Table 24.

**Table 24: Pharmacokinetic parameters of the compound of formula (I) after single transdermal administration to the Bama miniature pigs**

| Administration route | | Topical administration to the skin |
|---|---|---|
| Prescription | | 3% suspension ointment |
| Dose level | | 20 mg/cm$^2$ |
| Application area | | 10% of a body surface area |
| PK results | | Mean value |
| Plasma | $C_{max}$ (ng/mL) | 21.6 |
| | $T_{max}$ (h) | 16 |
| | $AUC_{0\text{-}last}$ (ng·h/mL) | 304 |
| | MRTInf (hr) | 15.2 |
| | Plasma drug concentration at 24 h (ng/mL) | 17.7 |
| Skin | Drug concentration in dorsal dermis skin tissues at 24 h (ng/g) | 88336 |
| | Drug concentration in dorsal epidermis skin tissues at 24 h (ng/g) | 1109245 |
| | Drug concentration in abdominal skin tissues at 24 h (ng/g) | 110952 |

**Example 30: Pharmacokinetic study after single topical administration of an ointment containing the compound of formula (I) to the skin of SD rats**

6 SD rats were divided into two groups, with 3 rats per group, where one group received an ointment containing the compound of formula (I) by single topical application to the dorsal skin at 20 mg/cm$^2$ (3% suspension ointment), with an application area of 20 cm$^2$, and the other group received an ointment containing the compound of formula (I) by single topical application to the dorsal skin at 20 mg/cm$^2$ (10% suspension ointment), with an application area of 20 cm$^2$. The plasma concentration and dorsal skin tissue homogenate concentration of the compound of formula (I) were determined by an LC-MS/MS method.

For the 3 SD rats receiving the 3% suspension ointment, a mean plasma drug concentration at the endpoint of 24 h was 3.21 ng/mL, and a drug concentration in a dorsal skin tissue homogenate at the endpoint of 24 h was 161,167 ng/g. For the 3 SD rats receiving the 10% suspension ointment, a mean plasma drug concentration at the endpoint of 24 h was 17.3 ng/mL, and a drug concentration in a dorsal skin tissue homogenate at the endpoint of 24 h was 799,500 ng/g. The concentration of the compound of formula (I) in skin tissues was much higher than the drug concentration in plasma.

At this dose, all the animals were tolerant. No abnormal manifestations were observed during the entire study process.

Mean PK parameters of the compound of formula (I) after single topical administration to the dorsal skin of the SD rats are summarized in Table 25.

**Table 25: Pharmacokinetic parameters of the compound of formula (I) after single transdermal administration to the SD rats**

| Administration route | Topical administration to the skin | |
|---|---|---|
| Prescription | 3% suspension ointment | 10% suspension ointment |
| Dose level | 20mg/cm$^2$ | 20mg/cm$^2$ |
| Application area | 20 cm$^2$ | 20 cm$^2$ |
| PK results | Mean value | Mean value |

(continued)

| Administration route | Topical administration to the skin | |
|---|---|---|
| Prescription | 3% suspension ointment | 10% suspension ointment |
| Plasma drug concentration at 24 h (ng/mL) | 3.21 | 17.3 |
| Drug concentration in skin tissues at 24 h (ng/g) | 161167 | 799500 |

From the above PK results, it can be seen that after topical administration of the suspension ointments of the compound of formula (I) to the skin of the mice, the rats, and the pigs, their exposure amounts in the plasma were much lower than those in the skin. The drug ointments had low systemic exposure amounts and high safety in the treatment of skin-related diseases.

**Example 31: Stability study of suspension ointment prescriptions**

The 1% suspension ointment formulation, the 3% suspension ointment formulation, and the 10% suspension ointment formulation that were formulated by the methods in Example 19, Example 20, and Example 21 were respectively placed under two different conditions of 25°C/60%RH and 40°C/75%RH for 3 months and 1 month, and changes in related substance contents were detected by HPLC to observe prescription stability situations. Results are shown in Table 26. The formulations had small changes in related substances and generally stable prescriptions.

**Table 26: Stability study of the suspension ointment formulations**

| Specification | 1% suspension ointment formulation | | | |
|---|---|---|---|---|
| Test item | Initial | 40°C/75%RH [2] | | 25°C/60%RH |
| | | 1 month | | 3 months |
| Appearance | | White ointment | White ointment | White ointment |
| Related substances (%) | RRT[1]=0.87 | <0.05 | <0.05 | <0.05 |
| | RRT=0.94 | 0.07 | 0.07 | 0.07 |
| | RRT=1.05 | N.D.[3] | N.D. | N.D. |
| | RRT=1.13 | N.D. | N.D. | N.D. |
| | RRT=1.16 | N.D. | N.D. | N.D. |
| | RRT=1.36 | N.D. | N.D. | N.D. |
| | RRT=1.82 | N.D. | N.D. | N.D. |
| | Total impurities | 0.07 | 0.07 | 0.07 |
| Specification | | 3% suspension ointment formulation | | |
| Test item | | Initial | 40°C/75%RH | 25°C/60%RH |
| | | | 1 month | 3 months |
| Appearance | | White ointment | White ointment | White ointment |
| Related substances (%) | RRT=0.87 | N.D. | N.D. | N.D. |
| | RRT=0.94 | 0.05 | 0.06 | 0.07 |
| | RRT=1.05 | N.D. | N.D. | N.D. |
| | RRT=1.13 | N.D. | N.D. | N.D. |
| | RRT=1.16 | N.D. | N.D. | N.D. |
| | RRT=1.36 | N.D. | N.D. | N.D. |
| | RRT=1.82 | N.D. | N.D. | N.D. |
| | Total impurities | 0.05 | 0.06 | 0.07 |

(continued)

| Specification | | 10% suspension ointment formulation | | |
|---|---|---|---|---|
| Test item | | Initial | 40°C/75%RH | 25°C/60%RH |
| | | | 1 month | 3 months |
| Appearance | | White ointment | White ointment | White ointment |
| Related substances (%) | RRT=0.87 | N.D. | N.D. | N.D. |
| | RRT=0.94 | 0.05 | 0.07 | 0.06 |
| | RRT=1.05 | N.D. | N.D. | N.D. |
| | RRT=1.13 | N.D. | N.D. | N.D. |
| | RRT=1.16 | N.D. | N.D. | N.D. |
| | RRT=1.36 | N.D. | N.D. | N.D. |
| | RRT=1.82 | N.D. | N.D. | N.D. |
| | Total impurities | 0.05 | 0.07 | 0.06 |
| [1] RRT: relative retention time; [2] RH: relative humidity; [3] N.D.: not detected. | | | | |

The three drug-containing prescriptions of Examples 19, 20, and 21 exhibited excellent performance in the stability test, had appropriate comprehensive ointment performance in aspects such as appearance, viscosity, hardness, and spreadability, also comprehensively considered the feasibility of large-scale production, and had simple and commercially available auxiliary material ingredients, thus being formulation prescriptions highly suitable for clinical development of the compound of formula (I).

It should be understood that the above examples are all exemplary and are not intended to encompass all possible embodiments covered by the claims. Without departing from the scope of the present disclosure, various modifications and alterations may also be made based on the above examples. Similarly, various technical features of the above examples may also be arbitrarily combined to form other examples of the present disclosure that may not have been explicitly described. Therefore, the above examples merely express several embodiments of the present disclosure and do not limit the scope of protection of the present disclosure.

## Claims

**1.** An RNA m6A regulator composition, comprising:
a compound of formula (I) or a pharmaceutically acceptable salt thereof or a pharmaceutically acceptable co-crystal thereof, and an oil phase substance,

Cl
N
H
O
$NH_2$

(I).

**2.** The composition according to claim 1, wherein

the pharmaceutically acceptable salt comprises hydrochloride, sulfate, phosphate, acetate, methanesulfonate, benzenesulfonate, and p-toluenesulfonate;
preferably, a co-crystal former of the pharmaceutically acceptable co-crystal comprises nicotinamide, isonicotinamide, L-proline, and glycolic acid;
preferably, the oil phase substance comprises an oil phase substance 1, an oil phase substance 2, and an oil phase substance 3;
preferably, the oil phase substance 1 is selected from one or more of lanolin, beeswax, white beeswax, and

paraffin, and preferably white beeswax;
preferably, the oil phase substance 2 is selected from one or more of petrolatum and white petrolatum, and preferably white petrolatum; and
preferably, the oil phase substance 3 is selected from one or more of light liquid paraffin and vegetable oil, and preferably light liquid paraffin.

**3.** The composition according to claim 2, wherein

in terms of mass percentage, the composition comprises 0.1%-20% of the compound of formula (I) or the pharmaceutically acceptable salt thereof or the pharmaceutically acceptable co-crystal thereof, 10%-30% of the oil phase substance 1, 30%-70% of the oil phase substance 2, and 15%-40% of the oil phase substance 3;
preferably, in terms of mass percentage, the composition comprises 0.2%-15% of the compound of formula (I) or the pharmaceutically acceptable salt thereof or the pharmaceutically acceptable co-crystal thereof, 15%-25% of the oil phase substance 1, 35%-64% of the oil phase substance 2, and 20%-35% of the oil phase substance 3;
preferably, in terms of mass percentage, the composition comprises 0.3%-10% of the compound of formula (I) or the pharmaceutically acceptable salt thereof or the pharmaceutically acceptable co-crystal thereof, 18%-20% of the oil phase substance 1, 40%-58.7% of the oil phase substance 2, and 22%-30% of the oil phase substance 3;
preferably, in terms of mass percentage, the composition comprises 0.3% of the compound of formula (I) or the pharmaceutically acceptable salt thereof or the pharmaceutically acceptable co-crystal thereof, 18% of the oil phase substance 1, 58.7% of the oil phase substance 2, and 23% of the oil phase substance 3;
preferably, in terms of mass percentage, the composition comprises 1% of the compound of formula (I) or the pharmaceutically acceptable salt thereof or the pharmaceutically acceptable co-crystal thereof, 18% of the oil phase substance 1, 58% of the oil phase substance 2, and 23% of the oil phase substance 3;
preferably, in terms of mass percentage, the composition comprises 3% of the compound of formula (I) or the pharmaceutically acceptable salt thereof or the pharmaceutically acceptable co-crystal thereof, 18% of the oil phase substance 1, 57% of the oil phase substance 2, and 22% of the oil phase substance 3; and
preferably, in terms of mass percentage, the composition comprises 10% of the compound of formula (I) or the pharmaceutically acceptable salt thereof or the pharmaceutically acceptable co-crystal thereof, 20% of the oil phase substance 1, 40% of the oil phase substance 2, and 30% of the oil phase substance 3.

**4.** The composition according to claim 3, wherein

the composition comprises the compound of formula (I) or the pharmaceutically acceptable salt thereof or the pharmaceutically acceptable co-crystal thereof, the white beeswax, the white petrolatum, and the light liquid paraffin;
preferably, in terms of mass percentage, the composition comprises 0.1%-20% of the compound of formula (I) or the pharmaceutically acceptable salt thereof or the pharmaceutically acceptable co-crystal thereof, 10%-30% of the white beeswax, 30%-70% of the white petrolatum, and 15%-40% of the light liquid paraffin;
preferably, in terms of mass percentage, the composition comprises 0.2%-15% of the compound of formula (I) or the pharmaceutically acceptable salt thereof or the pharmaceutically acceptable co-crystal thereof, 15%-25% of the white beeswax, 35%-64% of the white petrolatum, and 20%-35% of the light liquid paraffin;
preferably, in terms of mass percentage, the composition comprises 0.3%-10% of the compound of formula (I) or the pharmaceutically acceptable salt thereof or the pharmaceutically acceptable co-crystal thereof, 18%-20% of the white beeswax, 40%-58.7% of the white petrolatum, and 22%-30% of the light liquid paraffin;
preferably, in terms of mass percentage, the composition comprises 0.3% of the compound of formula (I), 18% of the white beeswax, 58.7% of the white petrolatum, and 23% of the light liquid paraffin;
preferably, in terms of mass percentage, the composition comprises 1% of the compound of formula (I), 18% of the white beeswax, 58% of the white petrolatum, and 23% of the light liquid paraffin;
preferably, in terms of mass percentage, the composition comprises 3% of the compound of formula (I), 18% of the white beeswax, 57% of the white petrolatum, and 22% of the light liquid paraffin; and
preferably, in terms of mass percentage, the composition comprises 10% of the compound of formula (I), 20% of the white beeswax, 40% of the white petrolatum, and 30% of the light liquid paraffin.

**5.** The composition according to any one of claims 1-4, wherein

preferably, the composition further comprises a penetration enhancer, more preferably, the penetration enhancer is selected from one or more of isopropyl myristate, isopropyl palmitate, propylene glycol dipelargonate, diethyl sebacate, laurocapram, and propylene glycol, and further preferably, the penetration enhancer is isopropyl

myristate;
preferably, the composition further comprises an antioxidant, more preferably, the antioxidant is selected from one or more of vitamin E, alkyl gallate, butylated hydroxyanisole (BHA), and butylated hydroxytoluene (BHT), and further preferably, the antioxidant is butylated hydroxytoluene (BHT); and
preferably, the composition further comprises other pharmaceutically acceptable auxiliary materials.

**6.** The composition according to claim 5, wherein

in terms of mass percentage, the composition comprises 0.1%-20% of the compound of formula (I) or the pharmaceutically acceptable salt thereof or the pharmaceutically acceptable co-crystal thereof, 10%-30% of the oil phase substance 1, 30%-70% of the oil phase substance 2, 15%-30% of the oil phase substance 3, 0.1%-20% of the penetration enhancer, and 0%-2% of the antioxidant;
preferably, in terms of mass percentage, the composition comprises 0.2%-15% of the compound of formula (I) or the pharmaceutically acceptable salt thereof or the pharmaceutically acceptable co-crystal thereof, 15%-25% of the oil phase substance 1, 35%-63% of the oil phase substance 2, 20%-25% of the oil phase substance 3, 1%-15% of the penetration enhancer, and 0%-1% of the antioxidant; and
preferably, in terms of mass percentage, the composition comprises 0.3%-10% of the compound of formula (I) or the pharmaceutically acceptable salt thereof or the pharmaceutically acceptable co-crystal thereof, 18%-20% of the oil phase substance 1, 40%-58% of the oil phase substance 2, 20%-21% of the oil phase substance 3, 2%-10% of the penetration enhancer, and 0%-0.2% of the antioxidant.

**7.** The composition according to claim 6, wherein

the composition comprises the compound of formula (I) or the pharmaceutically acceptable salt thereof or the pharmaceutically acceptable co-crystal thereof, the white beeswax, the white petrolatum, the light liquid paraffin, and the isopropyl myristate;
preferably, in terms of mass percentage, the composition comprises 0.1%-20% of the compound of formula (I) or the pharmaceutically acceptable salt thereof or the pharmaceutically acceptable co-crystal thereof, 10%-30% of the white beeswax, 30%-70% of the white petrolatum, 15%-30% of the light liquid paraffin, and 0.1%-20% of the isopropyl myristate;
preferably, in terms of mass percentage, the composition comprises 0.2%-15% of the compound of formula (I) or the pharmaceutically acceptable salt thereof or the pharmaceutically acceptable co-crystal thereof, 15%-25% of the white beeswax, 35%-63% of the white petrolatum, 20%-25% of the light liquid paraffin, and 1%-15% of the isopropyl myristate;
preferably, in terms of mass percentage, the composition comprises 0.3%-10% of the compound of formula (I) or the pharmaceutically acceptable salt thereof or the pharmaceutically acceptable co-crystal thereof, 18%-20% of the white beeswax, 40%-58% of the white petrolatum, 20%-21% of the light liquid paraffin, and 2%-10% of the isopropyl myristate;
preferably, in terms of mass percentage, the composition comprises 1% of the compound of formula (I), 18% of the white beeswax, 58% of the white petrolatum, 21% of the light liquid paraffin, and 2% of the isopropyl myristate;
preferably, in terms of mass percentage, the composition comprises 10% of the compound of formula (I), 20% of the white beeswax, 40% of the white petrolatum, 20% of the light liquid paraffin, and 10% of the isopropyl myristate;
preferably, the composition comprises the compound of formula (I) or the pharmaceutically acceptable salt thereof or the pharmaceutically acceptable co-crystal thereof, the white beeswax, the white petrolatum, the light liquid paraffin, the isopropyl myristate, and the butylated hydroxytoluene (BHT);
preferably, in terms of mass percentage, the composition comprises 0.1%-20% of the compound of formula (I) or the pharmaceutically acceptable salt thereof or the pharmaceutically acceptable co-crystal thereof, 10%-30% of the white beeswax, 30%-70% of the white petrolatum, 15%-30% of the light liquid paraffin, 0.1%-20% of the isopropyl myristate, and 0%-2% of the butylated hydroxytoluene (BHT);
preferably, in terms of mass percentage, the composition comprises 0.2%-15% of the compound of formula (I) or the pharmaceutically acceptable salt thereof or the pharmaceutically acceptable co-crystal thereof, 15%-25% of the white beeswax, 35%-63% of the white petrolatum, 20%-25% of the light liquid paraffin, 1%-15% of the isopropyl myristate, and 0%-1% of the butylated hydroxytoluene (BHT);
preferably, in terms of mass percentage, the composition comprises 0.3%-10% of the compound of formula (I) or the pharmaceutically acceptable salt thereof or the pharmaceutically acceptable co-crystal thereof, 18%-20% of the white beeswax, 40%-58% of the white petrolatum, 20%-21% of the light liquid paraffin, 2%-10% of the isopropyl myristate, and 0%-0.2% of the butylated hydroxytoluene (BHT); and

preferably, in terms of mass percentage, the composition comprises 1% of the compound of formula (I), 18% of the white beeswax, 58% of the white petrolatum, 20.9% of the light liquid paraffin, 2% of the isopropyl myristate, and 0.1% of the butylated hydroxytoluene (BHT).

**8.** A method for preparing the composition according to any one of claims 1-7, wherein

when the composition does not comprise the penetration enhancer and/or the antioxidant, the method comprises:

(1) weighing the oil phase substance 1 and the oil phase substance 2, heating to melt in a water bath, and homogenizing to obtain a homogeneous mixture A;
(2) weighing the oil phase substance 3, adding the weighed compound of formula (I) or the pharmaceutically acceptable salt thereof or the pharmaceutically acceptable co-crystal thereof, heating in a water bath, and stirring to obtain a homogeneous mixture B; and
(3) cooling the mixture A, then adding the mixture B into the mixture A, stirring, then homogenizing, and cooling to obtain the composition; and

when the composition comprises the penetration enhancer and/or the antioxidant, the method comprises:

(1) weighing the oil phase substance 1, the oil phase substance 2, the penetration enhancer and/or the antioxidant, heating to melt in a water bath, and homogenizing to obtain a homogeneous mixture A;
(2) weighing the oil phase substance 3, adding the weighed compound of formula (I) or the pharmaceutically acceptable salt thereof or the pharmaceutically acceptable co-crystal thereof, heating in a water bath, and stirring to obtain a homogeneous mixture B; and
(3) cooling the mixture A, then adding the mixture B into the mixture A, stirring, then homogenizing, and cooling to obtain the composition.

**9.** Use of the RNA m6A regulator composition according to any one of claims 1-7 or the method according to claim 8 in preparation of a formulation for preventing or treating a skin disease caused by RNA m6A methylation, wherein

preferably, the skin disease comprises acanthosis, eczema, ichthyosis, psoriasis, keratosis, systemic lupus erythematosus, a hand-foot syndrome, a hand-foot skin reaction, and dermatitis; and
more preferably, the skin disease comprises a hand-foot syndrome and a hand-foot skin reaction.

**10.** The use according to claim 9, wherein

the formulation of the RNA m6A regulator composition comprises an ointment, an emulsifiable paste, a gel, a cream, an emulsion, a lotion, a solution, a paste, a film, an oil formulation, or a patch;
preferably, the formulation of the RNA m6A regulator composition is an ointment; and
more preferably, the formulation of the RNA m6A regulator composition is a suspension ointment.

Cellar m6A level
Relative m6A methylation level
8
6
4
2
0
Blank control group
Sorafenib group
Capecitabine group
Docetaxel group
Osimertinib group
NAP1L2 m6A level
Relative m6A methylation level
25
20
15
10
5
0
Blank control group
Sorafenib group
Capecitabine group
Docetaxel group
Osimertinib group
Nanopore sequencing
−Log10 P
Log2 fold change
m6A level
Blank control
Modeling drug
UGAGGACUCA


FIG. 1

m6A level of skin tissues of toes of mice
Relative m6A methylation level
20
15
10
5
0
Blank control group
Sorafenib group
Capecitabine group
Docetaxel group
Osimertinib group
NAP1L2 m6A level of skin tissues of toes of mice
Relative m6A methylation level
25
20
15
10
5
0
Blank control group
Sorafenib group
Capecitabine group
Docetaxel group
Osimertinib group


FIG. 2

Relative expression amount of mRNA
20
15
10
5
0
Blank control group
METTL3
sh-METTL3
FTO
sh-FTO

FIG. 3

MMP2
Relative expression amount of mRNA
8
6
4
2
0
Blank control group
NAP1L2
sh-NAP1L2
Sorafenib group
Capecitabine group
Docetaxel group
Osimertinib group
MMP9
Relative expression amount of mRNA
15
10
5
0
Blank control group
NAP1L2
sh-NAP1L2
Sorafenib group
Capecitabine group
Docetaxel group
Osimertinib group
NAP1L2
-
+
kDa
MMP2
(supernatant)
55
MMP2
(lysate)
55
β-actin
40
NAP1L2
-
+
kDa
MMP9
(supernatant)
100
MMP9
(lysate)
100
β-actin
40

FIG. 4

Expression amount of KIRT1
Relative expression amount of mRNA
Blank control group
HBEGF
HBEGF antibody
sh-METTL3
sh-NAP1L2
Expression amount of KIRT10
Relative expression amount of mRNA
Blank control group
HBEGF
HBEGF antibody
sh-METTL3
sh-NAP1L2
Expression amount of LOR
Relative expression amount of mRNA
Blank control group
HBEGF
HBEGF antibody
sh-METTL3
sh-NAP1L2
Expression amount of IVL
Relative expression amount of mRNA
Blank control group
HBEGF
HBEGF antibody
sh-METTL3
sh-NAP1L2
sh-METTL3
Anti-HBEGF (4.0 μg/ml)
HBEGF (2.5 ng/ml)
kDa
METTL3
IVL
KRT1
KRT10
LOR
β-actin

FIG. 5

80
60
40
20
0
-20
m6A inhibition rate (%)
Compound of formula (I) (4 nM)
Compound of formula (I) (400 nM)
Nicotinamide (4 nM)
Nicotinamide (4 μM)
UZH2 (5 μM)

FIG. 6

KRT1
Relative expression amount of mRNA
0
1
2
3
4
Control
HBEGF (2.5 ng/mL)
Compound of formula (I)

KRT10
Relative expression amount of mRNA
0
1
2
3
4
Control
HBEGF (2.5 ng/mL)
Compound of formula (I)

Loricrin
Relative expression amount of mRNA
0
5
10
15
Control
HBEGF (2.5 ng/mL)
Compound of formula (I)

Involucrin
Relative expression amount of mRNA
0
1
2
3
4
Control
HBEGF (2.5 ng/mL)
Compound of formula (I)

FIG. 7

Sorafenib modeling group
Stratum corneum thickness (μm)
0
30
60
90
120
Blank control
Sorafenib
Compound of formula (I)
Erlotinib modeling group
Stratum corneum thickness (μm)
0
50
100
150
Blank control
Erlotinib
Compound of formula (I)
Afatinib modeling group
Stratum corneum thickness (μm)
0
30
60
90
120
Blank control
Afatinib
Compound of formula (I)
Osimertinib modeling group
Stratum corneum thickness (μm)
0
30
60
90
120
Blank control
Osimertinib
Compound of formula (I)

FIG. 8

Sorafenib
Compound of formula (I)
Blisters
Inflammation
Congestion

FIG. 9

1.6
1.2
0.8
0.4
0
Pathological score
Blank control
Sorafenib
Compound of formula (I)
Blisters
Inflammation
Congestion

FIG.10

Docetaxel group
Capecitabine group
Normal control group

FIG. 11

Capecitabine - left paw
Capecitabine - right paw
Degree of swelling
0.2
0.1
0.0
-0.1
-0.2
-0.3
Normal control group
Capecitabine group
Capecitabine + compound of formula (I)
Capecitabine + nicotinamide
Docetaxel - left paw
Docetaxel - right paw
0.3
Docetaxel group
Docetaxel + compound of formula (I)
Docetaxel + nicotinamide


FIG. 12

Blank control group
Capecitabine group
Capecitabine + compound of formula (I)
Capecitabine + nicotinamide
Blank control group
Docetaxel group
Docetaxel + compound of formula (I)
Docetaxel + nicotinamide

FIG. 13

Blank control group
Capecitabine group
Capecitabine + compound of formula (I)
Capecitabine + nicotinamide
IL-8
Relative optical density value IOD
100000
50000
0
-50000
Blank control group
Capecitabine group
Capecitabine + compound of formula (I)
Capecitabine + nicotinamide

FIG. 14

Blank control group
Docetaxel group
Docetaxel + compound of formula (I)
Docetaxel + nicotinamide
IL-6
Relative optical density value IOD
600000
400000
200000
0
-200000
-400000
Blank control group
Docetaxel group
Docetaxel + compound of formula (I)
Docetaxel + nicotinamide

FIG. 15

Sorafenib group
Osimertinib group
Normal control group

FIG. 16

Sorafenib - left paw
Sorafenib - right paw
Degree of swelling
Normal control group
Sorafenib group
Sorafenib + compound of formula (I)
Sorafenib + nicotinamide
Osimertinib - left paw
Osimertinib - right paw
Blank control group
Osimertinib group
Osimertinib + compound of formula (I)
Osimertinib + nicotinamide

FIG. 17

Blank control group
Sorafenib group
Sorafenib + compound of formula (I)
Sorafenib + nicotinamide
Blank control group
Osimertinib group
Osimertinib + compound of formula (I)
Osimertinib + nicotinamide

FIG. 18

Blank control group
Sorafenib group
Sorafenib + compound of formula (I)
Sorafenib + nicotinamide
IL-1β
Relative optical density value IOD
300000
200000
100000
0
-100000
Blank control group
Sorafenib group
Sorafenib + compound of formula (I)
Sorafenib + nicotinamide

FIG. 19

Blank control group
Osimertinib group
Osimertinib + compound of formula (I)
Osimertinib + nicotinamide
IL-1β
Relative optical density value IOD
600000
400000
200000
0
-200000
Blank control group
Osimertinib group
Osimertinib + compound of formula (I)
Osimertinib + nicotinamide

FIG. 20

Counts
0
2,000
4,000
6,000
8,000
10,000
12,000
14,000
16,000
10
20
30
2Theta (Coupled TwoTheta/Theta) WL=1.54060
7.020 °
8.001 °
9.860 °
11.529 °
13.311 °
14.036 °
16.007 °
17.017 °
17.503 °
18.433 °
19.752 °
20.856 °
21.661 °
22.122 °
23.161 °
23.638 °
23.887 °
24.147 °
24.503 °
24.780 °
26.165
25.671 °
26.405 °
26.573 °
27.028 °
27.215 °
28.866 °
29.289 °
29.791 °
30.207 °
30.766 °
31.486 °
31.723 °
32.284 °
32.820 °
33.238 °
33.622 °
34.379 °
34.795 °
35.138 °
35.999 °
36.741 °
37.087 °
37.911 °
38.328 °
38.591
38.665 °

FIG. 21

Counts
0
100
200
300
400
500
10
20
30
2Theta (Coupled TwoTheta/Theta) WL=1.54060
6.139 °
9.386 °
11.847 °
13.641 °
14.019 °
15.097 °
16.746 °
17.556 °
17.893 °
18.822 °
19.428 °
21.196 °
21.875 °
23.762 °
24.300 °
26.354 °
26.566 °
27.454 °
28.722 °
31.980 °
33.296 °
34.680 °
35.936 °

FIG. 22

Counts
0
10,000
20,000
30,000
10
20
30
40
2Theta (Coupled TwoTheta/Theta) WL=1.54060
6.586 °
7.120 °
10.302 °
13.153 °
14.229 °
14.871 °
15.763 °
16.443 °
18.719 °
19.762 °
20.113 °
20.643 °
21.217 °
21.383 °
21.899 °
22.829 °
23.211 °
23.611 °
24.012 °
24.982 °
25.287 °
26.465 °
26.627 °
27.860 °
28.300 °
28.621 °
28.687 °
30.305 °
31.158 °
31.789 °
32.728 °
33.199 °
35.727 °
36.432 °
37.176 °
38.026 °

FIG. 23

Counts
0
100
200
300
400
2Theta (Coupled TwoTheta/Theta) WL=1.54060
10
20
30
40
10.588 °
12.575 °
14.928 °
17.645 °
18.231 °
18.444 °
19.706 °
20.417 °
21.232 °
21.527 °
23.125 °
24.776 °
25.300 °
25.928 °
26.357 °
26.840 °
27.608 °
28.937 °
30.116 °
30.376 °
32.070 °
33.925 °
35.159 °
35.368 °
35.719 °
36.010 °
36.881 °
37.253 °
38.478 °
39.512 °

FIG. 24

**INTERNATIONAL SEARCH REPORT**

International application No. **PCT/CN2024/142961**

**A. CLASSIFICATION OF SUBJECT MATTER**

A61K31/403(2006.01)i； A61K9/00(2006.01)i； A61P17/00(2006.01)i

According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNTXT, ENTXT, ENTXTC, JPTXT, KRTXT, WPABS, VEN, CNKI, REGISTRY, CAPLUS, ISI_Web of science, PubMed: EX527, 赛利司他, 塞来司他, 司来司他, 赛利西塔, 基于式（I）的结构进行了检索, search based on the structure of formula (I), 羊毛脂, 蜂蜡, 石蜡, 凡士林, 油, 皮肤, 棘皮病, 湿疹, 鱼鳞病, 银屑病, 角化病, 红斑狼疮, 皮炎, selistat, structure search based on the formula (I), skin, acanthosis, eczema, ichthyosis, proriasis, keratosis, erythematosus, dermatisis, lanolin, beewax, cera alba, paraffin, vaseline, oil

**C. DOCUMENTS CONSIDERED TO BE RELEVANT**

| Category* | Citation of document, with indication, where appropriate, of the relevant passages | Relevant to claim No. |
|---|---|---|
| E | WO 2025007928 A1 (SUZHOU MERNA THERAPEUTICS CO., LTD.) 09 January 2025 (2025-01-09)<br>description, page 55, embodiment 2, and page 70, embodiments 22-23 | 1-4, 9-10 |
| A | CN 109745563 A (FUDAN UNIVERSITY) 14 May 2019 (2019-05-14)<br>claims 5-10 | 1-10 |
| A | CN 112891344 A (FIRST AFFILIATED HOSPITAL OF ANHUI MEDICAL UNIVERSITY) 04 June 2021 (2021-06-04)<br>description, paragraphs [0041]-[0043] | 1-10 |
| A | CN 113101289 A (ZHEJIANG UNIVERSITY) 13 July 2021 (2021-07-13)<br>claim 1 | 1-10 |
| A | CN 104971051 A (LIU XIAOBIN et al.) 14 October 2015 (2015-10-14)<br>claim 1 | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 February 2025** | **22 March 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/142961**

**C. DOCUMENTS CONSIDERED TO BE RELEVANT**

| Category* | Citation of document, with indication, where appropriate, of the relevant passages | Relevant to claim No. |
|---|---|---|
| A | CN 103230603 A (XIDIAN UNIVERSITY) 07 August 2013 (2013-08-07)<br>claim 1 | 1-10 |
| A | 韦安吉 等 (WEI, Anji et al.). "m6A 修饰与调节免疫在系统性红斑狼疮的研究进展 (Research Progress of m6A Modification and Immune Regulation in Systemic Lupus Erythematosus)"<br>*右江医学 (Chinese Youjiang Medical Journal),*<br>Vol. 51, No. 6, 30 June 2023 (2023-06-30), 486-491<br>page 488, section 3 | 1-10 |

Form PCT/ISA/210 (second sheet) (July 2022)

**INTERNATIONAL SEARCH REPORT**
**Information on patent family members**

International application No.
**PCT/CN2024/142961**

| Patent document cited in search report | Publication date (day/month/year) | Patent family member(s) | Publication date (day/month/year) |
|---|---|---|---|
| WO 2025007928 A1 | 09 January 2025 | None | |
| CN 109745563 A | 14 May 2019 | CN 109745563 B | 16 April 2021 |
| CN 112891344 A | 04 June 2021 | None | |
| CN 113101289 A | 13 July 2021 | None | |
| CN 104971051 A | 14 October 2015 | None | |
| CN 103230603 A | 07 August 2013 | CN 103230603 B | 22 October 2014 |

Form PCT/ISA/210 (patent family annex) (July 2022)

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Patent documents cited in the description


- CN 2023118444059


### Non-patent literature cited in the description


- **FRYE M. et al.** *Science*, 2018, vol. 361, 2073-2092
- **YANG C. et al.** *Cell Death & Disease*, 2020, vol. 11, 960
- **MEYER K.D** ; **JAFFREY S.R.** *Nature Review Molecular Cell Biology*, 2014, vol. 15, 313-326
- **HSU P.J. et al.** *Journal of Biological Chemistry*, 2019, vol. 294, 19889-19895
- **YAO Y. et al.** *FASEB Journal*, 2019, vol. 33, 7529-7544
- **LE ZHANGHUI**. Preliminary Study on the Pathogenesis of LncRNA and RNA m6A Methylation in Acral Melanoma. *Dissertation*, 2019
- **LI T. D. et al.** *Cancer Cell*, 2020, vol. 20, 239
- **CUI Y. H. et al.** *Nature Communications*, 2021, vol. 12, 2183
- **KUMPER M et al.** *Am. J. Physiol. Cell. Physiol.*, 2022, vol. 323 (4), 1290-1303